(19) Europäisches Patentamt
European Patent Office
Office européen des brevets

(11) EP 2 808 400 A1

(12) EUROPEAN PATENT APPLICATION

(43) Date of publication:
**03.12.2014 Bulletin 2014/49**

(51) Int Cl.:
***C12Q 1/68*** *(2006.01)*

(21) Application number: **14176354.0**

(22) Date of filing: **21.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.02.2007 DK 200700273**
**24.04.2007 DK 200700601**

(62) Document number(s) of the earlier application(s) in accordance with Art. 76 EPC:
**12154273.2 / 2 474 628**
**08709178.1 / 2 129 796**

(71) Applicant: **Oslo Universitetssykehus HF**
**0514 Oslo (NO)**

(72) Inventors:
- **Skotheim, Rolf I.**
**0372 Oslo (NO)**
- **Lothe, Ragnhild A.**
**0267 Oslo (NO)**
- **Lind, Guro E.**
**0770 Oslo (NO)**
- **Ahlquist, Terje C.**
**0655 Oslo (NO)**

(74) Representative: **Plougmann & Vingtoft A/S**
**Rued Langgaards Vej 8**
**2300 Copenhagen S (DK)**

Remarks:
This application was filed on 09-07-2014 as a divisional application to the application mentioned under INID code 62.

# (54) New markers for cancer

(57) The present invention relates to novel markers for hypermethylation of gene promoters in cancers. In particular the present invention relates to a method of determining whether a tumour is developing in the aero-digestive system, or whether a subject is relapsing after treatment of such a tumour. The method comprises determining the methylation level, the number of methylated CpG sites or the methylation state of CpG sites in a nucleic acid sequence in the promoter region, first exon or intron, of one or more genes selected from the group consisting of CNRIP1, MAL, FBN1, SPG20, SNCA, and INA. The method further relates to a diagnostic kit for detecting tumours in the aero-digestive tract.

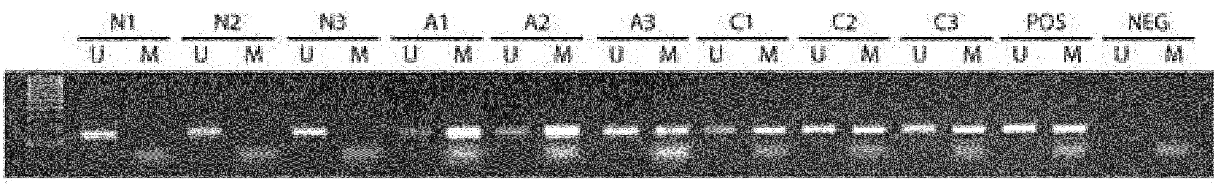


Fig. 1

EP 2 808 400 A1

## Description

### Technical field of the invention

The present invention relates to novel markers for hypermethylation of gene promoters in cancers. In particular the present invention relates to a method of determining whether a tumour is developing in the aero-digestive system, or whether a subject is relapsing after treatment of such a tumour. The methods of the present invention comprise determining the methylation state of CpG sites in the promoter region/sequence of one or more particular genes. The invention further relates to the use of such methylated genes and to diagnostic kits for detecting cancer.

### Background of the invention

Impaired epigenetic regulation is as common as gene mutations in human cancer. These mechanisms lead to quantitative and qualitative gene expression changes causing a selective growth advantage, which may result in cancerous transformation. Aberrantly hypermethylated CpG islands in the gene promoter associated with transcriptional inactivation are among the most frequent epigenetic changes in cancer. Since early detection of disease can result in improved clinical outcome for most types of cancer, the identification of cancer-associated aberrant gene methylation represents promising novel biomarkers. For cancers in the aero-digestive system, including colorectal cancer, initial studies have identified the presence of aberrantly methylated DNA in patient blood and feces. Genes aberrantly hypermethylated in high frequencies already among benign tumours and only rarely in normal mucosa would be good candidate diagnostic biomarkers due to the potential clinical benefit of early detection of high risk adenomas as well as of low risk stages of carcinomas.

In general, however, the sensitivity and specificity of existing early markers for cancers in the aero-digestive system remain poor and, this far, only a few of the genes that have actually been screened for methylation have shown a reasonably high sensitivity and specificity. Specific hypermethylation is seen in *VIM* (vimentin) and *SFRP2* as reported by Muller et al. and Chen et al. and recently, *ADAMTS1* and *CRABP1* were suggested to have a high frequency of cancer specific hypermethylation in colorectal tumours in a report from Lind et al. while the frequency of hypermethylation of *NR3C1* was considerably lower. Lind et al. further identified 18 genes as potential markers for colorectal cancers. In a study by Mori et al. it was concluded that the T-cell differentiation protein MAL, one of the 18 candidate genes discussed by Lind et al. would not be an appropriate diagnostic biomarker for cancers in the aero-digestive tract since the methylation frequency *of MAL* is low, corresponding to only 6% methylation (2/34 samples). Further analyses conducted by the inventors of a number of the 18 candidate genes did not provide encouraging results: NDRG1 was unmethylated in all samples analyzed, NR3C1 was methylated but only at a very low frequency and in subsequent sequence analyses of SDHA it was impossible to confirm the identity of this gene, rendering it unsuitable as a marker for cancer development.

In conclusion, there is no indication that any of the genes discussed by Lind et al. would provide any improvement to current technology for detection of cancer. Consequently, there is a need for a panel of genes in which each gene is hypermethylated at a high frequency and specificity in cancers. In particular, there is a need for a gene panel which is useful in non-invasive techniques, such as techniques involving the use of stool samples, or in techniques which may be used on sample material which is easily obtained, such as blood or mucous. Such a gene panel would greatly improve the possibility for early detection of these cancers. The ultimate goal would be to develop diagnostic tests determining hypermethylation in only a few, such as 2 or 3, high frequency gene markers.

Hence, identification of further genes in which CpG islands in the promoter region are hypermethylated at a high frequency in cancers is desirable.

### Summary of the invention

The present invention is based on the realization by the inventors that a particular subset of the genes which were identified as potential markers by Lind et al. contain CpG sites that are methylated at an exceptionally high frequency in aero-digestive cancers.

Thus, it is an object of the present invention to provide a panel of diagnostic markers for cancer e.g. cancers in the aero-digestive system, in particular colon cancer and colorectal cancer. This panel of markers solves the problems relating to the low specificity and frequency of methylation in the majority of known markers for cancers.

Accordingly, one aspect of the invention relates to a method for determining whether a subject has developed, is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

The method may further comprise the steps of;

b) comparing the methylation level, the number of methylated CpG sites or the methylation state of CpG sites to a reference; and

c) identifying said subject as being likely to develop, being developing or being predisposed for developing cancer, or relapsing after treatment of cancer, if the methylation level, the number of methylated CpG sites or the methylation state of CpG sites is higher than the methylation level, the number of methylated CpG sites or the methylation state of CpG sites of the reference and identifying a subject as unlikely to develop, being developing or being predisposed for developing cancer, or relapsing after treatment of cancer, if the methylation level, the number of methylated CpG sites or the methylation state of CpG sites is below the methylation level, the number of methylated CpG sites or the methylation state of CpG sites of said reference.

Another aspect of the present invention relates to a method for determining whether a subject has developed, is developing or is predisposed for developing cancer, or whether a subject is relapsing after treatment of cancer, comprising the step of;

a) determining the methylation level, the number of methylated CpG sites or the methylation state of CpG sites in a sample from a subject

b) constructing a percentile plot of the methylation level, the number of methylated CpG sites or the methylation state of CpG sites of said at least one gene obtained from a sample from a healthy population;

c) constructing a ROC (receiver operating characteristics) curve based on the methylation level, the number of methylated CpG sites or the methylation state of CpG sites determined in the healthy population and on the methylation level, the number of methylated CpG sites or the methylation state of CpG sites determined in a population with cancer;

d) selecting from the ROC-curve the desired combination of sensitivity and specificity

e) determining from the percentile plot the methylation level, the number of methylated CpG sites or the methylation state of CpG sites corresponding to the determined or chosen specificity; and

f) predicting that the subject is likely to have cancer, if methylation level, the number of methylated CpG sites or the methylation state of CpG sites of said at least one gene in the sample is equal to or higher than said methylation level, the number of methylated CpG sites or the methylation state of CpG sites corresponding to the desired combination of sensitivity/specificity, and predicting that the subject is unlikely or not to have cancer, if the methylation level, the number of methylated CpG sites or the methylation state of CpG sites in the sample is lower than said methylation level, the number of methylated CpG sites or the methylation state of CpG sites corresponding to the desired combination of sensitivity/specificity.

The invention further concerns a diagnostic kit for the determination cancer comprising one or more oligonucleotide primers or one or more sets of oligonucleotide primers, which are each complementary to a nucleic acid sequence of the genes selected from:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

The invention also concerns the use of markers according to the invention. Thus the invention further concerns: The use of one or more genes selected from the group comprising of

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

In addition, the invention concerns the use of a nucleic acid sequence, wherein said nucleic acid comprises a nucleic acid sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), A) or C).

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer

The invention further provides an antibody recognizing a methylated nucleic acid sequences selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in a);
C) A sub-sequence of a nucleic acid sequence as defined in a) or b);
D) A nucleic acid sequence which is at least 75% identical to a sequence as defined in A), B) or C).

**Brief description of the figures**

Figure 1
Show representative methylation-specific polymerase chain reaction results from the analysis of MAL in three normal mucosa samples, three adenomas, and three carcinomas. A visible PCR product in lanes U indicates the presence of unmethylated alleles whereas a PCR product in lanes M indicates the presence of methylated alleles. Abbreviations: A, adenoma; C, carcinoma, N normal mucosa; POS, positive control consisting of normal blood (control for unmethylated samples) and in vitro methylated DNA (control for methylated samples); NEG, negative control (containing water as template); U, lane for unmethylated MSP product; M, lane for methylated MSP product. The illustration is a merge of two gel panels as the adenomas were run on a separate gel.

Figure 2-4
Show up-regulation of gene expression after epigenetic drug treatment of initially methylated cell lines. Up-regulated mRNA expression of CNRIP1, INA, and SPG20 was found in colon cancer cell lines after treatment with the demethylating 5-aza-2'deoxycytidine, alone and in combination with the deacetylase inhibitor trichostatin A. The panels demonstrate the relative expression values of CNRIP1, INA, and SPG20, respectively (linear scale) in six colon cancer cell lines, HT29, SW48, HCT15, SW480, RKO and LS1034, treated with 5-aza-2'deoxycytidine alone (1uM and 10uM), trichostatin A alone, and the two drugs in combination (1 $\mu$M 5-aza-2'deoxycytidine alone and 0.5 $\mu$M trichostatin A). The two doses (low and high) of 5-aza-2'deoxycytidine gave comparable increases in relative expression values for all three genes. This means that demethylation of cell lines can be achieved by culturing them in the presence of low doses of 5-aza-2'-deoxycytidine, which is an advantage considering the cytotoxicity of this drug. For CNRIP1 and INA the combined treatment was more effective than the individual treatment with 5-aza-2'-deoxycytidine alone and trichostatin A alone. The combined treatment also increased SPG20 expression, however similar or higher reactivation could be achieved by 5-aza-2'-deoxycytidine treatment alone. As expected, treatment with the deacetylase inhibitor trichostatin A alone did not increase the gene expression of neither CNRIP, INA nor SPG20. Abbreviation: AZA, 5-aza-2'deoxycytidine; TSA, trichostatin A.

Figure 5

Show methylation status of the *MAL* promoter in normal colon mucosa samples and colorectal carcinomas. Representative results from methylation-specific polymerase chain reaction are shown. A visible PCR product in lanes U indicates the presence of unmethylated alleles whereas a PCR product in lanes M indicates the presence of methylated alleles. N, normal mucosa; C, carcinoma; Pos, positive control (unmethylated reaction: DNA from normal blood, methylated reaction: *in vitro* methylated DNA); Neg, negative control (containing water as template); U, lane for unmethylated MSP product; M, lane for methylated MSP product.

Figure 6

Show site specific methylation within the *MAL* promoter. Bisulfite sequencing of the *MAL* promoter verifies methylation status assessed by methylation-specific polymerase chain reaction. The upper part of the figure is a schematic presentation of the CpG sites successfully amplified by the two analyzed bisulfite sequencing fragments, A (-68 to +168; to the right) and B (-427 to -85; to the left). The transcription start site is represented by +1 and the vertical bars indicate the location of individual CpG sites. The two arrows indicate the location of the MSP primers. For the lower part of the figure, filled circles represent methylated CpGs; open circles represent unmethylated CpGs; and open circles with a slash represent partially methylated sites (the presence of approximately 20-80% cytosine, in addition to thymine). The column of U, M and U/M at the right side of this lower part lists the methylation status of the respective cell lines as assessed by us using MSP analyses. Abbreviations: MSP, methylation-specific PCR; s, sense; as, antisense; U, unmethylated; M, methylated; U/M, presence of both unmethylated and methylated band.

Figure 7

Show the "Bisulfite sequence" of the *MAL* promoter. Representative bisulfite sequencing electropherograms of the *MAL* promoter in colon cancer cell lines. A subsection of the bisulfite sequence electropherogram, covering CpG sites +11 to +15 relative to transcription start. Cytosines in CpG sites are indicated by a black arrow, whereas cytosines that have been converted to thymines are underlined in red. The *MAL* promoter sequencing electropherograms illustrated here, are from the unmethylated V9P cell line and the hypermethylated ALA and HCT116.

Figure 8

Show MAL expression in cancer cell lines and colorectal carcinomas. Promoter hypermethylation *of MAL* was associated with reduced or lost gene expression in *in vitro* models. The quantitative gene expression level *of MAL* is displayed as a ratio between the average of two *MAL* assays (detecting various splice variants) and the average of the two endogenous controls, *GUSB* and *ACTB.* The value has been multiplied by a factor of 1000. Below each sample the respective methylation status is shown, as assessed by methylation-specific polymerase chain reaction. Filled circles represent promoter hypermethylation *of MAL,* open circles represent unmethylated *MAL,* and open circles with a slash represent the presence of both unmethylated and methylated alleles. Colorectal carcinomas are divided in an unmethylated group (n = 3) and a hypermethylated group (n = 13), and the median expression is displayed here. The tissue of origin for the individual cell lines can be found in table 1.

Figure 9

Show up-regulation of MAL expression after drug treatment. Decreased promoter methylation of MAL followed by up-regulated mRNA expression in colon cancer cell lines was found after treatment with the demethylating 5-aza-2'deoxycytidine, alone and in combination with the deacetylase inhibitor trichostatin A. Upper panel demonstrate the relative expression values of MAL (linear scale) in two colon cancer cell lines, HT29 and HCT15, treated with 5-aza-2'deoxycytidine alone, trichostatin A alone, and the two drugs in combination. Lower panel illustrate MAL MSP results for the same samples. A visible PCR product in lanes U indicates the presence of unmethylated alleles whereas a PCR product in lanes M indicates the presence of methylated alleles. Abbreviation: AZA, 5-aza-2'deoxycytidine; TSA, trichostatin A; Pos, positive control (unmethylated reaction: DNA from normal blood, methylated reaction: in vitro methylated DNA); Neg, negative control (containing water as template); U, lane for unmethylated MSP product; M, lane for methylated MSP product.

Figure 10

Show MAL expression in colorectal carcinomas. Positive cytoplasmic staining of MAL was found in kidney tubuli (A), and no staining was observed in heart muscle (B), in agreement with earlier reports (Marazuela M, et al J Histochem Cytochem 2003, 51: 665-674). The epithelial cells of colorectal carcinomas were MAL negative (C, D), whereas in normal colon tissue, cytoplasmic expression of MAL was found in both epithelia and connective tissue (E, F). All images were captured using the 40x lens (400x magnification).

The present invention will now be described in more detail in the following.

**Detailed description of the invention**

Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the following terms are defined:

*Epigen tics*

Methylation is an epigentic change which is defined as non-sequence-based alterations that are inherited through cell division.

*Methylation*

"Hypermetylation" is in this context simply methylation above reference methylation. Reference methylation is methylation of the gene in a sample from a healthy subject, or from normal tissue. Thus a methylated gene which in normal tissues is unmethylated will be classified as hypermethylated.
The "methylation state" is a measure of the presence or absence of a methyl modification in one or more CpG sites in at least one nucleic acid sequence. It is to be understood that the methylation state of one or more CpG sites is preferably determined in multiple copies of a particular gene of interest.

The "methylation level" is an expression of the amount of methylation in one or more copies of a gene or nucleic acid sequence of interest. The methylation level may be calculated as an absolute measure of methylation within the gene or nucleic acid sequence of interest. Also a "relative methylation level" may be determined as the amount of methylated DNA, relative to the total amount DNA present or as the number of methylated copies of a gene or nucleic acid sequence of interest, relative to the total number of copies of the gene or nucleic acid sequence. Additionally, the "methylation level" can be determined as the percentage of methylated CpG sites within the DNA stretch of interest.

The term methylation level also encompasses the situation wherein one or more CpG site in e.g. the promoter region is methylated but where the amount of methylation is below amplification threshold. Thus methylation level may be an estimated value of the amount of methylation in a gene of interest.

The invention is not in any way limited to certain types of assays for measuring methylation status or methylation level of the genes according to the invention.

In one embodiment if the methylation level of the gene of interest is 15% to 100%, such as 50% to 100%, more preferably 60%- 100 %, more preferably 70-100 %, more preferably 80% to 100%, more preferably 90% to 100%. Thus in one embodiment of the present invention the methylation level of the genes according to the invention is 80%, 81%, 82%, 83%, 84%, 85%, 86%, 87%, 88%, 89%, 90%, 91%, 92%, 93%, 94%, 95%, 96%, 97%, 98%, 99% or 100%.

*CpG*

A "CpG site" is a region of DNA where a cytosine nucleotide occurs next to a guanine nucleotide in the linear sequence of bases along its length. "CpG" stands for cytosine and guanine separated by a phosphate, which links the two nucleosides together in DNA. The "CpG" notation is used to distinguish a cytosine followed by a guanine from a cytosine base paired to a guanine.

A CpG islands may be defined as a contiguous window of DNA of at least 200 base pairs in which the G:C content is at least 50% and the ratio of observed CpG frequency over the expected frequency exceeds 0.6. However, they may also be defined more stringent definition as a 500-base-pair window with a G:C content of at least 55% and an observed over expected CpG frequency of at least 0.65.

*Promoter region or sequence*

A "promoter region or sequence" comprises a consecutive nucleic acid sequence extending 1000bp upstream from the transcription start site of a given gene and a consecutive nucleic acid sequence extending 300 base pairs downstream from the transcription start site. In the sequencelist, the upstream sequence is indicated in small letters whereas the downstream sequence is indicated in capital letters. In the 3' part of the sequences small letters indicate intronic sequence.

*Transcription start site*

"Transcription start site" is used in relation to the current invention to describe the point at which transcription is initiated. Transcription can initiate at one or more sites within the gene, and a single gene may have multiple transcriptional start sites, some of which may be specific for transcription in a particular cell-type or tissue.

*Methylation of nucleic acid sequences*

A gene is a region of DNA that is responsible for the production and regulation of a polypeptide chain. Genes include both coding and non-coding portions, including introns, exons, promoters, initiators, enhancers, terminators, microRNAs, and other regulatory elements. As used herein, "gene" is intended to mean at least a portion of a gene. Thus, for example, "gene" may be considered a promoter for the purposes of the present invention. Accordingly, in one embodiment of the present invention, at least one member of the panel of genes comprises a non-coding portion of the entire gene. In a particular embodiment, the non-coding portion of the gene is a promoter. In another embodiment, all members of the entire panel of genes comprise non-coding portions of the genes, such as but not limited to, introns. In another particular embodiment, the non-coding portions of the members of the genes are promoters. In another embodiment of the present invention, at least one member of the panel of genes comprises a coding portion of the gene. In another embodiment, all members of the entire panel of genes comprise coding portions of the genes.

The term "nucleic acid sequence" refers to a polymer of deoxyribonucleotides in either single- or double-stranded form.

A "subsequence" is any portion of an entire sequence. Thus, a subsequence refers to a consecutive sequence of amino acids or nucleic acids which is part of a longer sequence of nucleic acids (e.g. polynucleotide).

The term "sequence identity" indicates a quantitative measure of the degree of homology between two nucleic acid sequences of equal length. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\frac{(N_{ref}-N_{dif})100}{N_{ref}}$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$=2 and $N_{ref}$=8).

With respect to all claims of the invention relating to nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). Alternatively, the sequences may be analysed using the program DNASIS Max and the comparison of the sequences may be done at www.paralign.org. This service is based on the two comparison algorithms called Smith-Waterman (SW) and ParAlign. The first algorithm was published by Smith and Waterman (1981) and is a well established method that finds the optimal local alignment of two sequences The other algorithm, ParAlign, is a heuristic method for sequence alignment; details on the method is published in Rognes et al. Default settings for score matrix and Gap penalties as well as E-values were used.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA molecule, then the oligonucleotide and the DNA are considered to be complementary to each other at that position. The DNA strand are considered complementary to each other when a sufficient number of nucleotides in the oligonucleotide can form hydrogen bonds with corresponding nucleotides in the target DNA to enable the formation of a stable complex.

In the present context the expressions "complementary sequence" or "complement" therefore also refer to nucleotide sequences which will anneal to a nucleic acid molecule of the invention under stringent conditions.

The term "stringent conditions" refers to general conditions of high, weak or low stringency.

The term "stringency" is well known in the art and is used in reference to the conditions (temperature, ionic strength and the presence of other compounds such as organic solvents) under which nucleic acid hybridizations are conducted. With "high stringency" conditions, nucleic acid base pairing will occur only between nucleic acid fragments that have a high frequency of complementary base sequences, as compared to conditions of "weak" or "low" stringency. Suitable conditions for testing hybridization involve pre-soaking in 5xSSC and prehybridizing for 1 hour at ~40°C in a solution of 20% formamide, 5xDenhardt's solution, 50mM sodium phosphate, pH 6.8, and 50mg of denatured sonicated calf thymus DNA, followed by hybridization in the same solution supplemented with 100mM ATP for 18 hours at ~40°C, followed by three times washing of the filter in 2xSSC, 0.2% SDS at 40°C for 30 minutes (low stringency), preferred at 50°C (medium stringency), more preferably at 65°C (high stringency), even more preferably at ~75°C (very high stringency). More details about the hybridization method can be found in Sambrook et al., Molecular Cloning: A Laboratory Manual, 2nd Ed., Cold Spring Harbor, 1989.

*Cancer*

"Cancer" is a group of diseases in which cells are aggressive (grow and divide without respect to normal limits), invasive (invade and destroy adjacent tissues), and sometimes metastatic (spread to other locations in the body). These three malignant properties of cancers differentiate them from benign tumours, which are self-limited in their growth and do usually not invade or metastasize Cancer is usually classified according to the tissue from which the cancerous cells originate, as well as the normal cell type they most resemble. A definitive diagnosis usually requires histologic examination of a tissue biopsy specimen by a pathologist. The prognosis of cancer patients is most influenced by the type of cancer, as well as the stage, or extent of the disease. An early diagnose is usually associated with a more successful treatment and increased survival rate.

"Tumour suppressor genes" are genes often inactivated in cancer cells, resulting in the loss of normal functions in those cells, such as accurate DNA replication, control over the cell cycle, orientation and adhesion within tissues, and interaction with protective cells of the immune system. In several cancers including colorectal cancer, several tumour suppressor genes have been identified to be epigenetically inactivated by CpG island promoter hypermethylation

A tumour may be any abnormal swelling, lump or mass however as the term is interpretation herein the term means neoplasm, specifically solid neoplasm. Neoplasm is defined as an abnormal proliferation of genetically altered cells. Neoplasms can be benign or malignant. Malignant neoplasm or malignant tumour, is to be understand here as cancer. Benign neoplasm or benign tumour is a tumour (solid neoplasm) that normally stops growing by it self, and does not invade other tissues and does not form metastases. However, benign tumours may become malignant.

Tumours invading surrounding tissues are to be understood herein as cancer. Premalignancy, pre-cancer or non-invasive tumour is to be understood herein as a neoplasm that is not invasive but has the potential to progress to cancer (become invasive) if left untreated.

The methods according to the invention can be used to determine the degree of severity i.e. stages, such as Dukes system, the Astler-Coller system and TNM staging AJCC (American joint committee on cancer). Duke system is a four-class staging system that classifies colorectal carcinoma from A to D based on the extent of the tumour: A, penetration into but not through the bowel wall; B, penetration through the bowel wall; C, lymph node involvement regardless of extent of bowel wall penetration; D, spreading of cancer to distant organs, e.g. liver and lung. Many modifications of this classification exist, e.g. TNM staging

*Biomarker*

A biomarker can be a substance whose detection indicates a particular disease state. A biomarker may also indicate a change in expression or state of a protein that correlates with the risk or progression of a disease, or with the susceptibility of the disease to a given treatment. A good biomarker can be used to diagnose disease risk, presence of disease in an individual, or to tailor treatments for the disease in an individual. The terms biomarker and marker is used interchangeably in the present context.

Cancer marker, tumour marker and in this context methylation marker is a marker for detecting cancer and/or tumour. The marker may be used for detecting in a subject the presence of cancer and/or tumour, or a developing cancer and/or tumour, or weather the subject is predisposed or relapsing from cancer and/or tumour

The genes according to the invention may be a marker, a biomarker, a cancer marker or a tumour marker respectively.

*Tumour progression*

In addition to determining whether a subject has developed, is developing or is predisposed for developing cancer, or whether a subject is relapsing after treatment of cancer, the methods according to the invention may also be used for detecting the progression of cancer in a subject. This may be done by determining the methylation state or level of one or more genes in a subject at different time points, and then determine the difference in methylation state or level of one or more genes over time. A difference in methylation state or level over time may be indicative of whether the subject has developed, is developing or is predisposed for developing cancer, or whether a subject is relapsing after treatment of cancer.

The present invention also provides a method for making a prognosis about disease course in a human cancer patient. For the purposes of this invention, the term "prognosis" is intended to encompass predictions and likelihood analysis of disease progression, particularly tumor recurrence, metastatic spread and disease relapse. The prognostic methods of the invention are intended to be used clinically in making decisions concerning treatment modalities, including therapeutic intervention, diagnostic criteria such as disease staging, and disease monitoring and surveillance for metastasis or recurrence of neoplastic disease. Treatment is to be understood herein as both preventive and curative treatment.

The present invention also provides methods for confirming the results or indications obtained by a preceding method such as a test or screening method.

Thus the phrase "developed, are developing or is predisposed for developing cancer, or whether a subject is relapsing after treatment of cancer" as used herein encompasses determination and/or prediction such as estimation or determination the likelihood of current presence of, future occurrence of or future recurrence of cancer.

*Sample*

A sample may be but is not limited to a tissue section or biopsy, such as a portion of the neoplasm that is being treated or it may be a portion of the surrounding normal tissue. The sample may preferably be but is not limited to blood, stool (feaces), urine, pleural fluid, gall, bronchial fluid, oral washings, tissue biopsies, ascites, pus, cerebrospinal fluid, aspitate, follicular fluid, tissue or mucus. The sample may be processed prior to being assayed. For example, the sample may be diluted, concentrated or purified and/or at least one compound, such as an internal standard, may be added to the sample. The procedures for handling different samples are known the skilled artisan.

It is to be understood the all methods according to the invention preferably concern in vitro analyses of a sample.

*Sample methylation frequency*

The term "sample methylation frequency" is defined herein as a quantitative measurement of methylated samples i.e. the relative number of samples in which the gene of interest is methylated. As an example, the sample methylation frequency of CNRIP1 is 100 %, 20 out of 20 samples from colon cell lines are methylated, as apparent from table 3. The relative amount of methylated samples is compared to a reference or cut-off level which is estimated on basis of the sensitivity and the specificity of each gene

*Reference*

In order to determine whether a subject has developed, is developing or is predisposed for developing cancer, or whether a subject is relapsing after treatment of cancer, a reference or reference level or value has to be established. The reference also makes it possible to count in assay and method variations, kit variations, handling variations, variations related to combining the markers with each other or with other known markers, and other variations not related directly or indirectly to methylation.

In the context of the present invention, the term "reference" relates to a standard in relation to quantity, quality or type, against which other values or characteristics can be compared, such as a standard curve.

The reference or reference level is to be understood in the present context as a value or level, which has been determined by measuring the parameter (methylation state or methylation level) in both a healthy control population and a population with known cancer thereby determining the reference value which identifies the cancer population with either a predetermined specificity or a predetermined sensitivity based on an analysis of the relation between the parameter values and the known clinical data of the healthy control population and the cancer patient population.

As will be generally understood by those of skill in the art, methods for screening for cancers are processes of decision making by comparison. For any decision-making process, reference-values, reference-levels or cut-off points based on subjects having cancer or a condition of interest and/or subjects not having cancer, or a condition of interest are needed.

The reference level (or the cut-off point or cut-off level) can be established taking into account several criteria including the acceptable number of subjects who would go on for further invasive diagnostic testing, the average risk of having and/or developing e.g. cancer to all the subjects who go on for further diagnostic testing, a decision that any subject whose patient specific risk is greater than a certain risk level such as 1 in 400 or 1:250 (as defined by the screening organization or the individual subject) should go on for further invasive diagnostic testing or other criteria known to those skilled in the art.

The reference level can be adjusted based on several criteria such as but not restricted to certain groups of individuals tested. As an example the cut-off level may be set lower in individuals with immunodeficiency and in patients at great risk of progressing to active disease or the reference level may be set higher in groups of otherwise healthy individuals with low risk of developing active disease.

The reference level may be different for various stages of disease (e.g. begnin tumour or malign tumour), the source of normal mucosa (from cancer free individuals versus cancer patients) or from the source of blood and faces. In addition, the reference level may be different for subjects predisposed for or subjects relapsing from treatment of disease.

Reference levels can be customized to accommodate a specific sensitivity or specificity: If one desires a test with high sensitivity the reference level can be set low. If one seeks a test with high specificity the reference level can be set higher.

Depending on the prevalence or expected prevalence of presence of disease, the reference level can be adjusted for obtaining as few false positive or as few false negative results as wanted, depending on the severity of the disease and the consequences of determining, whether the patient is positive for the test or negative for the test.

The methods for measuring methylation, the chosen part of nucleic acid sequences comprising the promoter region of the maker genes or other parameters will result in other reference values, which can be determined in accordance with the teachings herein.

The reference level can be different, if a single patient with symptoms has to be diagnosed or the test is to be used in a screening of a large number of individuals in a population.

The reference level can be based on combined methylation state or level measurements of different markers such as but not limited to CNRIP1, SPG20, FBN1, SNCA, INA, MAL, ADAMTS1, VIM, SFRP1 and/or SFRP2. A compound reference level may result in other values, which can be determined in accordance with the teachings of the present invention.

The level of methylation is compared to a set of reference data or a reference-level, such as the cut-off value, to determine whether the subject is at an increased risk or likelihood of cancer.

*Specificity and sensitivity*

The sensitivity of any given screening test is the proportion of individuals with the condition who are correctly identified or diagnosed by the test, e.g. the sensitivity is 100%, if all individuals with a given condition have a positive test. The specificity of a given screening test is the proportion of individuals without the condition who are correctly identified or diagnosed by the test, e.g. 100 % specificity is, if all individuals without the condition have a negative test result.

Thus the sensitivity is defined as the (number of true-positive test results) / (number of true-positive + number of false- negative test results).

The specificity is defined as (number of true-negative results) / (number of true-negative + number of false-positive results)

The genes according to the present application is characterized by having high sensitivity (the relative amount of samples comprising the methylated gene of interest from subjects with cancer is high) and high specificity (the relative amount of samples comprising the methylated gene of interest from subjects without cancer is low).

A good marker for cancer is a gene which is methylated in almost all samples when a subject has cancer, and not methylated when in samples from subject not having cancer.

The specificity of the method according to the present invention is preferably from 70% to 100%, such as from 75% to 100%, more preferably 80% to 100%, more preferably 90% to 100%. Thus in one embodiment of the present invention the specificity of the invention is 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

The sensitivity of the method according to the present invention is preferably from 80% to 100%, more preferably 85% to 100%, more preferably 90% to 100%. Thus in one embodiment of the present invention the sensitivity of the invention is 80%, 81%, 82%, 83%, 84%, 85%, 86%, 87%, 88%, 89%, 90%, 91%, 92%, 93%, 94%, 95%, 96%, 97%, 98%, 99% or 100%.

It is to be understood that the markers according to the invention may be used in combinations in the methods according to the invention. Using several markers in combination is likely to increase the specificity and/or sensitivity of the assay as compared with an assay involving the use of a single marker. When several markers are used in combination it may therefore be acceptable that the specificity and sensitivity of each marker is lower than as specified above.

As an example illustrated in table 3 the gene CNRIP1 is methylated in all samples 20 of 20 from a colon cancer cell line (100%) and in 45 of 48 (94 %) adenoma samples - that is this gene has high sensitivity, the probability of detecting disease is 100 and 94 % from the respective samples. Whereas the methylation of the same genes in samples from normal tissue is 0 of 21 the gene has thus high specificity the chance of detecting false positive are 0 and all cancer free individuals are detected. Samples from normal mucoca from cancer patients showed methylation in 9 of 21 samples indicating that cancer can be detected in a distance from a tumour.

*Receiver-operating characteristics*

Accuracy of a diagnostic test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the reference level over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease, latent or recent infection versus no infection, or benign versus malignant disease.

In each case, the ROC plot depicts the overlap between the two distributions by plotting the sensitivity versus 1 - specificity for the complete range of decision thresholds. On the y-axis is the sensitivity, which is calculated entirely from the affected subgroup. On the x axis is the false-positive fraction, or 1 - specificity, which is calculated entirely from the unaffected subgroup.

Because the sensitivity and specificity are calculated entirely separately, by using the test results from two different subgroups, the ROC plot is independent of the prevalence of disease in the sample. Each point on the ROC plot represents a sensitivity/specificity pair corresponding to a particular decision threshold. A test with perfect discrimination (no overlap in the two distributions of results) has an ROC plot that passes through the upper left corner, where the true- positive fraction is 1.0, or 100% (perfect sensitivity), and the false- positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always $\geq 0.5$ (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the novel cancer marker genes may be assessed in comparison to and in combination with other markers for the given cancer e.g. clinical utility of the novel cancer markers CNRIP1, SPG20, FBN1, SNCA, INA and MAL assessed in comparison to: established diagnostic tools e.g. measuring the expression level of the corresponding or established methylation markers such as but not limited to ADAMTS1, VIM, SFRP1, SFRP2 and CRABP1.

*Risk assessment and cut-off*

To determine whether the subject is at increased risk of developing e.g. cancer, a cut-off limit for positive test must be established. This cut-off may be established by the laboratory, the physician or on a case by case basis by each subject.

Alternatively cut point can be determined as the mean, median or geometric mean of the negative control group (e.g. not having cancer) +/- one or more standard deviations or a value derived from the standard deviation)

The cut-off limit for positive test result according to the invention is the methylation state or level for which methylation is an indicator of cancer.

Another cut-off point may be the amount of CpG sites which needed to be methylated for a gene will be determined to be methylated.

The present inventors have successfully identified new markers for cancer. The methylation state of CpG sites or level of methylation in the promoter region of the nucleic acid sequence of a gene selected from CNRIP1, SPG20, FBN1, SNCA, INA and MAL is increased in subjects with cancer and thus these genes are efficient markers for detection of e.g. cancer.

Cut-off points can vary based on specific conditions of the individual tested such as but not limited to the risk of having the disease, occupation, geographic residence or exposure.

Cut-off points can vary based on specific conditions of the individual tested such as but not limited to age, sex, genetic background (i.e. HLA-type), acquired or inherited compromised immune function (e.g. HIV infection, diabetes, patients with renal or liver failure, patients in treatment with immune-modifying drugs such as but not limited to corticosteroids, chemotherapy, TNF-$\alpha$ blockers, mitosis inhibitors).

Doing adjustment of decision or cut-off limit will thus determine the test sensitivity for detecting cancer, if present, or its specificity for excluding cancer or disease if below this limit. Then the principle is that a value above the cut-off point indicates an increased risk and a value below the cut-off point indicates a reduced risk.

*Expression*

Several tumour suppressor genes have been identified to be inactivated by CpG island promoter methylation. As an example the *MLH1* gene in which hypermethylation of a limited number of CpG sites approximately 200 base pairs upstream of the transcription start point invariably correlates with the lack of gene expression.
The present analyses of cancer cell lines from several tissues indicate that the hypermethylation of a limited area in the proximity of the transcription start point of MAL is associated with reduced or lost gene expression. Quantitative gene expression results from colon cancer cell lines analyzed before and after epigenetic drug treatment indicate that this also holds true for SPG20, INA and CRNIP1
Thus measuring of methylation state or level and, in addition, the expression of the gene of interest increases the specificity and sensitivity of the method.

Methods

The present invention is based on the finding that genes, which are hypermethylated at an exceptionally high frequency in cancers such as colorectal cancer are found within a particular subset of 6 genes selected from the 21 genes previously discussed by Lind et al. These highly suitable hypermethylation markers include CNRIP1, SPG20, FBN1, SNCA, INA and MAL. The findings on e.g. MAL are contrary to previous reports where MAL hypermethylation was only seen at low frequency in colorectal cancers.
In a first aspect the present invention provides a method for determining whether a subject has developed is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

In one embodiment cancer is a tumour such as a tumour in the aero-digestive system (benign or malignant),
The method may further comprise the steps of:

b) comparing the methylation level, the number of methylated CpG sites or the methylation state of CpG sites to a reference; and

c) identifying said subject as being likely to develop, being developing or being predisposed for developing cancer, or relapsing after treatment of cancer, if the methylation level, the number of methylated CpG sites or the methylation state of CpG sites is higher than the methylation level, the number of methylated CpG sites or the methylation state of CpG sites of the reference and identifying a subject as unlikely to develop, being developing or being predisposed for developing cancer, or relapsing after treatment of cancer, if the methylation level, the number of methylated CpG sites or the methylation state of CpG sites is below the methylation level, the number of methylated CpG sites or the methylation state of CpG sites of said reference.

In another aspect the present invention provides a method for determining whether a subject has developed, is developing or is predisposed for developing cancer or whether a subject is relapsing after treatment of cancer, comprising the steps of

a) determining the methylation level, the number of methylated CpG sites or the methylation state of CpG sites in a sample from a subject

b) constructing a percentile plot of the methylation level, the number of methylated CpG sites or the methylation state of CpG sites of said at least one gene obtained from a sample from a healthy population;

c) constructing a ROC (receiver operating characteristics) curve based on the methylation level, the number of methylated CpG sites or the methylation state of CpG sites determined in the healthy population and on the methylation level, the number of methylated CpG sites or the methylation state of CpG sites determined in a population with cancer;

d) selecting from the ROC-curve the desired combination of sensitivity and specificity

e) determining from the percentile plot the methylation level, the number of methylated CpG sites or the methylation state of CpG sites corresponding to the determined or chosen specificity; and

f) predicting that the subject is likely to have cancer, if methylation level, the number of methylated CpG sites or the methylation state of CpG sites of said at least one gene in the sample is equal to or higher than said methylation level, the number of methylated CpG sites or the methylation state of CpG sites corresponding to the desired combination of sensitivity/specificity, and predicting that the subject is unlikely or not to have cancer, if the methylation level, the number of methylated CpG sites or the methylation state of CpG sites in the sample is lower than said methylation level, the number of methylated CpG sites or the methylation state of CpG sites corresponding to the desired combination of sensitivity/specificity.

In particular the method as described above comprises a method comprising determining methylation level, the number of methylated CpG sites or the methylation state of CpG sites in a nucleic acid sequence comprising a sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

The method as described above may also comprise determining the methylation state of CpG sites in a nucleic acid sequence of the additional genes selected from the group consisting of:

A) A nucleic acid sequence as defined by any of SEQ ID NO.: 1, SEQ ID NO.: 2, SEQ ID NO.: 3, SEQ ID NO.: 4, SEQ ID NO.: 5, SEQ ID NO.: 8, SEQ ID NO.: 10, SEQ ID NO.: 11, SEQ ID NO.: 12, SEQ ID NO.: 15 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

In another embodiment the method of the invention comprises determining the methylation state of CpG sites in the promoter region of MAL. According to this embodiment the nucleic acid sequence is

A) A nucleic acid sequence as defined by SEQ ID NO.: 1;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

Sequence identifiers 1-16 represent nucleic acids sequences of the above mentioned genes. As the person of skills in the art will realize, it is within the scope of the present invention to analyze the methylation state of CpG sites within these sequences as well as within their complementary sequences.
The following table lists the genes according to the invention, and corresponding id numbers, sequence identifiers and aliases

| HGNC name | Entrez | Ensemb | SEQ ID NO. | Aliases | Approved name |
|---|---|---|---|---|---|
| MAL | 4118 | ENSG00000172005 | 1-4 | | T-cell differentiation protein |

(continued)

| HGNC name | Entrez | Ensemb | SEQ ID NO. | Aliases | Approved name |
|---|---|---|---|---|---|
| FBN1 | 2200 | ENSG00000166147 | 6 | FBN; SGS; WMS; MASS; MFS1; OCTD | fibrillin 1 |
| CNRIP1 | 25927 | ENSG00000119865 | 7 | DKFZP566K1924, CRIP1, CRIP1a, CRIP1b, chromosome 2 open reading frame 32, C2orf32 | cannabinoid receptor interacting protein 1 |
| SPG20 | 23111 | ENSG00000133104 | 9 | SPARTIN; TAHCCP1; KIAA0610 | spastic paraplegia 20 |
| SNCA | 6622 | ENSG00000145335 | 13, 14 | NACP, PD1, PARK1, PARK4, MGC110988 | synuclein, alpha (non A4 component of amyloid precursor) |
| INA | 9118 | ENSG00000148798 | 16 | NEF5; NF-66; TXBP-1; MGC12702 | internexin neuronal intermediate filament protein, alpha |

The nucleic acid sequences according to the invention are listed in the sequence list. Each sequence comprises in the order of mentioning and in the 5' to 3' orientation: a consecutive sequence of nucleic acid residues located within the 1000 bp region upstream of the transcription start site (indicated in small letters) followed by a consecutive sequence of nucleic acid residues located downstream of the transcription start site (indicated in capitol letters) and by intronic sequence of nucleic acid residues (indicated in small letters)

The method of the invention may be directed against analyzing particular subsequences as suggested under item C) above. According to these embodiments, the sub-sequence in C) has a length of at least 8 nucleic acid residues, such as a length of at least 9 nucleic acid residues, at least 10 nucleic acid residues, at least 11 nucleic acid residues, at least 12 nucleic acid residues, at least 13 nucleic acid residues, at least 14 nucleic acid residues, at least 15 nucleic acid residues, at least 20 nucleic acid residues, at least 25 nucleic acid residues, at least 30 nucleic acid residues, at least 35 nucleic acid residues, at least 40 nucleic acid residues, at least 45 nucleic acid residues, at least 50 nucleic acid residues, at least 70 nucleic acid residues, or such as a length of at least 90 nucleic acid residues. It is generally desirable to direct the analyses against sequences of a certain length in order to ensure that the method is sufficiently sensitive.

For practical purposes it may also be desirable to minimize the length of the sub-sequences that are subject to the methylation studies in the method of the invention. Accordingly, it may be desirable that the said sub-sequence in C) has a length of at the most 10 nucleic acid residues, such as at the most 13 nucleic acid residues, at the most 14 nucleic acid residues, at the most 15 nucleic acid residues, at the most 20 nucleic acid residues, at the most 25 nucleic acid residues, at the most 30 nucleic acid residues, at the most 35 nucleic acid residues, at the most 40 nucleic acid residues, at the most 45 nucleic acid residues, at the most 50 nucleic acid residues, at the most 70 nucleic acid residues, at the most 90 nucleic acid residues, at the most 110 nucleic acid residues, at the most 150 nucleic acid residues, or such as at the most 200 nucleic acid residues.

More particularly it may be desirable that the sub-sequence in C) have a length of between 8 and 200 nucleic acid residues, such as a length between 8 and 150 nucleic acid residues, between 8 and 100 nucleic acid residues, between 8 and 75 nucleic acid residues, between 8 and 50 nucleic acid residues, between 9 and 200 nucleic acid residues, such as a length between 9 and 150 nucleic acid residues, between 9 and 100 nucleic acid residues, between 9 and 75 nucleic acid residues, between 9 and 50 nucleic acid residues, such as a length between 10 and 200 nucleic acid residues, between 10 and 150 nucleic acid residues, between 10 and 100 nucleic acid residues, between 10 and 75 nucleic acid residues, between 10 and 50 nucleic acid residues, such as a length between 11 and 200 nucleic acid residues, between 11 and 150 nucleic acid residues, between 11 and 100 nucleic acid residues, between 11 and 75 nucleic acid residues, between 11 and 50 nucleic acid residues, or such as a length between 12 and 200 nucleic acid residues, such as a length between 12 and 150 nucleic acid residues, between 12 and 100 nucleic acid residues, between 12 and 75 nucleic acid residues, or such as a length between 12 and 50 nucleic acid residues.

The promoter regions of the genes according to the invention are listed in the table below:

| HGNC name | Entrez | Ensemb | SEQ ID NO. |
|---|---|---|---|
| MAL | 4118 | ENSG00000172005 | 17-20 |
| FBN1 | 2200 | ENSG00000166147 | 22 |
| CNRIP1 | 25927 | ENSG00000119865 | 23 |
| SPG20 | 23111 | ENSG00000133104 | 25 |
| SNCA | 6622 | ENSG00000145335 | 29, 30 |
| INA | 9118 | ENSG00000148798 | 32 |

For each of the genes mentioned above, the inventors have identified subsequences that are particularly useful in the method of the invention. For MAL the sub-sequence in C) may, accordingly, be selected from the group of sequences consisting of the sequence specified by SEQ ID NO.: 17 and its complementary sequence, the sequence specified by SEQ ID NO.: 18 and its complementary sequence, the sequence specified by SEQ ID NO.: 19 and its complementary sequence, the sequence specified by SEQ ID NO.: 20 and its complementary sequence, and sub-sequences of any of these sequences.

For the fibrillin 1 gene the sub-sequence in C) is preferably the sequence specified by SEQ ID NO.: 22, or its complementary sequence, or a subsequence of one of these.

For the chromosome 2 open reading frame 32, (CNRIP1), the sub-sequence in C) is preferably the sequence specified by SEQ ID NO.:23, or its complementary sequence, or a subsequence of one of these.

For the spastic paraplegia 20, spartin (Troyer syndrome) the sub-sequence in C) is preferably the sequence specified by SEQ ID NO.:25, or its complementary sequence, or a subsequence of one of these.

For synuclein, alpha (non A4 component of amyloid precursor) the sub-sequence in C) are preferably selected from the group of sequences consisting of the sequence specified by SEQ ID NO.: 29 and its complementary sequence, the sequence specified by SEQ ID NO.: 30 and its complementary sequence, and subsequences of any of these sequences.

For internexin neuronal intermediate filament protein, alpha the sub-sequence in C) is preferably the sequence specified by SEQ ID NO.:32, or its complementary sequence, or a subsequence of one of these.

Also useful in the present invention may be the nucleic acids comprising the promoter region of additional genes selected from but not limited to the group of: myocyte enhancer factor 2C (SEQ ID NO.: 24), C3orf14/14HT021 (SEQ ID NO.:21), ubiquitin protein ligase E3A (SEQ ID NO.: 26, 27 and 28), brain expressed, X-linked 1 (SEQ ID NO.:31), or their complementary sequence, or a subsequence of one of these.

Hitherto few genes have proven useful for early detection of cancer based on methylation events in the promoter regions. It is however within the scope of the present invention to include in the method analyses of the methylation state or level in promoter regions of known markers for hypermethylation in cancer. In further embodiments of the invention, therefore, the method comprises determining the methylation state or level of CpG sites in the promoter region/sequence of one or more genes, said one or more genes being selected from the group consisting of:

Adam metallopeptidsase with thrombospondin type 1 motif (ADAMTS1, C3-C5, KIAA1346, METH1)
Vimentin (VIM)
Secreted Frizzled-related protein 1 (SFRP1); and
Secreted Frizzled-related protein 2 (SFRP2)

The promoter regions of these genes are represented by sequence identifiers 35-38. Accordingly, the method of the invention may comprise determining the methylation state of CpG sites in a nucleic acid sequence comprising a sequence selected from the group consisting of:

i) A nucleic acid sequence as defined by any of SEQ ID NO.: 33, SEQ ID NO.: 34, SEQ ID NO.: 35, and SEQ ID NO.: 36;
ii) A nucleic acid sequence which is complementary to a sequence as defined in i);
iii) A sub-sequence of a nucleic acid sequence as defined in i) or ii);
iv) A nucleic acid sequence which is at least 75%, such as at least 80%, at least 85%, at least 90%, at least 95%, at least 98% or at least 99%, identical to a sequence as defined in i), ii) or iii).

The skilled person will further realize that the various promoter regions will show some degree of degeneracy. Accordingly, as indicated under item D) above the promoter sequence for any of the particular genes may be one which is not entirely identical to one of the sequences represented by sequence identifiers 1-16. In particular embodiments the nucleic acid sequence in D) is at least 80% identical to a sequence as defined in A), B) or C), such as at least 85%, at least 90%, at least 95%, at least 98%, at least 99%, or such as at least 99.5% identical to a sequence as defined in A), B) or C).

The specificity and sensitivity of the genes according to the invention are very high and each of the genes may be comprised in the methods of the invention.

In one embodiment the method comprises determining the methylation level, the number of methylated CpG sites or the methylation state of CpG sites in a nucleic acid sequence in the promoter region in

I) a nucleic acid sequence comprising a sequence selected from the group consisting of: SEQ ID NO.s.: 1-4, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 6, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 7, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I).a nucleic acid sequence comprising a sequence consisting of: SEQ ID NO: 9, and its related sequences, and
II).in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence selected from the group consisting of: SEQ ID NO: 13-14, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 16, and its related sequences, and
II) in 1, 2 or 3 nucleic acid sequences as defined in the previous paragraph.

The methods according to the invention are aimed at detecting or diagnosing a cancer, such as a tumour, within the aero-digestive system..The "Aero-digestive system" or "aero-digestive tract" includes the lungs and the gastrointestinal tract: esophageus, stomach, pancreas, liver, gall bladder/bile duct, small bowel, and large intestine, including the colon and rectum. In particular, the tumour may be selected from the group consisting of: colorectal tumours, lung tumours (including small cell lung cancer and/or non-small cell lung cancer), esophageal tumours, gastric tumours, pancreas tumours, liver tumours, tumours of the gall bladder and/or bile duct, tumours of the small bowel and tumours of the large bowel

Thus in one embodiment of the invention cancer is selected from the group consisting of: colorectal tumours, lung tumours (including small cell lung cancer and/or non-small cell lung cancer), esophageal tumours, gastric tumours, pancreas tumours, liver tumours, tumours of the gall bladder and/or bile duct, tumours of the small bowel and tumours of the large bowel.

In order to determine the number of methylated CpG sites, the methylation state of the CpG sites or the methylation level in said promoter region/sequence, the method according to the invention requires that a sufficient amount of DNA be isolated from the particular subject. The skilled artisan will know of suitable techniques for isolating and purifying DNA in the amounts and quality required. For most purposes DNA may be isolated from a blood sample, a fecal sample, a tissue sample or a sample of mucus from the lungs from said subject. In general, it is desirable to perform the method according to the invention in a non-invasive manner whenever this is possible: For gastro intestinal cancers collecting DNA from faecal samples will often be practical and convenient. In relation to lung tumours isolating DNA from mucus samples from the lung may offer a convenient approach to non-invasive collection of DNA. For other tumours, including tumours in the liver and pancreas, it may be preferred to collect tissue samples for subsequent isolation of DNA.

Thus in one embodiment the sample is obtained from blood, stool, urine, pleural fluid, gall, bronchial fluid, oral washings, tissue biopsies, ascites, pus, cerebrospinal fluid, aspitate, follicular fluid, tissue or mucus.

When the methods according to the invention is used for the purpose of merely determining the presence of cancer or a tumour in the aero-digestive system, in particular where a "yes/no"- type of result is required. It is desirable if the method can be limited to analyzing the methylation level or methylation state of CpG sites in the promoter regions of 2-4 genes. This clearly requires that the genes have an extremely high frequency of hypermethylation during cancer development and progression.

Thus for simple diagnostic purposes it is mostly preferred to limit the analysis to the promoter regions within very few genes. As mentioned above this requires the availability of a panel of markers for hypermethylation, wherein each marker has a high sensitivity and specificity. For other more subtle purposes, however, it may be necessary to analyze the methylation state or level of promoter regions in a larger number of marker genes. The methods according to the invention may therefore comprise determining the methylation state of CpG sites in a nucleic acid sequence in the promoter region/sequence of at least 2 genes, such as at least 3 genes, such as at least 4 genes, such as at least 5 genes, such as at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes or at least 20 genes, including at least 1 gene as defined in claim 1 in order to determine the risk level for tumour initiation and/or progression in a subject.

In accordance with what is explained above the method of the invention further comprising determining the methylation state of CpG sites in at least one such as at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or such as at least 20, additional nucleic acid sequences as defined above or their related sequences.

Thus for most purposes it will be insufficient to analyze the methylation level, the number of methylated CpG sites or the methylation state of CpG sites of the promoter region of a single gene. The methods according to the invention may therefore comprise determining the methylation state of CpG sites in a nucleic acid sequence in the promoter region/sequence of at least 2 genes, such as at least 3 genes, such as at least 4 genes, such as at least 5 genes, such as at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes or at least 20 genes, wherein at least one gene is selected from the group of genes defined above

Thus in another aspect the invention concern a method wherein the methylation level, the number of methylated CpG sites or the methylation state of CpG sites of at least one additional marker is determined.

Wherein at least one additional marker is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118
MAL, e.g. as identified by ensembl gene id ENSG00000172005

*Combination of maker genes*

Thus for the reasons explained above the methylation level or methylation state may be combined with measurements of one or more other markers, and compared to a combined reference-level. The measured marker levels can be combined by arithmetic operations such as addition, subtraction, multiplication and arithmetic manipulations of percentages, square root, exponentiation, and logarithmic functions. Levels can also be combined following manipulations using various models e.g. logistic regression and maximum likelihood estimates. Various biomarker combinations and various means of calculating the combined referencevalue can be performed by means known to the skilled addressee.

Thus, another embodiment of the invention concerns a method according to any of the proceeding claims, where the methylation level or methylation state of at least one additional marker is determined.

The at least one additional marker may be but are not limited to CNRIP1, SPG20, FBN1, SNCA, INA, MAL, ADAMTS1, VIM, SFRP1 or SFRP2, CRABP1. The markers can be compared to a set of reference data to determine whether the subject has cancer or is at increased risk of developing cancer.

A method of constructing a diagnostic test based on a combined marker may be achieved by combining the methylation levels or methylation state (or a value derived hereof) of two or more individual markers by arithmetic manipulation (e.g. addition). As there may be variety in methylation level or methylation state of the different markers it is relevant to weigh the measurements in order for a combination to be achieved independent of differences in e.g. the level or state of methylation. This can be done by simple normalization from a median or mean from a standard material

*Synergy*

By combination of the different marker genes according to the invention a synergistic effect may be achieved.
Specifically as used herein synergy refers to the phenomenon in which several markers acting together creates a "combined marker signal" with greater sensitivity or specificity for diagnosis, than that predicted by knowing only the separate markers sensitivity or specificity.
Thus in one embodiment of the present invention the combined use of at least one additional the marker (e.g. CNRIP1, SPG20, FBN1, SNCA, INA, MAL) provides a synergistic effect in relation to sensitivity and/or specificity.
In another embodiment of the present invention the combined use of the markers CNRIP1 and INA provides a synergistic effect in relation to sensitivity and/or specificity.
In another embodiment of the present invention the combined use of the markers CNRIP1 and SNCA provides a synergistic effect in relation to sensitivity and/or specificity.
In another embodiment of the present invention the combined use of the markers CNRIP1 and FBN1 provides a synergistic effect in relation to sensitivity and/or specificity.
In another embodiment of the present invention the combined use of the markers CNRIP1 and SPG20 provides a synergistic effect in relation to sensitivity and/or specificity.
In another embodiment of the present invention the combined use of the markers INA and SNCA provides a synergistic effect in relation to sensitivity and/or specificity.
In another embodiment of the present invention the combined use of the markers INA and FBN1 provides a synergistic effect in relation to sensitivity and/or specificity.
In another embodiment of the present invention the combined use of the markers INA and SPG20 provides a synergistic effect in relation to sensitivity and/or specificity.
In another embodiment of the present invention the combined use of the markers SNCA and FBN1 provides a synergistic effect in relation to sensitivity and/or specificity.
In another embodiment of the present invention the combined use of the markers SNCA and SPG20 provides a synergistic effect in relation to sensitivity and/or specificity.
In another embodiment of the present invention the combined use of the markers FBN1 and SPG20 provides a synergistic effect in relation to sensitivity and/or specificity.
In another embodiment of the present invention the combined use of the markers CNRIP1 and MAL provides a synergistic effect in relation to sensitivity and/or specificity.
In another embodiment of the present invention the combined use of the markers SPG20 and MAL provides a synergistic effect in relation to sensitivity and/or specificity.
In another embodiment of the present invention the combined use of the markers FBN1 and MAL provides a synergistic effect in relation to sensitivity and/or specificity.
In another embodiment of the present invention the combined use of the markers SNCA and MAL provides a synergistic effect in relation to sensitivity and/or specificity.
In another embodiment of the present invention the combined use of the markers INA and MAL provides a synergistic effect in relation to sensitivity and/or specificity.
In another embodiment of the present invention the combined use of the markers CNRIP1, SPG20 and INA provides a synergistic effect in relation to sensitivity and/or specificity.
In another embodiment of the present invention the combined use of the markers CNRIP1, SPG20 and FBN1 provides a synergistic effect in relation to sensitivity and/or specificity.
In another embodiment of the present invention the combined use of the markers CNRIP1, SPG20 and SNCA provides a synergistic effect in relation to sensitivity and/or specificity.
In another embodiment of the present invention the combined use of the markers CNRIP1, INA and SNCA provides a synergistic effect in relation to sensitivity and/or specificity.
In another embodiment of the present invention the combined use of the markers CNRIP1, INA and FBN1 provides a synergistic effect in relation to sensitivity and/or specificity.
In another embodiment of the present invention the combined use of the markers CNRIP1, SNCA and FBN1 provides a synergistic effect in relation to sensitivity and/or specificity.
In another embodiment of the present invention the combined use of the markers SNCA, SPG20 and FBN1 provides a synergistic effect in relation to sensitivity and/or specificity.

In another embodiment of the present invention the combined use of the markers INA, SPG20 and FBN1 provides a synergistic effect in relation to sensitivity and/or specificity.

In another embodiment of the present invention the combined use of the markers INA, SNCA and FBN1 provides a synergistic effect in relation to sensitivity and/or specificity.

In another embodiment of the present invention the combined use of the markers INA, SPG20 and SNCA provides a synergistic effect in relation to sensitivity and/or specificity.

In another embodiment of the present invention the combined use of the markers MAL, SPG20 and SNCA provides a synergistic effect in relation to sensitivity and/or specificity.

In another embodiment of the present invention the combined use of the markers MAL, INA and SNCA provides a synergistic effect in relation to sensitivity and/or specificity

In another embodiment of the present invention the combined use of the markers MAL, INA and SPG20 provides a synergistic effect in relation to sensitivity and/or specificity

In another embodiment of the present invention the combined use of the markers MAL, FBN1 and SNCA provides a synergistic effect in relation to sensitivity and/or specificity

In another embodiment of the present invention the combined use of the markers MAL, FBN1 and SPG20 provides a synergistic effect in relation to sensitivity and/or specificity

In another embodiment of the present invention the combined use of the markers MAL, FBN1 and INA provides a synergistic effect in relation to sensitivity and/or specificity

In another embodiment of the present invention the combined use of the markers MAL, FBN1 and CNRIP1 provides a synergistic effect in relation to sensitivity and/or specificity

In another embodiment of the present invention the combined use of the markers MAL, CNRIP1 and SNCA provides a synergistic effect in relation to sensitivity and/or specificity

In another embodiment of the present invention the combined use of the markers MAL, CNRIP1 and SPG20 provides a synergistic effect in relation to sensitivity and/or specificity

In another embodiment of the present invention the combined use of the markers MAL, CNRIP1 and INA provides a synergistic effect in relation to sensitivity and/or specificity

In another embodiment of the present invention the combined use of the markers CNRIP1, FBN1, SNCA, and INA provides a synergistic effect in relation to sensitivity and/or specificity.

In another embodiment of the present invention the combined use of the markers SPG20, FBN1, SNCA, and INA provides a synergistic effect in relation to sensitivity and/or specificity.

In another embodiment of the present invention the combined use of the markers CNRIP1, SNCA, INA and SPG20 provides a synergistic effect in relation to sensitivity and/or specificity.

In another embodiment of the present invention the combined use of the markers CNRIP1, FBN1, INA and SPG20 provides a synergistic effect in relation to sensitivity and/or specificity.

In another embodiment of the present invention the combined use of the markers CNRIP1, FBN1, SNCA and SPG20 provides a synergistic effect in relation to sensitivity and/or specificity.

In another embodiment of the present invention the combined use of the markers MAL, FBN1, SNCA and SPG20 provides a synergistic effect in relation to sensitivity and/or specificity.

In another embodiment of the present invention the combined use of the markers CNRIP1, MAL, SNCA and SPG20 provides a synergistic effect in relation to sensitivity and/or specificity.

In another embodiment of the present invention the combined use of the markers CNRIP1, FBN1, MAL and SPG20 provides a synergistic effect in relation to sensitivity and/or specificity.

In another embodiment of the present invention the combined use of the markers CNRIP1, FBN1, SNCA and MAL provides a synergistic effect in relation to sensitivity and/or specificity.

In another embodiment of the present invention the combined use of the markers INA, FBN1, SNCA and MAL provides a synergistic effect in relation to sensitivity and/or specificity.

In another embodiment of the present invention the combined use of the markers CNRIP1, INA, SNCA and MAL provides a synergistic effect in relation to sensitivity and/or specificity.

In another embodiment of the present invention the combined use of the markers CNRIP1, FBN1, INA and MAL provides a synergistic effect in relation to sensitivity and/or specificity.

In another embodiment of the present invention the combined use of the markers CNRIP1, SPG20, FBN1, SNCA, and INA provides a synergistic effect in relation to sensitivity and/or specificity.

In another embodiment of the present invention the combined use of the markers MAL, SPG20, FBN1, SNCA, and INA provides a synergistic effect in relation to sensitivity and/or specificity.

In another embodiment of the present invention the combined use of the markers CNRIP1, MAL, FBN1, SNCA, and INA provides a synergistic effect in relation to sensitivity and/or specificity.

In another embodiment of the present invention the combined use of the markers CNRIP1, SPG20, MAL, SNCA, and INA provides a synergistic effect in relation to sensitivity and/or specificity.

In another embodiment of the present invention the combined use of the markers CNRIP1, SPG20, FBN1, MAL, and INA provides a synergistic effect in relation to sensitivity and/or specificity.

In another embodiment of the present invention the combined use of the markers CNRIP1, SPG20, FBN1, SNCA, and MAL provides a synergistic effect in relation to sensitivity and/or specificity.

Thus in one embodiment the methods according to the invention comprises determining the methylation level, the number of methylated CpG sites or the methylation state of CpG sites of CNRIP1 combined with determining the methylation level, the number of methylated CpG sites or the methylation state of CpG sites of at least one additional marker selected from the group comprising:

SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Thus in one embodiment the methods according to the invention comprises determining the methylation level, the number of methylated CpG sites or the methylation state of CpG sites of SPG20 combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Thus in one embodiment the methods according to the invention comprises determining the methylation level, the number of methylated CpG sites or the methylation state of CpG sites of FBN1 combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111
SNCA, e.g. as identified by ensembl gene id ENSG00000145335, entrez id 6622; and
INA, e.g. as identified by ensembl gene id ENSG00000148798, entrez id 9118

Thus in one embodiment the methods according to the invention comprises determining methylation level, the number of methylated CpG sites or the methylation state of CpG sites of SNCA combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200; and
INA, e.g. as identified by ensembl gene id ENSG00000148798, entrez id 9118

Thus in one embodiment the methods according to the invention comprises determining methylation level, the number of methylated CpG sites or the methylation state of CpG sites of INA combined with determining methylation level, the number of methylated CpG sites or the methylation state of CpG sites of at least one additional marker selected from the group comprising:

CNRIP1, e.g. as identified by ensembl gene id ENSG00000119865, entrez id 25927;
SPG20, e.g. as identified by ensembl gene id ENSG00000133104, entrez id 23111;
FBN1, e.g. as identified by ensembl gene id ENSG00000166147, entrez id 2200; and
SNCA, e.g. as identified by ensembl gene id ENSG00000145335, entrez id 6622

*Bisulphite treatment and methylation-specific polymerase chain reaction*

The invention is not limited by the types of assays used to assess methylation state of the members of the gene or gene panel. Indeed, any assay that can be employed to determine the methylation state or level of the gene or gene panel should suffice for the purposes of the present invention.

A practical approach to determining the methylation state of CpG islands in a promoter region may comprise a step of treating the promoter sequence with bisulphite. Bisulphite treatment of DNA leads to sequence variations as unmethylated but not methylated cytosines are converted to uracil. Bisulphite treatment followed by sequence analyses allows a positive display of 5-methyl cytosines in the gene promoter after bisulphite modification as unmethylated cytosines appear as thymidines, whereas 5-methyl cytosines appear as cytosines in the final sequence. In particular embodiments of the invention the methylation state of said promoter region/sequence is therefore determined by nucleic acid sequencing (bisulphite sequencing).

In further embodiments of the invention, the number of methylated CpG sites, the methylation state of CpG sites or the methylation level of said promoter region/sequence is determined by methylation specific PCR. In the examples of the present application a set of suitable PCR conditions and primer designs is given. In general, however, the skilled person will have the knowledge required in order for him to be able to determine appropriate conditions and primer designs for PCR analyses.

As the skilled person will know real-time fluorescence offers a convenient and rapid approach to the detection of PCR products and may readily be applied in diagnostic procedures where a high throughput is required. In currently preferred embodiment of the invention said methylation specific PCR thus comprises real-time fluorescence detection of the PCR products.

As for most other PCR procedures the method of the invention may comprise a step of separating the products according to size. In particular, the methods of the invention may comprise a step of separating the resulting PCR products by gel- or capillary electrophoresis.

As part of the analyses the resulting PCR products may detected by the use of a label selected from the group consisting of fluorescent labels, chemiluminescent label and radioactive labels. For safety and practical reasons non-radioactive labels are preferred for most purposes.

The methylation state or level of said promoter region/sequence may also be determined by pyrosequencing, mass spectrometry or by use of methylation specific restriction enzymes.

The methylation level, the number of methylated CpG sites or the methylation state of CpG sites is determined by, but are not limited to, bisulphite sequencing, quantitative and/or qualitative methylation specific polymerase chain reaction (MSP), pyrosequencing, Southern blotting, restriction landmark genome scanning (RLGS), single nucleotide primer extension, CpG island microarray, SNUPE, COBRA, mass spectrometry, by use of methylation specific restriction enzymes, by measuring the expression level of said genes or a combination thereof.

In preferred embodiments the methylation specific PCR used in the method of the invention comprises the use of nucleic acid primers which are capable of hybridizing to a nucleic acid sequence comprising 2 CpG sites and a cytosine residue which is not within a CpG site. The inclusion of such a cytosine residue which is not methylated, is desired in order to better distinguish bisulphite converted DNA from non-bisulphite converted DNA. Primers for methylated sequences will always bind to methylated CpG sites, which are sites that remain CpG after bisulphite conversion. In the presence of unconverted DNA this will contain CpG sites independently of methylation status and the methylation specific primers will then bind to the unconverted DNA, creating false positives. The inclusion of a "C" which is not in a CpG site in the area targeted by the primer will prevent the primer from binding to un-converted DNA as this DNA will contain "C" while the converted DNA will contain "T" at the same site.

In still further embodiments the methylation specific PCR comprises the use of nucleic acid primers which are capable of hybridizing to a nucleic acid sequence comprising 2 CpG sites and a cytosine residue which is not within a CpG site.

The methods according to the invention can be combined with any other known parameter for cancer. Thus the methylation level or state of a gene of the invention may be combined with but not limited to any of the following parameters for cancer: a genetic DNA integrity assay, ploidi, mutation status of genes, genomic changes, fusions genes, splice variants, differences in expression, miRNAs.

Use

The markers according to the invention are due to their high sensitivity and specificity very suitable for use as markers for cancer. Thus another aspect of the invention concern the use of one or more genes selected from the group comprising of

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

Another embodiment concerns the use of a nucleic acid sequence, wherein said nucleic acid comprises a nucleic acid sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), A) or C).

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

Antibody

The invention also concerns an antibody for the methylated sequences. Thus another embodiment of the invention concerns. An antibody recognizing a methylated nucleic acid sequences selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in a);
C) A sub-sequence of a nucleic acid sequence as defined in a) or b);
D) A nucleic acid sequence which is at least 75% identical to a sequence as defined in A), B) or C).

Diagnostic kit

In a preferred aspect of the invention provides a diagnostic kit for the determination cancer comprising one or more oligonucleotide primers or one or more sets of oligonucleotide primers, which are each complementary to a nucleic acid sequence of the genes selected from:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

A second aspect of the invention provides a diagnostic kit comprising one or more oligonucleotide primers or one or more sets of oligonucleotide primers, such as 2 or more, 3 or more, 4 or more, 5 or more, 6 or more, 7 or more, 8 or more, 9 or more, 10 or more, 11 or more, 12 or more, 13 or more, 14 or more, 15 or more 16 or more, 17 or more, 18 or more, 19 or more or such as 20 or more oligonucleotide primers or sets of oligonucleotide primers which are each complementary to/capable of hybridizing to a nucleic acid sequence in the promoter region/sequence of one or more genes, said one or more genes being selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ensembl gene id ENSG00000148798, entrez id 9118

In particular, the kit according to this aspect of the invention comprises one or more oligonucleotide primers or sets of oligonucleotide primers which are each complementary to/capable of hybridizing to a nucleic acid sequence comprising a sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;

B) A nucleic acid sequence which is complementary to a sequence as defined in A);

C) A sub-sequence of a nucleic acid sequence as defined in A) or B); A nucleic acid sequence which is at least 75% identical to a sequence as defined in A), B) or C).

In particular embodiments the kit comprises one or more oligonucleotide primers or one or more sets of oligonucleotide primers which are each complementary to/capable of hybridizing to a nucleic acid sequence in the promoter region/sequence of a gene being selected from the group consisting of:

Adam metallopeptidsase with thrombospondin type 1 motif (ADAMTS1, C3-C5, KIAA1346, METH1)
Vimentin (VIM)
Secreted Frizzled-related protein 1 (SFRP1); and
Secreted Frizzled-related protein 2 (SFRP2)
MAL (T cell differentiation protein)
chromosome 3 open reading frame (C3orf14/14HT021),
ubiquitin protein ligase E3A (UBE3A, AS, ANCR, E6-AP, FU26981),
brain expressed, X-linked 1 (BEX1),
myocyte enhancer factor 2C, MEF2c

In another aspect of the invention, the kit comprises one or more oligonucleotide primers or sets of oligonucleotide primers which are each complementary to/capable of hybridizing to a nucleic acid sequence comprising a sequence selected from the group consisting of:

A) A nucleic acid sequence as defined by any of SEQ ID NO.: 1, SEQ ID NO.: 2, SEQ ID NO.: 3, SEQ ID NO.: 4, SEQ ID NO.: 5, SEQ ID NO.: 8, SEQ ID NO.: 10, SEQ ID NO.: 11, SEQ ID NO.: 12 and SEQ ID NO.: 15

B) A nucleic acid sequence which is complementary to a sequence as defined in A);

C) A sub-sequence of a nucleic acid sequence as defined in A) or B);

D) A nucleic acid sequence which is at least 75% identical to a sequence as defined in A), B) or C).

According to these embodiments the kit comprises one or more oligonucleotide primers or one or more sets of oligonucleotide primers which are each complementary to/capable of hybridizing to a nucleic acid sequence comprising a sequence selected from the group consisting of:

i) A nucleic acid sequence as defined by any of SEQ ID NO.: 33, SEQ ID NO.: 34, SEQ ID NO.: 35, and SEQ ID NO.: 36;

ii)A nucleic acid sequence which is complementary to a sequence as defined in i);

iii) A sub-sequence of a nucleic acid sequence as defined in i) or ii);

iv) A nucleic acid sequence which is at least 75%, such as at least 80%, at least 85%, at least 90%, at least 95%, at least 98% or at least 99%, identical to a sequence as defined in i), ii) or iii).

In view of the extremely high specificity of MAL as a marker for cancer the kit comprises one or more oligonucleotide primers or one or more sets of oligonucleotide primers which are each complementary to/capable of hybridizing to a nucleic acid sequence selected from the group consisting of

A) A nucleic acid sequence as defined by any of SEQ ID NO.: 1;

B) A nucleic acid sequence which is complementary to a sequence as defined in A);

C) A sub-sequence of a nucleic acid sequence as defined in A) or B);

D) A nucleic acid sequence which is at least 75% identical to a sequence as defined in A), B) or C).

Various designs may be contemplated for the kit of the invention. In one embodiment each of the said primers or sets of primers are in separate containers. This will allow the end user of the kit to prepare different primer mixtures for different purposes. According to other embodiments, however, the primers or sets of primers may be supplied in a mixture.

For certain use, such as in traditional diagnostic purposes, the diagnostic kit may include few primers or sets of primers. In particular, this is relevant when the kit is to be used in applications where a "yes/no"- type of result is required, such as when the kit is used simply in order to determine whether a tumour or carcinoma is developing in the aero-digestive system. For such purposes the number of primers or sets of primers in the kit may be limited to 2 to 4, wherein at least one primer or one set of primers, such as at least 2, at least 3 or at least 4 primers or sets of primers, is complementary to/capable of hybridizing to a nucleic acid sequence according to SEQ ID NO.: 1-16 or sequences that are complementary or partly identical thereto as defined above under items C) and D).

As discussed above, however, in relation to the method of the invention, the methylation state of CpG islands of the marker genes of the invention may also be used for more complex analyses, as in order to determine the risk level for tumour initiation and/or progression in a subject.

In such embodiments the diagnostic kit of the invention will typically contain primers or sets of primers that are able to target a larger number of marker genes. A kit for such purposes will typically need to include 5 or more primers or sets of primers, wherein at least one primer or one set of primers, such as at least 2, at least 3, at least 4, at least 5, at least 6, at least 7, at least 8, at least 9, at least 10, at least 12, at least 13, at least 14, at least 15, or such as at least 16 primers or one set of primers, is complementary to/capable of hybridizing to a nucleic acid sequence of a marker gene according to the invention.

The diagnostic kit according to the invention may further comprise any reagent or media needed in order to perform the required analyses, such as PCR analyses, such as specific polymerase chain reaction (MSP) sequence analyses, bisulphite treatment, bisulphate sequencing, electrophoresis, pyrosequencing, mass spectrometry and sequence analyses by restriction digestion, quantitative and/or qualitative methylation, pyrosequencing, Southern blotting, restriction landmark genome scanning (RLGS), single nucleotide primer extension, CpG island microarray, SNUPE, COBRA, mass spectrometry, by use of methylation specific restriction enzymes or by measuring the expression level of said genes. In particular, the kit may further comprise one or more components selected from the group consisting of: deoxyribonucleoside triphosphates, buffers, stabilizers, thermostable DNA polymerases, restriction endonucleases (including methylation specific endonucleases), and labels (including fluorescent, chemiluminescent and radioactive labels). The diagnostic assay according to the invention may further comprise one or more reagents required for isolation of DNA.

It should be noted that embodiments and features described in the context of one of the aspects of the present invention also apply to the other aspects of the invention.

When an object according to the present invention or one of its features or characteristics is referred to in singular this also refers to the object or its features or characteristics in plural. As an example, when referring to "a polypeptide" it is to be understood as referring to one or more polypeptides.

Throughout the present specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

All patent and non-patent references cited in the present application, are hereby incorporated by reference in their entirety.

The invention will now be described in further details in the following non-limiting examples.

## Examples

Example 1:

*Bisulphite treatment and methylation-specific PCR*

DNA from cell lines and colorectal carcinomas was bisulphite treated as previously described (Grunau et al. and Fraga et al.). Whereas DNA from the adenomas was bisulphite treated according to the protocol of the CpGenome™ DNA modification kit (Intergen Boston, MA) (Smith-Sørensen et al.). The promoter methylation status of MAL, C3orf14, FBN1, MEF2c, CNRIP1, SPG20, UBE3A, SNCA, BEX and INA was subsequently analyzed by methylation-specific PCR (MSP), a method allowing for distinction between unmethylated and methylated alleles (Herman et al. and Derks et al.). All primers were designed with MethPrimer (Li and Dahiya) or Methyl Primer Express (Applied Biosystems). Their sequences are listed in Table 1, along with the product fragment length, primer location, and annealing temperature for each PCR. The fragments were amplified using the HotStarTaq DNA Polymerase (QIAGEN Inc., Valencia, CA), and all results were confirmed with a second independent round of MSP.

Table 1: Primers for methylation-specific PCR

| Gene | Methylated sequence (forward/reverse primer sequence) | SEQ ID NO. | Unmethylated sequence (forward/reverse primer sequence) | SEQ ID NO. |
|---|---|---|---|---|
| *MAL* | TTCGGGTTTTTTTGTTTTTAATTC/<br>GAAAACCATAACGACGTACTAACG<br>T | 37/ 38 | TTTTGGGTTTTTTTGTTTTTAATTT/<br>ACAAAAACCATAACAACATACTAACATC | 39/ 40 |
| *C3orf14* | GTAATTTAGATTTCGGAGGGC/<br>CGACCAAAAAAAACGAAAA | 41/ 42 | TTTGTAATTTAGATTTTGGAGGGT/<br>CCAACCAAAAAAAACAAAAACA | 43/ 44 |
| *FBN1* | GTATTTTTTTTCGCGAGAAATC/<br>AATCGTAACCGCTACAACC | 45/ 46 | AAAGTATTTTTTTTGTGAGAAATT/<br>CCCAATCATAACCACTACAACC | 47/ 48 |
| *MEF2c* | GTTATTTTTAATTCGATCGGTC/<br>AAACCGCTCGAAAAAAAA | 49/ 50 | TTGGTTATTTTTAATTTGATTGGTT/<br>CCAAAACCACTCAAAAAAAAA | 51/ 52 |
| *CNRIP1* | TCGTTTTTTGGTATAGTGGTC/<br>CAAATCCGCGCAACTAAA | 53/ 54 | GTTTTGTTTTTTGGTATAGTGGTT/<br>CAAATCCACACAACTAAAAAC | 55/ 56 |
| *SPG20* | TGGAACGTTTTGGTTGTTAC/<br>TACCTCGAAAACTCCCTACG | 57/ 58 | GTGGAATGTTTTGGTTGTTAT/<br>TTACCTCAAAAACTCCCTACA | 59/ 60 |
| *UBE3A* | CGTTGTTTGTCGGGATATTC/<br>CCCGTCGTCTCCTATAATCA | 61/ 62 | GTGTTGTTTGTTGGGATATTT/<br>CCCCATCATCTCCTATAATCA | 63/ 64 |
| *SNCA* | CGGGTTGTAGCGTAGATTTC/<br>CGTCGAATAACCACTCCC | 65/ 66 | GTGTGGGTTGTAGTGTAGATTTT/<br>TCATCAAATAACCACTCCCAA | 67/ 68 |
| *BEX1* | AGTTAATTGGTCGTCGGTTC/<br>CGAATAACGACTACACCGAA | 69/ 70 | ATTAGTTAATTGGTTGTTGGTTT/<br>ACACAAATAACAACTACACCAAA | 71/ 72 |

(continued)

| Gene | Methylated sequence (forward/reverse primer sequence) | SEQ ID NO. | Unmethylated sequence (forward/reverse primer sequence) | SEQ ID NO. |
|---|---|---|---|---|
| *INA* | AGGAGTTTCGTTTTTAGCGC/<br>ACGACTTCAACGCGAACTAC | 73 /74 | AGTAGGAGTTTTGTTTTTAGTGT/<br>ACAACTTCAACACAAACTACAAA | 75/ 76 |

*Bisulphite sequencing*

All fragments were amplified with the HotStarTaq DNA Polymerase and eluted from a 2% agarose gel by the MinElute™Gel Extraction kit (QIAGEN). The samples were subsequently sequenced using the dGTP BigDye Terminator Cycle Sequencing Ready Reaction kit (Applied Biosystems, Foster City, CA) in a 3730 Sequencer (Applied Biosystems). The approximate amount of methyl cytosine of each CpG site in the various fragments was calculated by comparing the peak height of the cytosine signal with the sum of the cytosine and thymine peak height signals, as previously described by Melki, et al.

Table 2: Primers for bisulphite sequencing.

| Gene | Primer sequence (forward/reverse) | SEQ ID NO. |
|---|---|---|
| *MAL* | GGGTTTTTTTGTTTTTAATT/ACCAAAAACCACTCACAAACTC | 77/78 |
| *C3orf14* | GGAGGGTAGATGATTTTGAGAA/CTTCCCCTTCCCCTAACTACTA | 79/80 |
| *FBN1* | AGGGGGTGTTATTTTTTTTTTTTT/CCCAATCCCTATCCCTACC | 81/82 |
| *MEF2c* | TTTTTGGAYGAGTTTGGTTATT/CCACCTAATTCAAACATACAACC | 83/84 |
| *CNRIP1* | TTTTAYGTAGTTGGTYGAGG/CTCCTTAAACTATAACCCCCCT | 85/86 |
| *SPG20* | ATTTAGTTTGAGTAGGTYGGTG/CTCCATCCTAACAATCCATAAA | 87/88 |
| *UBE3A* | GGGGGGTGTTTAGAGGG/CCTCCTACCAAAAACTACAAACC | 89/90 |
| *SNCA* | AGAAGGGGTTTAAGAGAGG/ACTATCCCCAAAAAAAAACC | 91/92 |
| *BEX1* | ATTTGTGGGTTTTTAGATTGGA/CCAAAAAACCACTATATTCCCA | 93/94 |
| *INA* | GATGTAGATGGTTTTGTTTYGG/CAAACRAAAACCATCCCC | 95/96 |

When analyzed by methylation-specific polymerase chain reaction (MSP) analysis hypermethylation of MAL was observed in an exceptionally high frequency among malignant (83%, 40 of 48 carcinomas) as well as in benign large bowel tumours (73%, 43 of 59 adenomas) (Figure 1).

Example 2

*Methylation-specific polymerase chain reaction (MSP) was performed for genes: MAL, C3orf14, FBN1, SPG20, SNCA, BEX1, INA, CNRIP1, UBE3A, MEF2C*

For each sample (colon cancer cell lines, colorectal carcinomas, adenomas and normal mucosa), 1.3ug DNA was bisulphite treated using the EpiTect bisulphite kit (Qiagen Inc., Valencia, CA) following the manufacturers protocol. The modified DNA was eluted in 40ul eluation buffer (included in the kit). Since bisulphite modification leads to sequence differences, two pairs of primers were used to amplify each gene (see primer list in Example 1), one specific for unmethylated template and the other specific for methylated template. The 25 μl PCR mixture contained 1 x PCR buffer, 0.75ul bisulphite treated template, 1.5-2.0 mM $MgCl_2$, 20 pmol of each primer, 200 μM dNTP, and 0.625-1U HotStarTaq DNA Polymerase (Qiagen). Human placental DNA (Sigma Chemical Co, St. Louis, MO, USA) treated *in vitro* with *SssI* methyltransferase (New England Biolabs Inc., Beverly, MA, USA) was used as a positive control for the methylated MSP reaction, whereas DNA from normal lymphocytes was used as a positive control for unmethylated alleles. Water was used as a negative PCR control in both reactions.

The PCR program consisted of 15 min denaturation at 95°C, followed by 35 cycles of 30sek at 95°C, 30sek at annealing temperature, and 30sek at 72°C. A final elongation was performed at 72°C in 7minutes.

Annealing temperature and $MgCl_2$ content for the respective genes tested so far:

MAL: 56°C, 1.5mM $MgCl_2$
C3orf14: 53°C, 1.5mM $MgCl_2$
FBN1: 48°C, 1.7 mM $MgCl_2$ for unmethylated reaction and 2.0 for methylated reaction
SPG20: 56°C, 1.5mM $MgCl_2$
SNCA: 53°C, 1.5mM $MgCl_2$
BEX1: 51°C, 1.5mM $MgCl_2$
INA: 55°C, 1.5mM $MgCl_2$

CNRIP1: 52°C, 1.5mM $MgCl_2$

Results: Methylation of MAL, UBE3A, MEF2C, FBN1, C3orf14, BEX1, INA, SNCA, SPG20, and CNRIP1

The results obtained in methylation-specific polymerase chain reaction (MSP) are presented in table 3 below:

| | MAL | UBE3A | MEF2C | FBN1 | c3orf14 |
|---|---|---|---|---|---|
| **Colon cancer cell lines** | 19/20 (95%) | 0/20 (0%) | 4/20 (20%) | 18/20 (90%) | 18/20 (90%) |
| **Colorectal carcinomas** | 49/61 (80%) | | | 49/49 (82%) | 27/49 (55%) |
| **Adenomas** | 45/63 (71%) | | | 34/59 (58%) | 33/59 (56%) |
| **Mucosa (cancer normal)** | 2/21 (10%) | | | 2/21 (10%) | 9/21 (43%) |
| **Mucosa (normal normal)** | 1/23 (4%) | | | 1/19 (5%) | 5/21 (24%) |

| | BEX1 | INA | SNCA | SPG20 | CNRIP1 |
|---|---|---|---|---|---|
| **Colon cancer cell lines** | 18/19 (95%) | 19/20 (95%) | 19/20 (95%) | 20/20 (100%) | 20/20 (100%) |
| **Colorectal carcinomas** | 45/49 (92%) | 33/48 (69%) | 37/48 (77%) | 44/49 (90%) | 45/48 (94%) |
| **Adenomas** | 52/58 (90%) | 31/59 (53%) | 42/61 (69%) | 48/58 (83%) | 53/59 (88%) |
| **Mucosa (cancer normal)** | 15/21 (71%) | 2/20 (10%) | 14/21 (67%) | | 9/21 (43%) |
| **Mucosa (normal normal)** | 9/22 (45%) | 0/21 (0%) | 2/21 (10%) | 1/20 (5%) | 0/21 (0%) |

Table 3.

In general, the marker genes analyzed here are methylated at an extremely high frequency in colorectal cancer cell lines and colorectal carcinomas while methylation frequency is low in normal mucosa from non-cancerous donors. These results confirm utility of the marker genes in the diagnosis of tumour in the aero-digestive system and in the monitoring of tumour development.

In particular, MAL is methylated in 1/23 (4%) normal mucosa samples from non-cancerous donors, in 2/21 (10%) of normal mucosa samples taken in distance from the primary tumour, in 45/63 (71%) of adenomas, in 49/61 (80%) of carcinomas and in 19/20 (95%) of colon cancer cell lines. It will be noted that the methylation frequencies observed for MAL deviate slightly from those seen in example 1. This deviation is primarily due to the fact that the panel of samples analysed has been expanded.

FBN1 and INA are also rarely methylated in normal mucosa samples from both non-cancerous donors and normal mucosa samples taken in distance from the primary tumour (1/19, 5% and 2/21, 10%, respectively for FBN1; (0/21, 0% and 2/20, 10%, respectively for INA). Simultaneously, both FBN1 and INA are frequently methylated in carcinomas (40/49, 82%, and 33/48, 69%, respectively) as well as in adenomas (37/59, 58% and (31/59, 53%, respectively). The low methylation frequencies among both cohorts of normal mucosa samples and the high methylation frequencies in both benign and malignant tumours indicate that FBN1 and INA are particularly promising for detection of tumors early in the development. Simultaneously, a test comprising of these two markers would most likely have a high specificity.

SNCA, SPG20 and CNRP1 have in general higher methylation frequencies in carcinomas than the latter group (37/48 (77%), 44/49 (90%) and 45/48 (94%), respectively), as well as in adenomas (42/61 (69%), 48/58 (83%) and 52/59 (88%), respectively). By including these markers in a non-invasive test, the sensitivity is likely to increase. In addition to having low methylation frequencies in normal mucosa samples from non-cancerous donors, these markers have relatively high methylation frequencies in normal mucosa samples taken in distance from the primary tumour (14/21 (67%), (29%-90%) and 9/21 (43%), respectively). This may indicate a "field effect" around the tumour, where normal appearing cells in the close vicinity of the tumour also harbour methylation of these three genes. The presence of such a field effect could increase the sensitivity of a non-invasive feacal based test, as more cells harbouring methylation of SNCA, SPG20 and/or CNRP1 would be shed into the lumen of the colon and excreted with the faeces.

Example 3

*Methylation of the marker genes in different samples*

DNA was purified from stool and MSP was performed for the following genes MAL, FBN1, CNRIP1, INA, SPG20 and SNCA as described in the above examples.

Purification of DNA from 10 stool samples were analysed for all six genes. The methylation status of the corresponding primary tumour was known from 4-5 of the corresponding tumours.

In addition in nine of the 10 patients from which the stool samples were obtained a blood samples was also taken and the results are compared in table 5 DNA was isolated from 250mg faeces using the QIAamp DNA stool kit (QIAGEN).

The results of methylation state in genes from different samples are presented in table 4 below:

| Tissue/gene | MAL | CNRP1 | INA | FBN1 | SPG20 | SNCA |
|---|---|---|---|---|---|---|
| Stool | 0/3 | 1/4 | 2/6 | 0/4 | 2/5 | 3/8 |
| Blood | 3/9 | 8/9 | 3/9 | 0/9 | 6/9 | 9/9 |

With the exception of MAL and FBN1 methylation was detected in all markers in the samples from stool. In the blood samples methylation was detected in all genes except FBN1. In general the sample methylation frequency was high for all genes from blood samples. Particular the sample methylation frequency in blood samples comprising SNCA and CNRIP1 was very high. These markers seem particularly well suited for as cancer markers since they can be tested in a non-invasive procedure, such as in blood samples.

DNA was purified from blood (using a standard phenol/chloroform method) and MSP was performed for the following genes MAL, CNRIP1, INA, FBN1, SPG20 and SNCA as described in the above examples.

DNA was purified from 14 blood samples from patients with a corresponding primary tumour which was methylated from all six genes.

Table 5 expanded sample panel

| Tissue/gene | MAL | CNRP1 | INA | FBN1 | SPG20 | SNCA |
|---|---|---|---|---|---|---|
| Blood | 4/13 | 11/13 | 6/13 | 3/13 | 12/13 | 12/13 |

This example confirms the high sample methylation frequency of the genes in the blood samples and especially CNRIP1 and SNCA is highly suitable markers for diagnosis and/or screening for cancer or development of cancer. Also SPG20 seem as a very promising marker for blood sample screening.

Example 4

*Tissue-specific sample methylation frequencies of INA, SNCA, CNRIP1, SPG20 or FBN1 in different cell lines*

For each sample (cell lines from breast, kidney, ovary, pancreas, prostate, uterus and gastric.) the DNA was bisulphite treated before MSP as described in example 2.

The results of sample methylation frequency of genes in different samples are presented in table 6 below:

| | INA | SNCA | CNRIP1 | SPG20 | FBN1 |
|---|---|---|---|---|---|
| Breast | 4/6 | 6/7 | 2/6 | 2/6 | 4/6 |
| Kidney | 2/4 | 1/4 | 0/4 | 0/4 | 0/4 |
| Ovary | 2/4 | 0/4 | 0/4 | 1/4 | 1/4 |
| Pancreas | 3/6 | 4/6 | 5/6 | 4/6 | 2/6 |
| Prostate | 1/1 | 1/1 | 0/1 | 0/1 | 0/1 |
| Uterus | 2/4 | 2/4 | 1/3 | 2/4 | 2/4 |
| Gastric | 3/3 | 3/3 | 3/3 | 3/3 | 3/3 |

The promoter methylation status of INA, SNCA, CNRIP1, SPG20 and FBN1 was analyzed with MSP. In all samples from gastric cell lines the tested genes were methylated and thus the methylation frequency was (100 %) for all genes tested. In general, INA was methylated in at least one sample from all the tested tissues. The highest sample methylation frequency for this gene was seen in cell lines from gastric, 3/3 (100%), breast 4/6 (66%) and prostate 1/1 (100%). For cell lines from all other tissues the sample methylation frequency was 50%. SNCA was methylated in cell lines from all tested tissues, except for ovary. The highest samples methylation frequency was in cell lines from gastric, 3/3 (100%), breast 6/7 (85%), pancreas 4/6 (66%) and prostate 1/1 (100%). For uterus the sample methylation frequency was 50%. The sample methylation frequency of CNRIP1 was high in gastric, 3/3 (100%), and pancreas (83%) where 5 of 6 samples were methylated. SPG20 was methylated in 4 out of 6 (66%) pancreatic cell lines and in 2 of 4 uterus cell lines (50%). FBN1 was methylated in 4 of 6 breast cancer cell lines and 2 of 4 (50 %) samples was methylated in cell lines from uterus.

In general the all genes were methylated in samples from gastric cell lines thus the sample methylation frequency was high for all genes. In addition, all genes were methylated in breast cell lines although the frequencies were varying among the genes. Further, all genes were methylated in samples from pancreas and for all genes except FBN1 (33 %) the frequency was at or above 50 %.

This experiment clearly indicates that the genes according to the invention are methylated in cell lines from various cancer tissues and thus could be used as cancer markes for various cancers. It is obvious to the skilled artisan that each of the genes according to the invention may be combined differently dependent on the type of cancer to be detected. Thus the genes showing best results in breast cell lines would be selected as markers when detected breast cancer.

The result of tissue specific sample methylation frequency of MAL is listed in table 8.

Example 5

*Quantitative gene expression analyses were performed for the following genes: SPG20, INA and CNRIP1.*

Gene expression was measured in 6 colon cancer cell lines before and after treatment with epigenetic drugs. The relative expression levels of SPG20, INA and CNRIP1 in the colon cancer cell lines (n = 6) was measured. The expression levels are displayed as fold changes calculated from the deltadeltaCT method using the untreated sample as a calibrator. The mean expression of ACTB and GUSB was used as endogenous control.

TaqMan real-time fluorescence detection (Applied Biosystems, Foster city, CA) was used to quantify mRNA levels in the colon cancer cell lines, as previously described [Gibson et al. and Heid et al.]. cDNA was generated from five μg total RNA using a High-Capacity cDNA Archive kit (Applied Biosystems), including random primers according to the manufacturers' protocol. cDNA from the genes of interest (SPG20, INA and CNRIP1) and the endogenous controls *(ACTB* and *GUSB*) were amplified separately by the 7900HT Sequence Detection System (Applied Biosystems) following the protocol recommended by supplier. All samples were analyzed in triplicates. The expression levels were calculated as fold changes using the deltadeltaCT method and the untreated sample as a calibrator. In order to adjust for the possibly variable amounts of cDNA input in each PCR, we normalized the expression quantity of the target genes with the housekeeping genes *ACTB* and *GUSB.*

For SPG20, INA, and CNRIP1, the gene expression was significantly up-regulated in the majority of initially methylated colon cancer cell lines after promoter demethylation induced by the combined treatment 5-aza-2'-deoxycytidine and trichostatin A (Figures 2, 3 and 4). For INA and CNRIP1 the combined treatment was more effective than the individual treatment with 5-aza-2'-deoxycytidine alone and trichostatin A alone. The combined treatment also increased SPG20 expression, however similar or higher reactivation could be achieved by 5-aza-2'-deoxycytidine treatment alone. Treatment with the deacetylase inhibitor trichostatin A alone did not increase the gene expression of neither SPG20, INA nor CNRIP1. The two doses of 5-aza-2'-deoxycytidine tested here (1uM and 10uM) gave comparable effects. This means that demethylation of cell lines can be achieved by culturing them in the presence of low doses of 5-aza-2'-deoxycytidine, which is an advantage considering the cytotoxicity of this drug.

There was a clear relationship between methylation status and expression of SPG20, INA and CNRIP1. Thus the methylation measuring the methylation state or level by e.g. MSP and combining the result with the expression level of the corresponding gene could increase the sensitivity and specificity of a method of the invention.

Example 6

*Hypermethylation of MAL*

Patients and cell lines DNA from 218 fresh-frozen samples was subjected to methylation analysis, including 65 colorectal carcinomas (36 micro satellite stable; MSS, and 29 with micro satellite instability; MSI) from 64 patients, 63 adenomas, median size 8 mm, range 5-50 mm (61 MSS and 2 MSI) from 52 patients, 21 normal mucosa samples from 21 colorectal cancer patients (taken from distant sites from the primary carcinoma), and another 23 normal colorectal mucosa samples from 22 cancer-free individuals, along with 20 colon cancer cell lines (11 MSS and 9 MSI), and 29 cancer cell lines from various tissues (breast, gastric, kidney, ovary, pancreas, prostate, and uterus; Table 9). The mean age at diagnosis was 70 years (range 33 to 92) for patients with carcinoma, 67 years (range 62 to 72) for persons with adenomas, 64 years (ranging from 24 to 89) for the first group of normal mucosa donors, and 54 years (ranging from 33 to 86) for the second group of normal mucosa donors. The colorectal carcinomas and normal samples from cancer patients were obtained from an unselected prospective series collected from seven hospitals located in the South-East region of Norway. The adenomas were obtained from individuals attending a population based sigmoidoscopic screening program for colorectal cancer. The normal mucosa samples from cancer-free individuals were obtained from deceased persons, and the majority of the total set of normal samples (27/44) consisted of mucosa only, whereas the remaining samples were taken from the bowel wall. Additional clinico-pathological data for the current tumour series include gender and tumour location, as well as polyp size and total number of polyps per individual for the adenoma series.

All samples belong to approved research biobanks and are part of research projects approved according to national guidelines (Biobank; registered at the Norwegian Institute of Public Health. Projects: Regional Ethics Committee and National Data Inspectorate).

Six colon cancer cell lines, HCT15, HT29, SW48, SW480, RKO and LS1034 were subjected to treatment with the demethylating drug 5-aza-2'deoxycytidine (1 μM for 72 h and 10uM for 72 h), the histone deactetylase inhibitor trichostatin A (0.5 μM for 12 h) and a combination of both (1μM 5-aza-2'deoxycytidine for 72 h, 0.5 μM trichostatin A added the last 12 h).

*Bisulphite treatment and methylation-specific polymerase chain reaction (MSP)*

DNA from primary tumours and normal mucosa samples was bisulphite treated as previously described. DNA from colon cancer cell lines was bisulphite treated using the EpiTect bisulphite kit (Qiagen Inc., Valencia, CA, USA). The promoter methylation status of all genes was analyzed by methylation-specific polymerase chain reaction (MSP) using the HotStarTaq DNA polymerase (Qiagen). All results were confirmed with a second independent round of MSP. Human placental DNA (Sigma Chemical Co, St. Louis, MO, USA) treated *in vitro* with *Sss1* methyltransferase (New England Biolabs Inc., Beverly, MA, USA) was used as a positive control for the methylated MSP reaction, whereas DNA from normal lymphocytes was used as a positive control for unmethylated alleles. Water was used as a negative control in both reactions. The primers were designed with MethPrimer and Methyl Primer Express and their sequences are listed in Table 7.

| Primer set | Sense primer/ Antisense primer | Frg. Size bp | Annealing temp. | Fragment location | SEQ ID NO. |
|---|---|---|---|---|---|
| MAL MSP-M | TTCGGGTTTTTTTGTTTTTAATTC/ GAAAACCATAACGACGTACTAACGT | 139 | 56 | -71 to 68 | 37/38 |
| MAL MSP-U | TTTTGGGTTTTTTTGTTTTTAATTT/ ACAAAAACCATAACAACATACTAACATC | 142 | 56 | -72 to 70 | 39/40 |
| MAL BS_A | GGGTTTTTTTGTTTTTAATT/ ACCAAAAACCACTCACAAACTC | 236 | 53 | -68 to 168 | 97/98 |
| MAL BS_B | GGAAAAATGAAGGAGATTTAAATTT/ AATAACCTAAACRCCCCC | 404 | 50 | -427 to -23 | 99/100 |

Abbreviations: MSP, methylation-specific polymerase chain reaction; BS, bisulfite sequencing; M, methylated-specific primers; U, unmethylated-specific primers; Frg. Size, fragment size; An. Temp, annealing temperature (in degrees celsius). Fragment location lists the start and end point (in base pairs) of each fragment relative to the transcription start point provided by NCBI (RefSeq ID NM_002371), http://www.ncbi.nlm.nih.gov/mapview/map/search_cg

*Bisulphite sequencing*

All colon cancer cell lines (n = 20) were subjected to direct bisulphite sequencing of the *MAL* promoter. Two fragments were amplified: fragment A, covering bases - 68 to 168 relative to the transcription start point (overlapping with our MSP product), and fragment B covering bases -427 to -23. Fragment A covered altogether 24 CpG sites and was amplified using the HotStarTaq DNA polymerase and 35 PCR cycles. Fragment B covered altogether 32 CpG sites and was amplified using the same polymerase and 36 PCR cycles. The primer sequences are listed in Table 8. Excess primer and nucleotides were removed by ExoSAP-IT treatment following the protocol of the manufacturer (GE Healthcare, USB Corporation, Ohio, USA). The purified products were subsequently sequenced using the dGTP BigDye Terminator Cycle Sequencing Ready Reaction kit (Applied Biosystems, Foster City, CA, USA) in an AB Prism 3730 sequencer (Applied Biosystems). The approximate amount of methyl cytosine of each CpG site was calculated by comparing the peak height of the cytosine signal with the sum of the cytosine and thymine peak height signals, as previously described. CpG sites with ratios ranging from 0 - 0.20 were classified as unmethylated, CpG sites within the range 0.21 - 0.80 were classified as partially methylated, and CpG sites ranging from 0.81 - 1.0 were classified as hypermethylated.

cDNA *preparation and real-time quantitative gene expression*

Total RNA was extracted from cell lines (n = 46), tumours (n = 16), and normal tissue (n = 3) using Trizol (Invitrogen, Carlsbad, CA, USA) and the RNA concentration was determined using ND-1000 Nanodrop (NanoDrop Technologies, Wilmington, DE, USA). For each sample, total RNA was converted to cDNA using a High-Capacity cDNA Archive kit (Applied Biosystems), including random primers. MAL (Hs00242749_m1 and Hs00360838_m1) and the endogenous controls *ACTB* (Hs99999903_m1) and *GUSB* (Hs99999908_m1) were amplified separately in 96 well fast plates following the recommended protocol (Applied Biosystems), and the real time quantitative gene expression was measured by the 7900HT Sequence Detection System (Applied Biosystems). All samples were analyzed in triplicate, and the median value was used for data analysis. The human universal reference RNA (containing a mixture of RNA from ten different cell lines; Stratagene) was used to generate a standard curve, and the resulting quantitative expression levels of MAL were normalized against the mean value of the two endogenous controls.

*Tissue microarray*

For *in situ* detection of protein expression in colorectal cancers, a tissue microarray (TMA) was constructed, based on the technology previously described Embedded in the TMA are 292 cylindrical tissue cores (0.6 mm in diameter) from ethanol-fixed and paraffin embedded tumour samples derived from 281 individuals. Samples from the same patient series has been examined for various biological variables and clinical end-points. In addition, the array contains normal tissues from kidney, liver, spleen, and heart as controls. Ethanol-fixed normal colon tissues from four persons with no known history of colorectal cancer were obtained separately.

*Immunohistochemical in situ protein expression analysis*

Five μm thick sections of the TMA blocks were transferred onto glass slides for immunohistochemical analyses. The sections were deparaffinized in a xylene bath for 10 minutes and rehydrated via a series of graded ethanol baths. Heat-induced epitope retrieval was performed by heating in a microwave oven at full effect (850 W) for 5 minutes followed by 15 minutes at 100 W immersed in 10 mM citrate buffer at pH 6.0 containing 0.05% Tween-20. After cooling to room temperature, the immunohistochemical staining was performed according to the protocol of the DAKO Envision+™ K5007 kit (Dako, Glostrup, Denmark). The primary antibody, mouse clone 6D9 anti-MAL, was used at a dilution of 1:5000, which allowed for staining of kidney tubuli as positive control, while the heart muscle tissue remained unstained as negative control. The slides were counterstained with haematoxylin for 2 minutes and then dehydrated in increasing grades of ethanol and finally in xylene. Results from the immunohistochemistry were obtained by independent scoring by one of the authors and a reference pathologist.

*Statistics*

All P values were derived from two tailed statistical tests using the SPSS 13.0 software (SPSS, Chicago, IL, USA). Fisher's exact test was used to analyze 2 x 2 contingency tables. A 2 x 3 table and Chi-square test was used to analyze the potential association between quantitative gene expression of *MAL* and promoter methylation status. Samples were divided into two categories according to their gene expression levels: low expression included samples with gene expression equal to, or lower than, the median value across all cell lines or all tumours, high expression included samples with gene expression higher that the median. The methylation status was divided into three categories: unmethylated, partial methylation, and hypermethylated.

*Promoter methylation status of MAL in tissues and cell lines*

The promoter methylation status of *MAL* was analyzed with MSP (Figure 5). One of 23 (4%) normal mucosa samples from non-cancerous donors and two of 21 (10%) normal mucosa samples taken in distance from the primary tumour were methylated but displayed only low-intensity band compared with the positive control after gel electrophoresis. Forty-five of 63 (71%) adenomas and 49/61 (80%) carcinomas showed promoter hypermethylation. Nineteen of twenty colon cancer cell lines (95%), and 15/26 (58%) cancer cell lines from various tissues (breast, kidney, ovary, pancreas, prostate, and uterus) were hypermethylated (Table 9 lists tissue-specific frequencies).

Table 8. Promoter methylation status of *MAL* in cell lines of various tissues.

| Cell line | Tissue | Promoter methylation status | Methylation frequency |
|---|---|---|---|
| BT-20 | Breast | M | |
| BT-474 | Breast | U/M | |
| Hs 578T | Breast | U | |
| SK-BR-3 | Breast | U | 57% |
| T-47D | Breast | U/M | |
| ZR-75-1 | Breast | U | |
| ZR-75-38 | Breast | M | |
| Co115 | Colon | M | |
| HCT15 | Colon | M | |
| HCT116 | Colon | M | |
| LoVo | Colon | M | |
| LS174T | Colon | M | |
| RKO | Colon | M | |
| SW48 | Colon | M | |
| TC7 | Colon | M | |
| TC71 | Colon | M | |
| ALA | Colon | M | 95% |
| Colo320 | Colon | M | |
| EB | Colon | M | |
| FRI | Colon | U/M | |
| HT29 | Colon | M | |
| IS1 | Colon | M | |
| IS2 | Colon | M | |
| IS3 | Colon | M | |
| LS1034 | Colon | M | |
| SW480 | Colon | M | |
| V9P | Colon | U | |
| ACHN | Kidney | U | |
| Caki-1 | Kidney | U | 50% |
| Caki-2 | Kidney | M | |
| 786-0 | Kidney | U/M | |
| ES-2 | Ovary | U/M | |
| OV-90 | Ovary | U/M | 50% |
| Ovcar-3 | Ovary | U | |
| SK-OV-3 | Ovary | U | |
| AsPC-1 | Pancreas | M | |
| BxPC-3 | Pancreas | U | |
| CFPAC-1 | Pancreas | U | 67% |
| HPAF-II | Pancreas | M | |
| PaCa-2 | Pancreas | M | |
| Panc-1 | Pancreas | U/M | |

(continued)

| Cell line | Tissue | Promoter methylation status | Methylation frequency |
|---|---|---|---|
| LNCaP | Prostate | U | 0% |
| AN3 CA | Uterus | U/M | |
| HEC-1-A | Uterus | M | 75% |
| KLE | Uterus | U | |
| RL95-2 | Uterus | M | |

The promoter methylation status of the individual cell lines was assessed by methylation-specific polymerase chain reaction (MSP). The methylation frequency reflects the number of methylated (M and U/M) samples from each tissue. Abbreviations: U, unmethylated; M, methylated.

The hypermethylation frequency found in normal samples was significantly lower than in adenomas ($P < 0.0001$) and carcinomas ($P < 0.0001$). Hypermethylation of the *MAL* promoter was not associated with MSI status, gender, or age in neither malignant nor benign tumours. Among carcinomas, tumours with distal location in the bowel (left side and rectum) were more frequently hypermethylated than were tumours with proximal location, although not statistically significant ($P = 0.088$). Among adenomas, no significant association could be found between promoter methylation status of *MAL* and polyp size or number.

*Bisulphite sequencing verification of the promoter methylation status of MAL*

Two overlapping fragments of the *MAL* promoter were bisulphite sequenced in 20 colon cancer cell lines. The results are summarized in Figure 6, and representative raw data can be seen in Figure 7. A good association was seen between the methylation status, as assessed by MSP, and the bisulphite sequences of the overlapping fragment A. However, in fragment B there was poor association with the MSP data. For this fragment, which is located farther upstream relative to the transcription start point, several consecutive CpG sites were frequently unmethylated and/or partially methylated. This held true also in cell lines shown to be heavily methylated around the transcription start point (fragment A; Figure 6).

*Real-time quantitative gene expression*

The level of MAL mRNA expression in cell lines ($n = 46$), primary colorectal carcinomas ($n = 16$), and normal mucosa ($n = 3$) was assessed by quantitative real time PCR. There was a strong association between MAL promoter hypermethylation and reduced or lost gene expression among cell lines ($P = 0.041$; Figure 8). Furthermore, the gene expression of MAL was up-regulated in colon cancer cell lines after promoter demethylation induced by the combined treatment 5-aza-2'-deoxycytidine and trichostatin A (Figure 9). Treatment with the deacetylase inhibitor trichostatin A alone did not increase MAL expression, whereas treatment with the DNA demethylating 5-aza-2'-deoxycytidine led to high expression in HT29 cells, but more moderate levels in HCT15 cells (Figure 9). Among primary colorectal carcinomas, those harbouring promoter hypermethylation of MAL ($n = 13$) expressed somewhat lower levels of MAL mRNA compared with the unmethylated tumours ($n = 3$), although not statistically significant (Figure 8).

*MAL protein expression is lost in colorectal carcinomas*

To evaluate the immunohistochemistry analyses of MAL, kidney and heart muscle tissues were included as positive and negative controls, respectively (Figure 10 A-B) From the 231 scorable colorectal tissue cores, *i.e.* those containing malignant colorectal epithelial tissue, 198 were negative for MAL staining (Figure 10 C-D). Twenty-nine of these had positive staining in non-epithelial tissue components within the same tissue cores, mainly in neurons and blood vessels (not shown). In comparison, all the sections of normal colon tissue contained positive staining for MAL in the epithelial cells (Figure 10 E-F).

These experiments conclude that the *MAL* promoter close to the transcription start is hypermethylated in the vast majority of malignant, as well as in benign colorectal tumours, in contrast to normal colon mucosa samples which are unmethylated, and we contend that *MAL* remains a promising diagnostic biomarker for early colorectal tumourigenesis. In addition, hypermethylated *MAL* was found in cancer cell lines from breast, kidney, ovary, and uterus.

Hypermethylation of MAL has, by quantitative methylation-specific polymerase chain reaction (MSP), previously been shown by others to be present only in a small fraction (6%, 2/34) of colon carcinomas (Mori et al.). In contrast, the applicants demonstrate here a significantly higher methylation frequency of MAL in both benign and malignant colorectal tumours (71% in adenomas and 80% in carcinomas). The discrepancy in methylation frequencies between the present report and the previous study by Mori et al. is probably a consequence of study design. From direct bisulphite sequencing of colon cancer cell lines, we have now shown that the DNA methylation of MAL is unequally distributed within the CpG islands of its promoter (Figure 6). CpG islands often span more than one kilobase of the gene promoter, and the methylation status within this region is sometimes mistakenly assumed to be equally distributed. Since the results of an MSP analysis rely on the match or mismatch of the unmethylated and methylated primer sequences to bisulphite treated DNA, one should ensure that the primers anneal to relevant CpG sites in the gene promoter. In the present study, the applicants designed the MSP primers close to the transcription start point of the gene (-72 to +70) and found, by bisulphite sequencing, concordance between the overall methylation status of *MAL* as assessed by MSP and the methylation status of the individual CpG sites covered by our MSP primer set (Figure 6). This part of the CpG island was hypermethylated in the majority of colon cancer cell lines (95%). We also found that these cell lines, as well as those of other tissues, showed loss of MAL RNA expression from quantitative real time analyses, and that removal of DNA hypermethylation by the combined treatment of 5-aza-2'-deoxycytidine and Trichostatin A re-induced the expression of MAL in colon cancer cell lines (Figure 9). Furthermore, by analyzing a large series of clinically representative samples by protein immunohistochemistry we confirmed that the expression of MAL was lost in malignant colorectal epithelial cells as compared to normal mucosa.

The inventors have further analyzed the same region of the MAL promoter as Mori *et al.,* which is located -206 to -126 base pairs upstream of the transcription start point By direct bisulphite sequencing, we showed that only a minority of the CpG sites covered by the Mori antisense primer were methylated in the 19 colon cancer cell lines that were heavily methylated around the transcription start point (Figure 6). We therefore conclude that the very low (six percent) methylation frequency initially reported for MAL in colon carcinomas (Mori et al.) is most likely a consequence of the primer design and choice of CpG sites to be examined.

Inactivating hypermethylation of the MAL promoter might be prevalent also in other cancer types. In the present study, hypermethylated MAL was found in cancer cell lines from breast, kidney, ovary, and uterus.

The present analyses of cancer cell lines from seven tissues indicate that the hypermethylation of a limited area in the proximity of the transcription start point of MAL is associated with reduced or lost gene expression.

A sensitive non-invasive screening approach for colorectal cancer could markedly improve the clinical outcome for the patient. Such a diagnostic test could in principle measure the status of a single biomarker.

Hypermethylation of the MAL promoter represents a frequently hypermethylated gene among pre-malignant colorectal lesions, and is accompanied by low methylation frequencies in normal colon mucosa. The presence of such epigenetic changes in pre-malignant tissues might also have implications for cancer chemoprevention. By inhibiting or reversing these epigenetic alterations, the progression to a malignant phenotype might be prevented (Kopelovich et al.). Promoter hypermethylation of MAL remains one of the most promising diagnostic biomarkers for early detection of colorectal tumours.

**References**

1. Mori Y, Cai K, Cheng Y, Wang S, Paun B, Hamilton JP, Jin Z, Sato F, Berki AT, Kan T, Ito T, Mantzur C, Abraham JM, Meltzer SJ. A genome-wide search identifies epigenetic silencing of somatostatin, tachykinin-1, and 5 other genes in colon cancer. Gastroenterology 2006;131:797-808.

2. Lind GE, Kleivi K, Meling GI, Teixeira MR, Thiis-Evensen E, Rognum TO, Lothe RA. ADAMTS1, CRABP1, and NR3C1 Identified as Epigenetically Deregulated genes in Colorectal Tumourigenesis. Cell Oncol 2006;28:259-272.

3. Laird PW. The power and the promise of DNA methylation markers. Nat Rev Cancer 2003;3:253-266.

4. Muller HM, Oberwalder M, Fiegl H, Morandell M, Goebel G, Zitt M, Muhlthaler M, Ofner D, Margreiter R, Widschwendter M. Methylation changes in faecal DNA: a marker for colorectal cancer screening? Lancet 2004;363:1283-1285.

5. Chen WD, Han ZJ, Skoletsky J, Olson J, Sah J, Myeroff L, Platzer P, Lu S, Dawson D, Willis J, Pretlow TP, Lutterbaugh J, Kasturi L, Willson JK, Rao JS, Shuber A, Markowitz SD. Detection in fecal DNA of colon cancer-specific methylation of the nonexpressed vimentin gene. J Natl Cancer Inst 2005; 97:1124-1132.

6. C.Grunau, S.J.Clark and A.Rosenthal, Bisulfite genomic sequencing: systematic investigation of critical experimental parameters, Nucleic Acids Res. 29 (2001), E65.

7. M.F.Fraga and M.Esteller, DNA methylation: a profile of methods and applications, Biotechniques 33 (2002), 632-649.

8. B.Smith-Sørensen, G.E.Lind, R.I.Skotheim, S.D.Fosså, Ø.Fodstad, A.E.Stenwig, K.S.Jakobsen and R.A.Lothe, Frequent promoter hypermethylation of the 06-Methylguanine-DNA Methyltransferase (MGMT) gene in testicular cancer, Oncogene 21 (2002), 8878-8884.

9. J.G.Herman, J.R.Graff, S.Myöhänen, B.D.Nelkin and S.B.Baylin, Methylation-specific PCR: a novel PCR assay for methylation status of CpG islands, Proc. Natl. Acad. Sci. U. S. A. 93 (1996), 9821-9826.

10. S.Derks, M.H.Lentjes, D.M.Hellebrekers, A.P.de Bruine, J.G.Herman and E.M.van, Methylation-specific PCR unraveled, Cell Oncol. 26 (2004), 291-299.

11. L.C.Li and R.Dahiya, MethPrimer: designing primers for methylation PCRs, Bioinformatics. 18 (2002), 1427-1431.

12. J.R.Melki, P.C.Vincent and S.J.Clark, Concurrent DNA hypermethylation of multiple genes in acute myeloid leukemia, Cancer Res. 59 (1999), 3730-3740.

13. Zweig, M. H., and Campbell, G., Clin. Chem. 39 (1993) 561-577

SEQUENCE LISTING

<110> Rikshospitalet-Radiumhospitalet HF
Lothe, Ragnhild A.
Lind, Guro E.
Ahlquist, Terje
Skotheim, Rolf I

<120> New Markers For Cancer

<130> 42139EP06

<150> PA 2007 00273
<151> 2007-02-27

<150> PA 2007 00601
<151> 2007-04-24

<160> 100

<170> PatentIn version 3.5

<210> 1
<211> 1300
<212> DNA
<213> Homo Sapiens

<400> 1
gactcgggcg gatttcaggc ttcagtgttt gtaggaggaa acacagcaat cacactatta 60

atagtaaatt aaaataaatg ggcaactgct gcatggtaat actttttttt ttaaggcaaa 120

aaataaaaaa tagtgaaaca gagaaacaaa acatgaaaca ccggcagtca acaggcaggc 180

aaagaacctg gggtgggggg tagcagcggt cccaccctca aaaggcccgg gctgcccaga 240

ccaagagaaa gcgatgaatc tcttctggta acgtcccttc ctgtcgcatg gattcaaggc 300

cgacctgccc cagcaccacc accagcagcc ttctgctggg gccggcacag ctgggagcaa 360

cctcctactc tcaggcagac gcgcagcacc aagcagagag gcccggtgca ggatcccagc 420

gccgaaccag cgccggctca gtggacgcgg aaggggccgg cggccgcggc cggtcccatc 480

ccccactgca gacccccagc ctgtggcggt ggtccagttc cgccaggaaa ccgccgcctg 540

gagctgtggg tcgcgcacat taacgcatcc agcggaaaaa tgaaggagac ccaaattcaa 600

agttaaagta atggtgaccc gagaggtgcc ttgatgagaa ggtttggggt cccggttact 660

gatggttatc attcttacga gatgctggtc acctacgaag ggagaaaggc acgaggagcg 720

cctgaccaaa gtggttttgc cctgcttccc gcaagaggtg gcacccacgg ctggaacgca 780

ggagtcagac ccacagtccc cagctctgga cgcccgcagc ggggcctcga agaggttcag 840

ggcggtgccc gcggcgctcg ggccgggtct cccggggcgt ggggcggggg gcggggttgg 900

gcggcggccg gggctcctcc ctcttctgcc ccgggctccc ctgctcttaa cccgcgcgcg 960

ggggcgccca ggccactggg ctccgcggag ccagcgagag gtctgcgcgg agtctgagcg 1020

gcgctcgtcc cgtcccaagg ccgacgccag cacgccgtca tggcccccgc agcggcgacg 1080

gggggcagca ccctgcccag tggcttctcg gtcttcacca ccttgcccga cttgctcttc 1140

atctttgagt ttgtgagtgg ctcctggccg gggaagggac ggggtgggct gagccgtgcg 1200

ctctctcggg cgcccagcac agctgtcgga cgggatccgc tagctgcgca ggttctggga 1260

gcatcggggc agcaggcgca gggcggggac taagccaggg 1300

<210> 2
<211> 1300
<212> DNA
<213> Homo Sapiens

<400> 2
gactcgggcg gatttcaggc ttcagtgttt gtaggaggaa acacagcaat cacactatta 60

atagtaaatt aaaataaatg ggcaactgct gcatggtaat actttttttt ttaaggcaaa 120

aaataaaaaa tagtgaaaca gagaaacaaa acatgaaaca ccggcagtca acaggcaggc 180

aaagaacctg gggtgggggg tagcagcggt cccaccctca aaaggcccgg gctgcccaga 240

ccaagagaaa gcgatgaatc tcttctggta acgtcccttc ctgtcgcatg gattcaaggc 300

cgacctgccc cagcaccacc accagcagcc ttctgctggg gccggcacag ctgggagcaa 360

cctcctactc tcaggcagac gcgcagcacc aagcagagag gcccggtgca ggatcccagc 420

gccgaaccag cgccggctca gtggacgcgg aaggggccgg cggccgcggc cggtcccatc 480

ccccactgca gacccccagc ctgtggcggt ggtccagttc cgccaggaaa ccgccgcctg 540

gagctgtggg tcgcgcacat taacgcatcc agcggaaaaa tgaaggagac ccaaattcaa 600

agttaaagta atggtgaccc gagaggtgcc ttgatgagaa ggtttggggt cccggttact 660

gatggttatc attcttacga gatgctggtc acctacgaag ggagaaaggc acgaggagcg 720

cctgaccaaa gtggttttgc cctgcttccc gcaagaggtg gcacccacgg ctggaacgca 780

ggagtcagac ccacagtccc cagctctgga cgcccgcagc ggggcctcga agaggttcag 840

ggcggtgccc gcggcgctcg ggccgggtct cccggggcgt ggggcggggg gcggggttgg 900

gcggcggccg gggctcctcc ctcttctgcc ccgggctccc ctgctcttaa cccgcgcgcg 960

ggggcgccca ggccactggg ctccgcggag ccagcgagag gtctgcgcgg agtctgagcg 1020

gcgctcgtcc cgtcccaagg ccgacgccag cacgccgtca tggcccccgc agcggcgacg 1080

gggggcagca ccctgcccag tggcttctcg gtcttcacca ccttgcccga cttgctcttc 1140

atctttgagt ttgtgagtgg ctcctggccg gggaagggac ggggtgggct gagccgtgcg 1200

ctctctcggg cgcccagcac agctgtcgga cgggatccgc tagctgcgca ggttctggga 1260

gcatcggggc agcaggcgca gggcggggac taagccaggg 1300

<210> 3
<211> 1300

<212> DNA
<213> Homo Sapiens

<400> 3
gactcgggcg gatttcaggc ttcagtgttt gtaggaggaa acacagcaat cacactatta 60

atagtaaatt aaaataaatg ggcaactgct gcatggtaat acttttttttt ttaaggcaaa 120

aaataaaaaa tagtgaaaca gagaaacaaa acatgaaaca ccggcagtca acaggcaggc 180

aaagaacctg ggggtggggg tagcagcggt cccaccctca aaaggcccgg gctgcccaga 240

ccaagagaaa gcgatgaatc tcttctggta acgtcccttc ctgtcgcatg gattcaaggc 300

cgacctgccc cagcaccacc accagcagcc ttctgctggg gccggcacag ctgggagcaa 360

cctcctactc tcaggcagac gcgcagcacc aagcagagag gcccggtgca ggatcccagc 420

gccgaaccag cgccggctca gtggacgcgg aaggggccgg cggccgcggc cggtcccatc 480

ccccactgca gacccccagc ctgtggcggt ggtccagttc cgccaggaaa ccgccgcctg 540

gagctgtggg tcgcgcacat taacgcatcc agcggaaaaa tgaaggagac ccaaattcaa 600

agttaaagta atggtgaccc gagaggtgcc ttgatgagaa ggtttggggt cccggttact 660

gatggttatc attcttacga gatgctggtc acctacgaag ggagaaaggc acgaggagcg 720

cctgaccaaa gtggttttgc cctgcttccc gcaagaggtg gcacccacgg ctggaacgca 780

ggagtcagac ccacagtccc cagctctgga cgcccgcagc ggggcctcga agaggttcag 840

ggcggtgccc gcggcgctcg ggccgggtct cccggggcgt ggggcggggg gcggggttgg 900

gcggcggccg gggctcctcc ctcttctgcc ccgggctccc ctgctcttaa cccgcgcgcg 960

ggggcgccca ggccactggg ctccgcggag ccagcgagag gtctgcgcgg agtctgagcg 1020

gcgctcgtcc cgtcccaagg ccgacgccag cacgccgtca tggcccccgc agcggcgacg 1080

gggggcagca ccctgcccag tggcttctcg gtcttcacca ccttgcccga cttgctcttc 1140

atctttgagt ttgtgagtgg ctcctggccg gggaagggac ggggtgggct gagccgtgcg 1200

ctctctcggg cgcccagcac agctgtcgga cgggatccgc tagctgcgca ggttctggga 1260

gcatcggggc agcaggcgca gggcggggac taagccaggg 1300

<210> 4
<211> 1300
<212> DNA
<213> Homo Sapiens

<400> 4
gactcgggcg gatttcaggc ttcagtgttt gtaggaggaa acacagcaat cacactatta 60

atagtaaatt aaaataaatg ggcaactgct gcatggtaat acttttttttt ttaaggcaaa 120

aaataaaaaa tagtgaaaca gagaaacaaa acatgaaaca ccggcagtca acaggcaggc 180

aaagaacctg ggggtggggg tagcagcggt cccaccctca aaaggcccgg gctgcccaga 240

ccaagagaaa gcgatgaatc tcttctggta acgtcccttc ctgtcgcatg gattcaaggc 300

cgacctgccc cagcaccacc accagcagcc ttctgctggg gccggcacag ctgggagcaa 360

cctcctactc tcaggcagac gcgcagcacc aagcagagag gcccggtgca ggatcccagc 420

gccgaaccag cgccggctca gtggacgcgg aaggggccgg cggccgcggc cggtcccatc 480

ccccactgca gaccccccagc ctgtggcggt ggtccagttc cgccaggaaa ccgccgcctg 540

gagctgtggg tcgcgcacat taacgcatcc agcggaaaaa tgaaggagac ccaaattcaa 600

agttaaagta atggtgaccc gagaggtgcc ttgatgagaa ggtttggggt cccggttact 660

gatggttatc attcttacga gatgctggtc acctacgaag ggagaaaggc acgaggagcg 720

cctgaccaaa gtggttttgc cctgcttccc gcaagaggtg gcacccacgg ctggaacgca 780

ggagtcagac ccacagtccc cagctctgga cgcccgcagc ggggcctcga agaggttcag 840

ggcggtgccc gcggcgctcg ggccgggtct cccggggcgt ggggcggggg gcggggttgg 900

gcggcggccg gggctcctcc ctcttctgcc ccgggctccc ctgctcttaa cccgcgcgcg 960

ggggcgccca ggccactggg ctccgcggag ccagcgagag gtctgcgcgg agtctgagcg 1020

gcgctcgtcc cgtcccaagg ccgacgccag cacgccgtca tggcccccgc agcggcgacg 1080

gggggcagca ccctgcccag tggcttctcg gtcttcacca ccttgcccga cttgctcttc 1140

atctttgagt ttgtgagtgg ctcctggccg gggaagggac ggggtgggct gagccgtgcg 1200

ctctctcggg cgcccagcac agctgtcgga cgggatccgc tagctgcgca ggttctggga 1260

gcatcggggc agcaggcgca gggcggggac taagccaggg 1300

<210> 5
<211> 1300
<212> DNA
<213> Homo Sapiens

<400> 5
gcttcactct ctgaaactct ttcaacatat gcaggtctca ctttaaaagg tcttgaaatg 60

gagttcctac cagacggcct ttccctaggg ttcataaaga ccctttcaag ccaccctccc 120

gcgcgccgcg ttaccacacg cccacctcct ggttgccgcg actacacctc acgaactttt 180

cgcgccccaa ggcggccctt tataaagatg cggtgacgtc accgcgcgcc gcgcgtgcgc 240

aaatatggta acgtgaccac gccctggcct gcgctaaggc cccggcgtcg gcgcagtagc 300

tggtgccttc ccggaagggc tcagaggcgg gctcggtgag tggagtcgga cagtgggtgg 360

gtgccctgac tgcctgacgg ccgcgcaggg ccttgcagga ccgctgggga ggatgttgtt 420

aacgtaaaga gccaggctga tgaggaaggt tcatgagtcc tttgggtata agcgggagtt 480

ggggcggccg gagggcagat gactctgaga aggtgcggat cttcgattct aaggggagaa 540

tgtgatttgg ttttgtctaa agtgctgata gtgggcctca cagaaaagtt accatgaacc 600

gactttccgg gattcccttg gcgctgcatt aattggcctc gttgcaaaag gaaaccgaga 660

ggaaggcggc aagacttggc ctgcgggtga tgttagcgga aaatatctgt aatttagacc 720

ccggagggcg ggcctgtgag cgattgctgt ccgcggtgct gaaggaaagg cagccacgcc 780

cagaggagga aacctaatga gggcagggcg gtgtttccgt tttccttggc cgggttcagc 840

accctacact caataagcgt gggctttcta gcaggactgg acactcaggc ggtgtgtcaa 900

tctttagaca atctttatag agtgcataga tgtttaattt cataaaattt aggaaagctt 960

catttcagga aggaatttta ttcctctttt tctcccttag ggcaagcact ttaacctttt 1020

aagcccaacc agatgagttg cctgcagttt tggaggcctt cagagcattt cactagacct 1080

ctgtctgtgt cggtccagtg tctttagcca aggtgagctt gctcgtggtg tgaagtgtgc 1140

cacgaaaatg ggctttcctg cactccacaa atgagagtgc ttagcagcca ggggaagggg 1200

aaggtatcta tctaattgtg cttttcctcg cagttacaca tgaaaagtac tttcctattt 1260

agaagagaga tggtagaact gggcactctt ccaataaagg 1300

<210> 6
<211> 1300
<212> DNA
<213> Homo Sapiens

<400> 6
aggccttcct aaactgttac agggtgcagg catcattggg cattatccat ataaggatgc 60

cacaaatact atacctttca aagccactaa attcactgtg ccgcgcatat cctggggaga 120

ggcagctgtg tgtgggggag ggcgagggga ccgggagggt gtaagtagag tcctccagat 180

gaaccccagt cttaatctgg gctgggcaag aggaccatcc cttcaccgct cccatcctac 240

atcctaggtt ccaacattta caggcccctg ggttggacac cgacttagag agagcaccct 300

ggattcaggc ctgggtggct tgaattgagc ctcagaagag ccgcgtctgg agtgggctct 360

cgacacccag ggcaagtggg ggcggcagag ccctctcctc ggtcggcaca gcagcctctg 420

ccgcggtccc ggcctgcgac gcgcccagtc ttagcctccc ggcctccggc gtctgctgag 480

tgtccggcgg gagaggcgca gggagcgcgc taccgggagg cgcgggcagc ggggactggt 540

tttctctcgg gccagggcct ccggggcaac cgtctccagc gcgcattctt ggtgcaggtg 600

gaacagcttt ctgctccggt agggcttcac ctatcgcggg agaggttaat ctcggatcta 660

aacctcgcag ccgcagagcg ggctaaaacc gctactccac ctcttcccat ttctcccctc 720

cccacctcaa gacaaaaagt cccaggccgg gcaggacctg atcacctctg cctcctccca 780

ctgcgctaat cctgcgagcg agaggccccg caccgaggcg gaggctggca aaggggagtg 840

gaaagggagg atggatgggg ccggggggtg gggtggtgat gagggcgacg aaggaggggg 900

tgtcattttc tttttctttc tttttttaaa aaaagtattt ctctcgcgag aaaccgctgc 960

gcggacgata cttgaagagg tggggaaagg agggggctgc gggagccgcg gcagagactg 1020

tgggtgccac aagcggacag gagccacagc tgggacagct gcgagcggag ccgagcagtg 1080

gctgtagcgg ccacgactgg gagcagccgc cgccgcctcc tcgggagtcg gagccgccgc 1140

ttctccagtg ggtgcagccg gggtcccgac gggggtcggg cggccaccgg ggctggagct 1200

gcggccacgg aggcttttgc gtttgcgccg cgccgagggc agggacaggg actggggtga 1260

ggggctgtcc cggaacgtcc acagctggcg ctggccctcc 1300

<210> 7
<211> 1300
<212> DNA
<213> Homo Sapiens

<400> 7
acatatatat tatatgtata gcttattatt tcttaaaaaa gagcagtcta acttgggtaa 60

acaaaaactg ccaaggaaac gcatccctac ccatttgttc tcactagccc acttcatttc 120

ccaggtgtgc tccctggctc tggtactatc catctgggtg gtgctgaagc aggtgctctc 180

tctgctccgg gaagccattc ccagtatagt cctcaccccc tgagtctccc ctcgcttcca 240

aaagtcagct gtctaccgac ctctgccacg aaaactgtct aatcacgctt aatatgcaaa 300

tctaaactct gcggtgtcgc atatggcccc ctgccaataa tcctttgacc catcccacct 360

cttggccatt ttgtcaccag aatgccttag gctcatgtca gttgcctttt acagggttaa 420

tagctcaccc ttcaaaatct gtgtaaatat atgtgattca ttacatatat ttatgtatgt 480

gtcatttttg ggacgagcat acgtataatg gaaatgtaca cccacaagaa gatgtagctc 540

cttgaatatg gttattttct tcctcttgca tccattcctc atccagcgtg gctccatcac 600

gtggaattgc gcctttcctc ggccatttcc cctaagcttt ggaaagactc atatttcctg 660

aaatttaaat caccggctag cggtttcctg tccccccttc ccgcctccct ttaccccccc 720

tcagcctcct cccgcctggc tggcgccgga tccgcgagca gcagtgtcca cctcacgtag 780

ctggccgagg cggtatccag attccggggg tctcgctcct tggcatagtg gtcgcttaac 840

tccagcgcct tcccagtgcc tcccaaacct ctcctcctcc tgcccggggc tgttctagga 900

ggagcctaaa tgtccgcttc cccagctgaa ggggattgtg gtgcgtaaac aggattaact 960

ccctcgcccc cagctgcgcg gatctgcgcg gcggcctgcg ggccagagcg agccttcggg 1020

tgcgtggagg gcgcagcact ggagcggggg atggggaagc gcgcggcctt ggagccgtct 1080

cttgggcccg cttcctcgcc gagtggggag gggggccaca gcctaaggag ctgctcctca 1140

aaagcccggg cggacggcgg cgacggcgcg ggttcctagc ggcagcctca gctccaccac 1200

ctccctcacc ctccccgcgc ttcctctccg cccctcgcgc tccagccact cggccgcagc 1260

cggcgctgtc ctccgccccc cggagccgcc gcgccagacc 1300

<210> 8
<211> 1300
<212> DNA
<213> Homo Sapiens

<400> 8
cccgacttta gaaggacttt cctgctggct ggctcgcagc gtccccggag agcaagcgag 60

cgcgccgcag cgggagacta ggttctctcc gagcgtcctc cgcagaggcg ccgcgagagg 120

agcagggagc cgcagctcgc cgtgttgtct tcatttcgtg caataaagaa ttgtcattag 180

gtttgcgtat ggcatgtgct attaccccac cgtaaaacta aaattagcaa aatgtcagga 240

atggaaagat tgattcacca agatgcaatt atcatttaaa agtgcttgat tgaggtactg 300

atgttcagtg atttattctg cataccatat acataattaa agtagtgtag tggagtaatt 360

tatcaatcta gttgagactg gaggggtgag gagggagctg ctgtatgttt gttttattaa 420

aatgctccga ggtctagtcc cgcccccctt ttgcaagagt gaaactgatg atttctccag 480

ctcgcgagga aagagtcaac ggtttgggat tgtgggggag agagagacgg agagaaaaag 540

gcagcgcgaa gcaaaggcaa ggacaaaatt aaataaaggg ggaaaaaagg aggcaaaaga 600

catttcatcg gacgtgctgc ttagaacccc aaccattcgt gctccgtctt ccctaccacc 660

cccgcctccc ctcccagtat ccttcaatcc cccccgcccc caccaccccc agtctttttt 720

acgcgatgtt tcaaacgctg tgagctgttc tccttttccc attcgtcttc tgtcacttcc 780

ttcctggacg cagttttctg gacgagtctg gttactttta atccgaccgg ccgctgagag 840

ccactttctc ctcctcctcc tcctcctcct tctcttcctc ctccttcttc ctcctcctcc 900

tcctcttccg agcggcctcg gcgcgcgcga atgcgcggcc ccgcgccccc cccctcgcgc 960

gcgctcccct cgcgcgcgcg cacacacgca cacatcgtct ccagctctct gctcgctctg 1020

ctcgcagtca cagacacttg agcacacgcg tacacccaga catcttcggg ctgctattgg 1080

attgactttg aaggttctgt gtgggtcgcc gtggctgcat gtttgaatca ggtggagaag 1140

cacttcaacg ctggacgaag taaagattat tgttgttatt tttttttct ctctctctct 1200

ctcttaagaa aggaaaatat cccaaggact aatctgatcg ggtcttcctt catgtaagta 1260

cccctgatat ttctcgagga aatagaaaac ccgggtattg 1300

<210> 9
<211> 1300
<212> DNA
<213> Homo Sapiens

<400> 9
aacctgggaa gcagaggttg cagtgagccg agatcatgcc actgcactcc agcctgggca 60

acatagtaag actctgtctc aaaacaaaac aaaaacacaa caacaacaaa actggttcta 120

aatgctccgg gcaagtacat actgcttttt ctttttctg ttttttttc cctaaggcag 180

ccctattgaa gagtctgtca tctacaatat tttactaatt taaaaacact tgtcaagaag 240

ggtaaagtat gttcatcttg attttactct ttgtatcccg gaatcaggaa ctaacagttt 300

tatgatagtg atatagactt tacacacaca ccaagactta aaacacgtgg tgcaggcggg 360

cttttggaaa ttaactgtgc tctggataca gaagtcacaa agctcaggct gcagagacat 420

tgaggggata atcagccttt attgtctctc gtgcactgtg cccagcgctc cctctagcgg 480

ctacaagcgc tcactgccca cactctctac taggctctcc ggaggcctcg gtttcgctgg 540

agagaaagag attgcgcagg cgccgagcag ccgacgtggc cgcaggggac cgtgcctggg 600

ggggtctgcg cggggccgct gactcgcgag gttctctgtc gcctgccgcc ctcgctcctt 660

ttctcccctt ctagagtgtt acggtgactc ttgggtccga aggggacgga cgtcctccca 720

cgtaccattt agaaggtcag agtgggcaga acctagtctg agcaggccgg tgatgaatcc 780

gctggcaaga cgcgcgcgtg ggtactcacg tgacctaggc tgcggcggcg gcgtgctgcg 840

ggctctgtgg cgggagcgag gccgacgggc ggggccgcgc ggccgcgtga cgcgaagcgt 900

tcgagagcgc gcgtcgtgga acgtcttggt tgccacggca agcgcgcgcg cgaggccttg 960

ggaacctcgg gaccggcccc cggcgagcgc agcggcgccc agtgtaaggg agtgggagct 1020

ggtccgtgcc gcggcggccg cgcagggagc tctcgaggca acgccggggc gcccgaggtc 1080

tggaaggcgc aggtggggcg cgggggagtt cacccctgcg cggtcccttg ggtgggcggg 1140

gtgggcgccc caggccccgc ggactcggct gggaggctga gcgggcgacg gtcacgggac 1200

aagggtggag ggggtgggga cctcatccga gtctcagaga tggaattttc gaggaaggga 1260

gcgatttcga gagagaatag tttgagaagt tgttctcagt 1300

<210> 10
<211> 1300
<212> DNA
<213> Homo Sapiens

<400> 10
gttgctgatg ctgtttataa aagtgaatag atagggaaat actgattcat atctcgtgaa 60

atgaaaatga aaggcttttt agtgacttag aattttaaat atttctacat gagagagcag 120

tagtatattt agaaataaca aagtaactgg caactgttta aaactgaagt taattcacag 180

ctatccagtg caaaacttca cctcaggtga tacacttttg acaggtaata catacagtaa 240

gtgtattttt agggaaacag tttcattgtt gaaccaagat aatcatcatt agaatgttgt 300

atctgattta agtgtcttta aacttaccaa ggtattagat tttagtttga attgtctgga 360

gtagcggtag cggttggcac atttgttctt aaagggccag aaaataaata ttttaggctt 420

tatgggccat gtggtttctg ccacaagtcc tcagctctgt tcttttagtg tgaaagcagc 480

catagatgat acctaaatga atgagtatgg ctgcatccca ataaaacttc atttacaaaa 540

ctacatggct ggcctaagct ttagtctgtc tgcctaggga gtagtttact gagccactaa 600

tctaaagttt aatactgtga gtgaatacca gtgagtacct ttgttaatgt ggataaccaa 660

tacttggcta taggaagttt tttagttgtg tgttttatta cacgtatttg actttgtgaa 720

taattatggc ttataatggc ttgtctgttg gtatctatgt atagcgttta cagtttcctt 780

taaaaaacat gcattgagtt ttttaatagt ccaaccctta aaataaatgt gttgtatggc 840

cacctgatct gaccactttc tttcatgttg acatctttaa ttttaaaact gttttattta 900

gtgcttaaat cttgtttaca aaattgtctt cctaagtaat atgtctacct tttttttgg 960

aatatggaat attttgctaa ctgtttctca attgcatttt acagatcagg agaacctcag 1020

tctgacgaca ttgaagctag ccgaatgtaa gtgtaacttg gttgagactg tggttcttat 1080

tttgagttgc cctagactgc tttaaattac gtcacattat ttggaaataa tttctggtta 1140

aaagaaagga atcatttagc agtaaatggg agataggaac atacctactt tttttcctat 1200

cagataactc taaacctcgg taacagttta ctaggtttct actactagat agataaatgc 1260

acacgcctaa attcttagtc tttttgcttc cctggtagca 1300

<210> 11
<211> 1300
<212> DNA
<213> Homo Sapiens

<400> 11
caaaaaaatc catcccttga taggaagtca gtatatctgg gttttagtca gtccttcaac 60

actagttcca aaatcttggg caagtcagtt aatcactcgg aggctgctta attttttttt 120

tttttaattc ttagtatatt atctgtcttg attaggtaaa gccaaagttg ttataagact 180

caaggtacta ttgaccaaaa tgcttcagaa actataacat tacataaaag tacaaggaat 240

tattacattt tctcaaatga agagatcatt taagaaaaac aaaatgctgg aaaatagtcc 300

ttaaaaactt ttctataata attactcttc tgatgaattt tacattgaca cctaatttga 360

agctttgaat attatttttt gaacaatgaa ttgggtttat atattaaagt gttctaacca 420

aaggtttgtt taatttatta gagttattaa gcctacgctc agatcaaggt agcagctaga 480

ctggtgtgac aacctgtttt taatcagtga ctcaaagctg tgatcaccct gatgtcaccg 540

aatggccaca gcttgtaaaa ggtaattttg aattatttta cagcctttaa aaggctgtca 600

ttgtaaagtg aaatacatac tgtaaaaatg aagacaatgt atagttggca ggaatactgc 660

taagaatagt gggcctgaga gttgacctac ttcatttaat ctccttaaaa tattttagtg 720

tcttttttc tcattaaaaa tatcagtagc cactatcaaa gacctatctg tatgtataaa 780

caggaaagta tttctcaaaa aaacttcaag gttttaaaca ttttactata aacattatag 840

aaaacattaa aaatttctta atatgcattt taaagtatgc cttttatgtg gtgaacgttt 900

```
taactaaaca tttctctaga agtttttata acattaataa aactagtata cttttctctc      960

ctagagagtt acagtggagg taaaaggagt ggcttgcagg atggagaagc tgcaccagtg     1020

ttattggaag tgagccacca tttgaatttg ctagctcatg ctgcagtatt cagattagtg     1080

ggtgttttgt gatcatattt tctgcgattc aaatcaagac ctaaaattga ttctcattct     1140

gagatttaca ttttattatc caatgttatt tggttattgt ttcagtttta aaatttctga     1200

cttgttattc tgacacactt taggataacc taagtcaata gtttaataca aaaatttccc     1260

tttggttata tgttttgaat taggttatat gttctgcatt                           1300


<210>  12
<211>  1300
<212>  DNA
<213>  Homo Sapiens

<400>  12
agtacttcaa gggcttatca acaaagttgc aagttgtaac actatgaatt gttagtgata       60

ctcttttggc tttgctagca agtgttgtaa agctatacac acacacacac acacacacac      120

acatacacac acacaccttt taaaatggtg accctggtac caaatatgac tttaaatgga      180

tttaatttta atggctttaa ctacgttcag ctgtcatatg gatcaaaatt agcctctatc      240

cagctggggt caaccaggga gccacttttc ttaaccgacg acctactgaa cgtcaacaac      300

tgcaggagac gggactttac cttcgtctct ggtaaactag ttgacacatc ctgtgttggc      360

aagaggccta agtagatgac cttggtcctc taaaatctgg cctgcactct cggggcaccc      420

ctgcaacatc tacaaaggca gctccagata gaaaagggtt ggggtcgaaa agccaataac      480

ggcaggcacc tgccccgcct cggggctggg gggctattcc agcggcttca gctaactttc      540

agagccattc gtttcccaac aaagtctgag gcgttcctct gctgggtaca ccaaggggct      600

ctgcaaccct cctggggggg ggggtgccca gagggcttcc ggaagtccca ggtttattct      660

ttcgggtcac agacagcaga aactaaaaag agggattacc ctttctgtcc agtcgcaaga      720

tggcgaccga gcctggtggg actccgaggg gccgcaggcc acctcctctt cccaatggcc      780

cgtgcgccgg cggcgacggc aagcgggagg gaggcggggc cggcgaagga aggaggggcg      840

gagcgcggcg ccctcccgcg cgtcttggcc ccgccccacg tccccgcgtc ccggcctgga      900

gccctcgccc ggccgggcgg cgcgcgctgc ctgccgggat actcggcccg cccagccagt      960

cctcccgtct tgcgccgcgg ccgcgagatc cgtgtgtctc ccaagatggt ggcgctgggc     1020

tcggggtgac tacaggagac gacggggcct tttcccttcg ccaggacccg acacaccagg     1080

cttcgctcgc tcgcgcaccc ctccgccgcg tagccatccg ccagcgcggg cgcccgccat     1140

ccgccgccta cttacgcttc acctctgccg acccggcgcg ctcggctgcg ggcggcggcg     1200

cctccttcgg ctcctcctcg gaatagctcg cggcctgtag cccctggcag gagggcccct     1260
```

cagcccccg gtgtggacag gcagcggcgg ctggcgacga 1300

<210> 13
<211> 1300
<212> DNA
<213> Homo Sapiens

<400> 13
gcgaaaagcc ttaggaccgc ttgttttaga cggctgggga atatttattc cttgttccac 60

tgatgggaaa atcagcgtct ggcaggcgct gattggtgga aaggaaaatg gtgatagtgg 120

cgtggaaaga ggatttgctg agccttctcc tgcctcctca acctgtgact cttccttagt 180

agtctccctt tcaccctcag gaccctttcc ggctcttcct agattaagag caaacgaaaa 240

ccttgaagat atttgaacta aagcgacccc taacgttgta acctgtgacc gtgattaaat 300

ttcagcgatg cgagggcaaa gcgctctcgg cggtgcggtg tgagccacct cccggcgctg 360

cctgtctcct ccagcagctc cccaagggat aggctctgcc cttggtggtc gaccctcagg 420

ccctcggctc tcccagggcg actctgacga ggggtagggg gtggtccccg ggaggaccca 480

gaggaaaggc ggggacaaga agggaggga aggggaaaga ggaagaggca tcatccctag 540

cccaaccgct cccgatctcc acaagagtgc tcgtgaccct aaacttaacg tgaggcgcaa 600

aagcgccccc actttcccgc cttgcgcggc caggcaggcg gctggagttg atggctcacc 660

ccgcgccccc tgccccatcc ccatccgaga tagggacgag gagcacgctg cagggaaagc 720

agcgagcgcc gggagagggg cgggcagaag cgctgacaaa tcagcggtgg gggcggagag 780

ccgaggagaa ggagaaggag gaggactagg aggaggagga cggcgacgac cagaaggggc 840

ccaagagagg gggcgagcga ccgagcgccg cgacgcggaa gtgaggtgcg tgcgggctgc 900

agcgcagacc ccggccccggc ccctccgaga gcgtcctggg cgctccctca cgccttgcct 960

tcaagccttc tgcctttcca ccctcgtgag cggagaactg ggagtggcca ttcgacgaca 1020

ggttagcggg tttgcctccc actcccccag cctcgcgtcg ccggctcaca gcggcctcct 1080

ctggggacag tcccccccgg gtgccgcctc cgcccttcct gtgcgctcct tttccttctt 1140

ctttcctatt aaatattatt tgggaattgt ttaaattttt tttttaaaaa aagagagagg 1200

cggggaggag tcggagttgt ggagaagcag agggactcag gtaagtacct gtggatctaa 1260

acgggcgtct ttggaaatcc tggagaacgc cggatgggag 1300

<210> 14
<211> 1300
<212> DNA
<213> Homo Sapiens

<400> 14
gaatggtcgt gggcaccggg agggggtggt gctgccatga ggacccgctg ggccaggtct 60

ctgggaggtg agtacttgtc cctttgggga gcctaaggaa agagacttga cctggctttc 120

gtcctgcttc tgatattccc ttctccacaa gggctgagag attaggctgc ttctccggga 180

tccgcttttc cccgggaaac gcgaggatgc tccatggagc gtgagcatcc aacttttctc 240

tcacataaaa tctgtctgcc cgctctcttg gtttttctct gtaaagtaag caagctgcgt 300

ttggcaaata atgaaatgga agtgcaagga ggccaagtca acaggtggta acgggttaac 360

aagtgctggc gcggggtccg ctagggtgga ggctgagaac gccccctcgg gtggctggcg 420

cggggttgga gacggcccgc gagtgtgagc ggcgcctgct cagggtagat agctgagggc 480

gggggtggat gttggatgga ttagaaccat cacacttggg cctgctgttt gcctgagttt 540

gaaccacacc ccgagtgagc agttagttct gttgcctacg cctttccacc atcaacctgt 600

tagccttctt ctgggattca tgttaaggat acccctgacc ctaagcctcc agcttccatg 660

cttctaactc atactgttac cctttagacc ccgggaattt aaaaaagggg ttaatctttt 720

catgcaactc cacttctgaa atgcagtaat aacaactcag aggattcatc ctaatccgtg 780

gttaggtggc tagactttta ctagccaaga tggatgggag atgctaaatt tttaatgcca 840

gagctaaaaa tgtctgcttt gtccaatggt taaatgagtg tacacttaaa agagtctcac 900

actttggagg gtttctcatg atttttcagt gtttttgtt tatttttccc cgaaagttct 960

cattcaaagt gtattttatg ttttccagtg tggtgtaaag gaattcatta gccatggatg 1020

tattcatgaa aggactttca aaggccaagg agggagttgt ggctgctgct gagaaaacca 1080

aacagggtgt ggcagaagca gcaggaaaga caaaagaggg tgttctctat gtaggtaggt 1140

aaaccccaaa tgtcagtttg gtgcttgttc atgagtgatg ggttaggata atcaatactc 1200

taaatgctgg tagttctctc tcttgattca tttttgcatc attgcttgtc aaaaaggtgg 1260

actgagtcag aggtatgtgt aggtaggtga atgtgaacgt 1300

<210> 15
<211> 1300
<212> DNA
<213> Homo Sapiens

<400> 15
gccaaagtga tatattaagg tcaccacctt ggtcacctgt ccatctatat ttttggagta 60

agcaacagaa ttaggagtct gaacatagga attaagaagc tctcgaatat aacagtggaa 120

gaccagcacc ttcccaacct cttgtcaggg cagggatact ctatctctgc gtcactcaac 180

ctgccttggc ctcacagctc ttccttttgc ttaagtcttc aaaccatcaa cagtgatagc 240

cactaatttt agcaggtggc attagttgtc agcaaatgtt ttttcatatt taatgaatgt 300

ttattataat agaataaaat agaaatgttt attaataaaa cttgaaattt gtcccagaag 360

gataaaaacg tagattattt acataaaatg gccaagatct gacaacagaa ctgtttcctg 420

aagatttact tcacagatta ttttgaaaac accaactgtg ttcaggaaaa ttttctctcc 480

ttttttttcat aaaattttca aacaagtgag aatgctctaa aaatgatggt gtggagccta 540

gagaaaatgt tttctgtgtg ctatagatga acataaacta tatatatata ttactttccc 600

aggttgaagt gtttctttca aaaataaaat ttgattattt tcaatttttt gagggatgct 660

gatttttaaa aaggaaataa agagaaatta ttaggaagca tatacacagc cagtgagaag 720

aggaaagaag aaaaggccaa ggccagagga agggggtttg gggtggggga aaggaagggt 780

acagaccctc ccaaataacc gaagatgggt aaactccaac cgcaaagcga tgatgccact 840

tgtgggccct cagattggag aggacggaga tgagtgacgg ggctgtgcgc actggggcac 900

cgcgcacaga cgggaggagg ggggtgtctg tgtgttccaa agggggagt accagttaac 960

tggccgccgg cccgtggggg ttggtgagaa ggagggtgag cttggcggtg acgcacggcc 1020

ctcacgtgac cgggagctgc agagctacgc agccttcggt gcagtcgtca ctcgtgtctc 1080

gctaccagct ccccgctgcc ctgcgctcgg cgggctggca tccggcccgg gggaaagcgg 1140

accaggtaag ggccccgggg cccgcgcgcc ctggctaggc cagcagaggc tcaccgaccc 1200

ccccgacgag cgcagaacag cgcaggggat ccccgccagc tttctccaca ccccaacatt 1260

cttgggaaca tagtggtccc ctggggtggg gaagcagtgg 1300

<210> 16
<211> 1300
<212> DNA
<213> Homo Sapiens

<400> 16
actgtagtga acaggagagc acacttctta tacaaatggc aagggcaact cacctctgcc 60

actgtgggca tcccagctct tcccattttc agaagaagcc agaaatccaa tttctttaca 120

ggtgaaatct cctggtttaa aaatgctcct ttcgaaatat tttaaaatat aatgctctaa 180

acacaaaagt ctgcagcccc ctttccagca gcttgtggtc ctggcctgtg ccatgcttta 240

gtgccctaac gatgagcccc agcaattccc agcgcctcct gcttctccaa gtctggcctc 300

ttttcccctc tgtaccgccg cttctcccca ctctccgcag ctcggggtca ctttctttac 360

tgatctcccc tctcttctgg gagaggcact tgctgaacat ctttccgccc ggagcctccc 420

caggagctgc agaggggcag ctggagagat gctttctgac gtagcatctc ccacctttct 480

ccttggggac cctgaggaag gggacaaggg ctcgctctgc aggtcctcac tgtaactgga 540

aaaacacgac ctcgccctcg ggaaggcttt ctgtgcgcct cacctcagga tgagggtggg 600

tgtaggggac acctcccaga aacccctaac ctcccagtcg gttaaagaag aggggatagg 660

gtcaagggat gcgacagagc tgtgtggttt ccggatggga aacctcagtc gtttaggcac 720

ccctccgctc gagtcacttc cgaagcagtc gattcttggg gagaagcgct gcggaaaggg 780

gcgactccga tgcagatggc cctgtcccgg cgccccaggt cgtcgcgcgc gcagctgcgg 840

tagtcactgc gcctccccgc ccccactcct ggatgccccc cttccctctc ccggccagac 900

tctgagcagg agctccgccc ccagcgcgcc gccccagccc cggcgcctta aaagccgggc 960

gcaccgcccc gccgcgccct gcctgccgca cctctccttt cttctgtagc tcgcgttgaa 1020

gccgcacgtc cggccccgat cccggcacca tgagcttcgg ctcggagcac tacctgtgct 1080

cctcctcctc ctaccgcaag gtgttcgggg atggctctcg cctgtccgcc cgcctctctg 1140

gggccggcgg cgcgggcggc ttccgctcgc agtcgctgtc ccgcagcaat gtggcctcct 1200

cggccgcctg ctcctcggcc tcgtcgctcg gcctcggcct ggcctatcgc cggccgccgg 1260

cgtccgacgg gctggacctg agccaggcgg cggcgcgcac 1300

<210> 17
<211> 300
<212> DNA
<213> Homo Sapiens

<400> 17
cagctctgga cgcccgcagc ggggcctcga agaggttcag ggcggtgccc gcggcgctcg 60

ggccgggtct cccggggcgt ggggcggggg gcggggttgg gcggcggccg gggctcctcc 120

ctcttctgcc ccgggctccc ctgctcttaa cccgcgcgcg gggcgcccca ggccactggg 180

ctccgcggag ccagcgagag gtctgcgcgg agtctgagcg gcgctcgtcc cgtcccaagg 240

ccgacgccag cacgccgtca tggcccccgc agcggcgacg gggggcagca ccctgcccag 300

<210> 18
<211> 300
<212> DNA
<213> Homo Sapiens

<400> 18
cagctctgga cgcccgcagc ggggcctcga agaggttcag ggcggtgccc gcggcgctcg 60

ggccgggtct cccggggcgt ggggcggggg gcggggttgg gcggcggccg gggctcctcc 120

ctcttctgcc ccgggctccc ctgctcttaa cccgcgcgcg gggcgcccca ggccactggg 180

ctccgcggag ccagcgagag gtctgcgcgg agtctgagcg gcgctcgtcc cgtcccaagg 240

ccgacgccag cacgccgtca tggcccccgc agcggcgacg gggggcagca ccctgcccag 300

<210> 19
<211> 300
<212> DNA
<213> Homo Sapiens

<400> 19
cagctctgga cgcccgcagc ggggcctcga agaggttcag ggcggtgccc gcggcgctcg 60

ggccgggtct cccggggcgt ggggcggggg gcggggttgg gcggcggccg gggctcctcc 120

ctcttctgcc ccgggctccc ctgctcttaa cccgcgcgcg ggggcgccca ggccactggg 180

ctccgcggag ccagcgagag gtctgcgcgg agtctgagcg gcgctcgtcc cgtcccaagg 240

ccgacgccag cacgccgtca tggccccccgc agcggcgacg gggggcagca ccctgcccag 300

<210> 20
<211> 300
<212> DNA
<213> Homo Sapiens

<400> 20
cagctctgga cgcccgcagc ggggcctcga agaggttcag ggcggtgccc gcggcgctcg 60

ggccgggtct cccggggcgt ggggcggggg gcggggttgg gcggcggccg gggctcctcc 120

ctcttctgcc ccgggctccc ctgctcttaa cccgcgcgcg ggggcgccca ggccactggg 180

ctccgcggag ccagcgagag gtctgcgcgg agtctgagcg gcgctcgtcc cgtcccaagg 240

ccgacgccag cacgccgtca tggccccccgc agcggcgacg gggggcagca ccctgcccag 300

<210> 21
<211> 300
<212> DNA
<213> Homo Sapiens

<400> 21
gggcagggcg gtgtttccgt tttccttggc cgggttcagc accctacact caataagcgt 60

gggctttcta gcaggactgg acactcaggc ggtgtgtcaa tctttagaca atctttatag 120

agtgcataga tgtttaattt cataaaattt aggaaagctt catttcagga aggaatttta 180

ttcctctttt tctcccttag ggcaagcact ttaacctttt aagcccaacc agatgagttg 240

cctgcagttt tggaggcctt cagagcattt cactagacct ctgtctgtgt cggtccagtg 300

<210> 22
<211> 300
<212> DNA
<213> Homo Sapiens

<400> 22
agaggccccg caccgaggcg gaggctggca aaggggagtg gaaagggagg atggatgggg 60

ccggggggtg gggtggtgat gagggcgacg aaggaggggg tgtcattttc tttttctttc 120

ttttttttaaa aaaagtattt ctctcgcgag aaaccgctgc gcggacgata cttgaagagg 180

tggggaaagg agggggctgc gggagccgcg gcagagactg tgggtgccac aagcggacag 240

gagccacagc tgggacagct gcgagcggag ccgagcagtg gctgtagcgg ccacgactgg 300

<210> 23
<211> 300
<212> DNA
<213> Homo Sapiens

<400> 23
attccggggg tctcgctcct tggcatagtg gtcgcttaac tccagcgcct tcccagtgcc 60

tcccaaacct ctcctcctcc tgcccggggc tgttctagga ggagcctaaa tgtccgcttc 120

cccagctgaa ggggattgtg gtgcgtaaac aggattaact ccctcgcccc cagctgcgcg 180

gatctgcgcg gcggcctgcg ggccagagcg agccttcggg tgcgtggagg gcgcagcact 240

ggagcggggg atggggaagc gcgcggcctt ggagccgtct cttgggcccg cttcctcgcc 300

<210> 24
<211> 300
<212> DNA
<213> Homo Sapiens

<400> 24
gacgagtctg gttactttta atccgaccgg ccgctgagag ccactttctc ctcctcctcc 60

tcctcctcct tctcttcctc ctccttcttc ctcctcctcc tcctcttccg agcggcctcg 120

gcgcgcgcga atgcgcggcc ccgcgccccc cccctcgcgc gcgctcccct cgcgcgcgcg 180

cacacacgca cacatcgtct ccagctctct gctcgctctg ctcgcagtca cagacacttg 240

agcacacgcg tacacccaga catcttcggg ctgctattgg attgactttg aaggttctgt 300

<210> 25
<211> 300
<212> DNA
<213> Homo Sapiens

<400> 25
ggtactcacg tgacctaggc tgcggcggcg gcgtgctgcg ggctctgtgg cgggagcgag 60

gccgacgggc ggggccgcgc ggccgcgtga cgcgaagcgt tcgagagcgc gcgtcgtgga 120

acgtcttggt tgccacggca agcgcgcgcg cgaggccttg ggaacctcgg gaccggcccc 180

cggcgagcgc agcggcgccc agtgtaaggg agtgggagct ggtccgtgcc gcggcggccg 240

cgcagggagc tctcgaggca acgccggggc gcccgaggtc tggaaggcgc aggtggggcg 300

<210> 26
<211> 300
<212> DNA
<213> Homo Sapiens

<400> 26
ttttaatagt ccaaccctta aaataaatgt gttgtatggc cacctgatct gaccactttc 60

tttcatgttg acatctttaa ttttaaaact gttttattta gtgcttaaat cttgtttaca 120

aaattgtctt cctaagtaat atgtctacct tttttttgg aatatggaat attttgctaa 180

ctgtttctca attgcatttt acagatcagg agaacctcag tctgacgaca ttgaagctag 240

ccgaatgtaa gtgtaacttg gttgagactg tggttcttat tttgagttgc cctagactgc 300

<210> 27
<211> 300
<212> DNA
<213> Homo Sapiens

<400> 27
aaacttcaag gttttaaaca ttttactata aacattatag aaaacattaa aaatttctta 60

atatgcattt taaagtatgc cttttatgtg gtgaacgttt taactaaaca tttctctaga 120

agtttttata acattaataa aactagtata cttttctctc ctagagagtt acagtggagg 180

taaaaggagt ggcttgcagg atggagaagc tgcaccagtg ttattggaag tgagccacca 240

tttgaatttg ctagctcatg ctgcagtatt cagattagtg ggtgttttgt gatcatattt 300

<210> 28
<211> 300
<212> DNA
<213> Homo Sapiens

<400> 28
aagcgggagg gaggcggggc cggcgaagga aggaggggcg gagcgcggcg ccctcccgcg 60

cgtcttggcc ccgccccacg tccccgcgtc ccggcctgga gccctcgccc ggccgggcgg 120

cgcgcgctgc ctgccgggat actcggcccg cccagccagt cctcccgtct tgcgccgcgg 180

ccgcgagatc cgtgtgtctc ccaagatggt ggcgctgggc tcggggtgac tacaggagac 240

gacggggcct tttcccttcg ccaggacccg acacaccagg cttcgctcgc tcgcgcaccc 300

<210> 29
<211> 300
<212> DNA
<213> Homo Sapiens

<400> 29
gaggactagg aggaggagga cggcgacgac cagaaggggc ccaagagagg gggcgagcga 60

ccgagcgccg cgacgcggaa gtgaggtgcg tgcgggctgc agcgcagacc ccggcccggc 120

ccctccgaga gcgtcctggg cgctccctca cgccttgcct tcaagccttc tgcctttcca 180

ccctcgtgag cggagaactg ggagtggcca ttcgacgaca ggttagcggg tttgcctccc 240

actcccccag cctcgcgtcg ccggctcaca gcggcctcct ctggggacag tcccccccgg 300

<210> 30
<211> 300
<212> DNA
<213> Homo Sapiens

<400> 30
ctagccaaga tggatgggag atgctaaatt tttaatgcca gagctaaaaa tgtctgcttt 60

gtccaatggt taaatgagtg tacacttaaa agagtctcac actttggagg gtttctcatg 120

atttttcagt gttttttgtt tatttttccc cgaaagttct cattcaaagt gtattttatg 180

ttttccagtg tggtgtaaag gaattcatta gccatggatg tattcatgaa aggactttca 240

aaggccaagg agggagttgt ggctgctgct gagaaaacca aacagggtgt ggcagaagca 300

<210> 31
<211> 300
<212> DNA
<213> Homo Sapiens

<400> 31
gaagatgggt aaactccaac cgcaaagcga tgatgccact tgtgggccct cagattggag 60

aggacggaga tgagtgacgg ggctgtgcgc actggggcac cgcgcacaga cgggaggagg 120

ggggtgtctg tgtgttccaa agggggagt accagttaac tggccgccgg cccgtggggg 180

ttggtgagaa ggagggtgag cttggcggtg acgcacggcc ctcacgtgac cgggagctgc 240

agagctacgc agccttcggt gcagtcgtca ctcgtgtctc gctaccagct ccccgctgcc 300

<210> 32
<211> 300
<212> DNA
<213> Homo Sapiens

<400> 32
cctgtcccgg cgccccaggt cgtcgcgcgc gcagctgcgg tagtcactgc gcctccccgc 60

ccccactcct ggatgccccc cttccctctc ccggccagac tctgagcagg agctccgccc 120

ccagcgcgcc gccccagccc cggcgcctta aaagccgggc gcaccgcccc gccgcgccct 180

gcctgccgca cctctccttt cttctgtagc tcgcgttgaa gccgcacgtc cggccccgat 240

cccggcacca tgagcttcgg ctcggagcac tacctgtgct cctcctcctc ctaccgcaag 300

<210> 33
<211> 1300
<212> DNA
<213> Homo Sapiens

<400> 33
atttccttct ttccccctct gcacgcttgc tagccccagc gatcgctgct ggcccccggg 60

taggaaagtg gggttcctgg ccgtttctgc gacgctggcc tagggcttgc agctgctgtt 120

gagtgaaagc acgcagactg gcgggagccg atcatttctc gaatgaagaa gaaaaagcgc 180

aattccctcc ttatgctcta gggaattgag ccgcgtccca gatcacccat tccagaaatg 240

tgaaaccggg ccctcacaaa gtcgtctctg gtgaagaggt ggcgtgcggg gtgggggttg 300

gtggagggtg aaggcataag caaacatatt ttaaaatcca gatcgtagga agtgtcacct 360

ggcccctcac ccaggcatgc tttctggggg aagcgcaggg ccaagctttc cctagaaaag 420

ctggggcgaa gagagagcag gcggcggcta aggagctcct ggcaggctgg gaaggtggag 480

aagtggggtg aggtattttt ctagaaagtg tagccctagc tcatctccta gattggggaa 540

gagggaactg agggaggagg gaaggagacc cagggcagct ccaggatagg gaaatgttga 600

agaagggact gcgttctcca accgaaccct ccctcctggg aaccgcagcc cagcgcggta 660

actgagttac cgcaaccggg cggtggggag gaagggtggt ccaggaaacc ggcgagggag 720

aaaagcggtg gaagggagag tcttctccct ggagcggccc cagcagtaca aagtgctggt 780

cacagcgccc cttccgcccc tagattgacg agcagtggcg tggagccagc gcggaggctg 840

ccccctcccc ctcccgagcc cgcagcgcgg agcgcggttt agcaccaacg gagccggggg 900

cggcgtcttt gggatggaaa agggccaaag gggaggagtg gggtgggggt gggggtttca 960

ctggtccact ataaaaggac cgctcggctg cccggttctt gcactcgctg gaaagcggct 1020

ccgagccagg ggctattgca aagccagggt gcgctaccgg acggagaggg gagagccctg 1080

agcagagtga gcaacatcgc agccaaggcg gaggccgaag aggggcgcca ggcaccaatc 1140

tccgcgttgc ctcagccccg gaggcgcccc agagcgcttc ttgtcccagc agagccactc 1200

tgcctgcgcc tgcctctcag tgtctccaac tttgcgctgg aagaaaaact tcccgcgcgc 1260

cggcagaact gcagcgcctc cttttagtga ctccgggagc 1300

<210> 34
<211> 1300
<212> DNA
<213> Homo Sapiens

<400> 34
tccgcacatt cgagcaaaga caggctttag cgagttatta aaaacttagg ggcgctcttg 60

tcccccacag ggcccgaccg cacacagcaa ggcgatggcc cagctgtaag ttggtagcac 120

tgagaactag cagcgcgcgc ggagcccgct gagacttgaa tcaatctggt ctaacggttt 180

cccctaaacc gctaggagcc ctcaatcggc gggacagcag ggcgcggtga gtcaccgccg 240

gtgactaagc gaccccaccc ctctccctcg ggctttcctc tgccaccgcc gtctcgcaac 300

tcccgccgtc cgaagctgga ctgagcccgt taggtccctc gacagaacct cccctccccc 360

caacatctct ccgccaaggc aagtcgatgg acagaggcgc gggccggagc agccccccctt 420

tccaagcggg cggcgcgcga ggctgcggcg aggcctgagc cctgcgttcc tgcgctgtgc 480

gcgcccccac cccgcgttcc aatctcaggc gctctttgtt tctttctccg cgacttcaga 540

tctgagggat tccttactct ttcctcttcc cgctcctttg cccgcgggtc tccccgcctg 600

accgcagccc cgagaccgcc gcgcacctcc tcccacgccc ctttggcgtg gtgccaccgg 660

acccctctgg ttcagtccca ggcggacccc cccctcaccg cgcgaccccg cctttttcag 720

caccccaggg tgagcccagc tcagactatc atccggaaag cccccaaaag tcccagccca 780

gcgctgaagt aacgggacca tgcccagtcc caggccccgg agcaggaagg ctcgagggcg 840

cccccacccc acccgcccac cctccccgct tctcgctagg tccctattgg ctggcgcgct 900

ccgcggctgg gatggcagtg ggaggggacc ctctttccta acggggttat aaaaacagcg 960

ccctcggcgg ggtccagtcc tctgccactc tcgctccgag gtccccgcgc cagagacgca 1020

gccgcgctcc caccacccac acccaccgcg ccctcgttcg cctcttctcc gggagccagt 1080

ccgcgccacc gccgccgccc aggccatcgc caccctccgc agccatgtcc accaggtccg 1140

tgtcctcgtc ctcctaccgc aggatgttcg gcggcccggg caccgcgagc cggccgagct 1200

ccagccggag ctacgtgact acgtccaccc gcacctacag cctgggcagc gcgctgcgcc 1260

ccagcaccag ccgcagcctc tacgcctcgt ccccgggcgg 1300

<210> 35
<211> 1300
<212> DNA
<213> Homo Sapiens

<400> 35
cttcctctac ctcgctccct cccagcctgc ccatcttttg tgcccgtctt ttgtgccggc 60

caccagtctt tacaaacagt gccctggggg cccatactcc agctttccag agcgcacagc 120

tgcaagggcg gctgtgtgtt agagcccgtg atgccttcga gtattgagtc atgccattca 180

tttaagaagg aaaaatctct ccaaaacggg gaaatggtgg gcagtcctgt gtgactggtg 240

agactcttgt aggggcgttt ctacaacgac gaaacccttc ctaggcactc actccaacag 300

aataacaagc ccattttatt agtatttcgt tttccatgta aagttctgct catacgaata 360

tatttataat tctgattttt ttacggcatt ggggagcaca ccgacaggct gctgaacggt 420

ggctggagat tcgagggaaa acgaagttcg ccgaggcggc ctcgggcggg caggtcccgg 480

gctccatcac agggcacacg cggctaccag ggacgcagcc ccccaacaca cacacacaca 540

cacacacaca cacacacaca cacacacaca ccctctccca ctcatgcctg gcaacccagc 600

agaaacttcg gactggggca aaacaagccc gggccccggc ggcacgcggg gctaggcgcg 660

ttcccgccag tacctggtcg cgaggccgct cgcggggtgc cctgcgtgcc ccccactccc 720

gcagcccgcg ccctgctcgc tcactgtggg ggcgcagcgg ccaggcttct ctgtttgttg 780

tttaaagaaa tcctagggcg ggcgagcggc ggcatctagg ggagggggcg cagccagaat 840

tcccttccag caagcgcgtg aggggcattc tcaacgcaaa accagaccca gaaagtagtg 900

accagccctc ctcggattac ccttcattgg ctcctccctt gctcccccca ccctccagat 960

ttgcataaaa aaggccaaga aaactctggc tgtgccccag caacggctca ttctgctccc 1020

ccgggtcgga gcccccccgga gctgcgcgcg ggcttgcagc gcctcgcccg cgctgtcctc 1080

ccggtgtccc gcttctccgc gccccagccg ccggctgcca gcttttcggg gccccgagtc 1140

gcacccagcg aagagagcgg gcccgggaca agctcgaact ccggccgcct cgcccttccc 1200

cggctccgct ccctctgccc cctcggggtc gcgcgcccac gatgctgcag ggccctggct 1260

cgctgctgct gctcttcctc gcctcgcact gctgcctggg 1300

<210> 36
<211> 1300
<212> DNA
<213> Homo Sapiens

<400> 36
ttttctattt aaatatatat tatatattta aaaaagtgtc ctcccagagc taataccgtt 60

gctagcagct cttcctgccg ccacaccggg caaagtccac ccactgcccc agtgttgagg 120

gccaccatgg gcggccccac ctggagaggt gctgctcaca gcaaacagct ccaactgcgc 180

cttcgcctcg ccttccaggg agcccagcca ggcccactgg gtatttacaa gcagacctcc 240

ctcgcttcag ccttcctgaa cccctgttag ttgggaaacc acctgtctgc accgcagcta 300

gagaaccgag gagaggagcc gctagtctaa agggctgttg gttgaaatta ggaagcagtg 360

taaagaaaaa gaaaaaaaaa gtttgggagg ccaaggcagg agcatcacct gaggtcagga 420

gttcgagacc agcctggcta acatggtgaa accccgtctc tactaaaaat acaaaaaatt 480

agcgggggca tggtggcacg cggctgtaat cccagctact cgggaggctg aggcaggaga 540

atcgcttgaa cccgggaggc ggaggttgca gtcagcggag atagcgccat tgcactccag 600

cttaggcaac aagagcgaaa ctgtctcaaa aaaaaaaagt cttcataatt tcatgggttt 660

gcaagtatga tccaggctcc ccgcttctct gcaagccaat gcgagttaat tacagcgtcc 720

gccctggtct ctctccaccc cacgccgtga tccattcccc ttctttttct ccccttgtct 780

ctttcctcct ccccctttta tttatgtatt tttggttttg ttttttaagg ggtgttgagc 840

cgcgtctggt tctagtaaac cgaacccgct cgcgagggag gcgattggct cccgcgccgg 900

tgacggacgt ggtaacgagt gcggctcgcc ccgccgggag ctgattggct gcgcggggcg 960

gctccgaggg ctcggccgta ggagccccgc gcactccagc cctgcagcct ccggagtcag 1020

tgccgcgcgc ccgccgcccc gcgccttcct gctcgccgca cctccgggag ccggggcgca 1080

cccagcccgc agcgccgcct ccccgcccgc gccgcctccg accgcaggcc gagggccgcc 1140

actggccggg gggaccgggc agcagcttgc ggccgcggag ccgggcaacg ctggggactg 1200

cgccttttgt ccccggaggt ccctggaagt ttgcggcagg acgcgcgcgg ggaggcggcg 1260

gaggcagccc cgacgtcgcg gagaacaggg cgcagagccg 1300

<210> 37
<211> 24
<212> DNA
<213> Artificial Sequence

<220>
<223> PCR primer

<400> 37

ttcgggtttt tttgttttta attc 24

<210> 38
<211> 25
<212> DNA
<213> Artificial Sequence

<220>
<223> PCR primer

<400> 38
gaaaaccata acgacgtact aacgt 25

<210> 39
<211> 25
<212> DNA
<213> Artificial Sequence

<220>
<223> PCR primer

<400> 39
ttttgggttt ttttgttttt aattt 25

<210> 40
<211> 28
<212> DNA
<213> Artificial Sequence

<220>
<223> PCR primer

<400> 40
acaaaaacca taacaacata ctaacatc 28

<210> 41
<211> 21
<212> DNA
<213> Artificial Sequence

<220>
<223> PCR primer

<400> 41
gtaatttaga tttcggaggg c 21

<210> 42
<211> 19
<212> DNA
<213> Artificial Sequence

<220>
<223> PCR primer

<400> 42
cgaccaaaaa aaacgaaaa 19

<210> 43
<211> 24
<212> DNA
<213> Artificial Sequence

<220>
<223> PCR primer

<400> 43
tttgtaattt agattttgga gggt 24

<210> 44
<211> 22
<212> DNA
<213> Artificial Sequence

<220>
<223> PCR primer

<400> 44
ccaaccaaaa aaaacaaaaa ca 22

<210> 45
<211> 21
<212> DNA
<213> Artificial Sequence

<220>
<223> PCR primer

<400> 45
gtattttttt cgcgagaaat c 21

<210> 46
<211> 19
<212> DNA
<213> Artificial Sequence

<220>
<223> PCR primer

<400> 46
aatcgtaacc gctacaacc 19

<210> 47
<211> 24
<212> DNA
<213> Artificial Sequence

<220>
<223> PCR primer

<400> 47
aaagtatttt ttttgtgaga aatt 24

<210> 48
<211> 22
<212> DNA

<213> Artificial Sequence

<220>
<223> PCR primer

<400> 48
cccaatcata accactacaa cc 22

<210> 49
<211> 22
<212> DNA
<213> Artificial Sequence

<220>
<223> PCR primer

<400> 49
gttattttta attcgatcgg tc 22

<210> 50
<211> 18
<212> DNA
<213> Artificial Sequence

<220>
<223> PCR primer

<400> 50
aaaccgctcg aaaaaaaa 18

<210> 51
<211> 25
<212> DNA
<213> Artificial Sequence

<220>
<223> PCR primer

<400> 51
ttggttattt ttaatttgat tggtt 25

<210> 52
<211> 21
<212> DNA
<213> Artificial Sequence

<220>
<223> PCR primer

<400> 52
ccaaaaccac tcaaaaaaaa a 21

<210> 53
<211> 21
<212> DNA
<213> Artificial Sequence

<220>

<223> PCR primer

<400> 53
tcgtttttttg gtatagtggt c 21

<210> 54
<211> 18
<212> DNA
<213> Artificial Sequence

<220>
<223> PCR primer

<400> 54
caaatccgcg caactaaa 18

<210> 55
<211> 24
<212> DNA
<213> Artificial Sequence

<220>
<223> PCR primer

<400> 55
gttttgtttt ttggtatagt ggtt 24

<210> 56
<211> 21
<212> DNA
<213> Artificial Sequence

<220>
<223> PCR primer

<400> 56
caaatccaca caactaaaaa c 21

<210> 57
<211> 20
<212> DNA
<213> Artificial Sequence

<220>
<223> PCR primer

<400> 57
tggaacgttt tggttgttac 20

<210> 58
<211> 20
<212> DNA
<213> Artificial Sequence

<220>
<223> PCR primer

<400> 58

tacctcgaaa actccctacg 20

<210> 59
<211> 21
<212> DNA
<213> Artificial Sequence

<220>
<223> PCR primer

<400> 59
gtggaatgtt ttggttgtta t 21

<210> 60
<211> 21
<212> DNA
<213> Artificial Sequence

<220>
<223> PCR primer

<400> 60
ttacctcaaa aactccctac a 21

<210> 61
<211> 20
<212> DNA
<213> Artificial Sequence

<220>
<223> PCR primer

<400> 61
cgttgtttgt cgggatattc 20

<210> 62
<211> 20
<212> DNA
<213> Artificial Sequence

<220>
<223> PCR primer

<400> 62
cccgtcgtct cctataatca 20

<210> 63
<211> 21
<212> DNA
<213> Artificial Sequence

<220>
<223> PCR primer

<400> 63
gtgttgtttg ttgggatatt t 21

<210> 64
<211> 21
<212> DNA
<213> Artificial Sequence

<220>
<223> PCR primer

<400> 64
ccccatcatc tcctataatc a 21

<210> 65
<211> 20
<212> DNA
<213> Artificial Sequence

<220>
<223> PCR primer

<400> 65
cgggttgtag cgtagatttc 20

<210> 66
<211> 18
<212> DNA
<213> Artificial Sequence

<220>
<223> PCR primer

<400> 66
cgtcgaataa ccactccc 18

<210> 67
<211> 23
<212> DNA
<213> Artificial Sequence

<220>
<223> PCR primer

<400> 67
gtgtgggttg tagtgtagat ttt 23

<210> 68
<211> 21
<212> DNA
<213> Artificial Sequence

<220>
<223> PCR primer

<400> 68
tcatcaaata accactccca a 21

<210> 69
<211> 20
<212> DNA

<213> Artificial Sequence

<220>
<223> PCR primer

<400> 69
agttaattgg tcgtcggttc 20

<210> 70
<211> 20
<212> DNA
<213> Artificial Sequence

<220>
<223> PCR primer

<400> 70
cgaataacga ctacaccgaa 20

<210> 71
<211> 23
<212> DNA
<213> Artificial Sequence

<220>
<223> PCR primer

<400> 71
attagttaat tggttgttgg ttt 23

<210> 72
<211> 23
<212> DNA
<213> Artificial Sequence

<220>
<223> PCR primer

<400> 72
acacaaataa caactacacc aaa 23

<210> 73
<211> 20
<212> DNA
<213> Artificial Sequence

<220>
<223> PCR primer

<400> 73
aggagtttcg tttttagcgc 20

<210> 74
<211> 20
<212> DNA
<213> Artificial Sequence

<220>

<223> PCR primer

<400> 74
acgacttcaa cgcgaactac 20

<210> 75
<211> 23
<212> DNA
<213> Artificial Sequence

<220>
<223> PCR primer

<400> 75
agtaggagtt ttgtttttag tgt 23

<210> 76
<211> 23
<212> DNA
<213> Artificial Sequence

<220>
<223> PCR primer

<400> 76
acaacttcaa cacaaactac aaa 23

<210> 77
<211> 20
<212> DNA
<213> Artificial Sequence

<220>
<223> sequencing primer

<400> 77
gggtttttt gtttttaatt 20

<210> 78
<211> 22
<212> DNA
<213> Artificial Sequence

<220>
<223> sequencing primer

<400> 78
accaaaaacc actcacaaac tc 22

<210> 79
<211> 22
<212> DNA
<213> Artificial Sequence

<220>
<223> sequencing primer

<400> 79

ggagggtaga tgatttttgag aa 22

<210> 80
<211> 22
<212> DNA
<213> Artificial Sequence

<220>
<223> sequencing primer

<400> 80
cttccccttc ccctaactac ta 22

<210> 81
<211> 24
<212> DNA
<213> Artificial Sequence

<220>
<223> sequencing primer

<400> 81
agggggtgtt attttttttt tttt 24

<210> 82
<211> 19
<212> DNA
<213> Artificial Sequence

<220>
<223> sequencing primer

<400> 82
cccaatccct atccctacc 19

<210> 83
<211> 22
<212> DNA
<213> Artificial Sequence

<220>
<223> sequencing primer

<400> 83
tttttggayg agtttggtta tt 22

<210> 84
<211> 23
<212> DNA
<213> Artificial Sequence

<220>
<223> sequencing primer

<400> 84
ccacctaatt caaacataca acc 23

<210> 85
<211> 20
<212> DNA
<213> Artificial Sequence

<220>
<223> sequencing primer

<400> 85
ttttaygtag ttggtygagg 20

<210> 86
<211> 22
<212> DNA
<213> Artificial Sequence

<220>
<223> sequencing primer

<400> 86
ctccttaaac tataaccccc ct 22

<210> 87
<211> 22
<212> DNA
<213> Artificial Sequence

<220>
<223> sequencing primer

<400> 87
atttagtttg agtaggtygg tg 22

<210> 88
<211> 22
<212> DNA
<213> Artificial Sequence

<220>
<223> sequencing primer

<400> 88
ctccatccta acaatccata aa 22

<210> 89
<211> 17
<212> DNA
<213> Artificial Sequence

<220>
<223> sequencing primer

<400> 89
gggggtgtt tagaggg 17

<210> 90
<211> 23
<212> DNA

<213> Artificial Sequence

<220>
<223> sequencing primer

<400> 90
cctcctacca aaaactacaa acc 23

<210> 91
<211> 19
<212> DNA
<213> Artificial Sequence

<220>
<223> sequencing primer

<400> 91
agaaggggtt taagagagg 19

<210> 92
<211> 19
<212> DNA
<213> Artificial Sequence

<220>
<223> sequencing primer

<400> 92
actatcccca aaaaaaacc 19

<210> 93
<211> 22
<212> DNA
<213> Artificial Sequence

<220>
<223> sequencing primer

<400> 93
atttgtgggt ttttagattg ga 22

<210> 94
<211> 22
<212> DNA
<213> Artificial Sequence

<220>
<223> sequencing primer

<400> 94
ccaaaaaacc actatattcc ca 22

<210> 95
<211> 22
<212> DNA
<213> Artificial Sequence

<220>

<223> sequencing primer

<400> 95
gatgtagatg gttttgttty gg 22

<210> 96
<211> 18
<212> DNA
<213> Artificial Sequence

<220>
<223> sequencing primer

<400> 96
caaacraaaa ccatcccc 18

<210> 97
<211> 20
<212> DNA
<213> Artificial Sequence

<220>
<223> sequencing primer

<400> 97
gggttttttt gtttttaatt 20

<210> 98
<211> 22
<212> DNA
<213> Artificial Sequence

<220>
<223> sequencing primer

<400> 98
accaaaaacc actcacaaac tc 22

<210> 99
<211> 25
<212> DNA
<213> Artificial Sequence

<220>
<223> sequencing primer

<400> 99
ggaaaaatga aggagattta aattt 25

<210> 100
<211> 18
<212> DNA
<213> Artificial Sequence

<220>
<223> sequencing primer

<400> 100

aataacctaa acrccccc 18

## Claims

1. A method for determining whether a subject has developed, is developing, is predisposed for developing or is relapsing after treatment of a cancer within the aero-digestive system, comprising the step of

   a)

   (i) determining the methylation level, the number of methylated CpG sites or the methylation state of CpG sites in a nucleic acid sequence in the promoter region, first exon or intron of **FBN1** (as identified by ensembl gene id ENSG00000166147, entrez id 2200) in a sample obtained from said subject, and wherein said sequence comprises the nucleic acid sequence consisting of SEQ ID NO.: 6, and at least one additional marker, or
   (ii) determining the methylation level, the number of methylated CpG sites or the methylation state of CpG sites in nucleic acid sequences comprising a sequences selected from the group consisting of:

   1) A nucleic acid sequence as defined by SEQ ID NO: 6, and at least one additional marker;
   2) A nucleic acid sequence which is complementary to a sequence as defined in 1), and at least one additional marker;
   3) A sub-sequence of a nucleic acid sequence as defined in 1) or 2), and at least one additional marker;
   4) A nucleic acid sequence which is at least 75% identical to a sequence as defined in 1), 2) or 3), and at least one additional marker,

   b) comparing said methylation level, the number of methylated CpG sites or the methylation state of CpG sites to a reference; and
   c) identifying the subject as being likely to develop, developing or being predisposed for developing said cancer, or relapsing after treatment of said cancer, if the methylation level, the number of methylated CpG sites, or the methylation state of CpG sites, are higher than the references, and identifying the subject as unlikely to develop, developing or being predisposed for developing said cancer, or relapsing after treatment of said cancer, if the methylation level, the number of methylated CpG sites or the methylation state of CpG sites is below the references.

2. A method according to claim 1, wherein the methylation level, the number of methylated CpG sites or the methylation state of CpG sites is determined by bisulphite sequencing, quantitative and/or qualitative methylation specific polymerase chain reaction (MSP), pyrosequencing, Southern blotting, restriction landmark genome scanning (RLGS), single nucleotide primer extension, CpG island microarray, SNUPE, COBRA, mass spectrometry, by use of methylation specific restriction enzymes, by measuring the expression level of said genes or a combination thereof.

3. A method according to claim 2, wherein said methylation specific PCR comprises the use of nucleic acid primers which are capable of hybridizing to a nucleic acid sequence comprising 2 CpG sites and a cytosine residue which is not within a CpG site.

4. A method according to any of the preceding claims, wherein the cancer within the aero-digestive system is selected from the group consisting of: colorectal tumours, lung tumours, small cell lung cancer, non-small cell lung cancer, esophageal tumours, gastric tumours, pancreas tumours, liver tumours, tumours of the gall bladder and/or bile duct, tumours of the small bowel, and tumours of the large bowel.

5. A method according to any of the preceding claims, wherein sample is obtained from blood, stool, urine, pleural fluid, gall, bronchial fluid, oral washings, tissue biopsies, ascites, pus, cerebrospinal fluid, aspirate, follicular fluid, tissue or mucus.

6. A method according to any of the preceding claims, wherein said at least one additional marker is selected from the list consisting of CNRIP1, SPG20, SNCA, INA, MAL, ADAMTS1, VIM, SFRP1 and SFRP2, CRABP1.

7. A method according to any of the preceding claims, wherein the methylation states of at least 2 genes are determined.

**8.** A method according to any of the preceding claims, wherein the methylation states of at least 5 genes are determined.

**9.** A method according to any of the preceding claims, wherein the markers create a combined marker signal.

**10.** A method according to any of the preceding claims, wherein the sensitivity and specificity is 80% to 100%.

**11.** A method according to any of the preceding claims, wherein the sensitivity and specificity is determined using receiver-operating characteristics (ROC).

**12.** A method according to any of the preceding claims, wherein if colorectal cancer, the colorectal cancer is selected from the group consisting of pre-cancer, stage A, stage B, stage C, and stage D using the Duke system.

**13.** The use of a FBN1 gene and at least one additional marker in a diagnostic assay, wherein the methylation level, the number of methylated CpG sites or the methylation state of CpG sites is assessed as an indicator of whether a subject has developed, is developing or is predisposed for developing cancer within the aero-digestive system, or whether a subject is relapsing after treatment of cancer within the aero-digestive system.

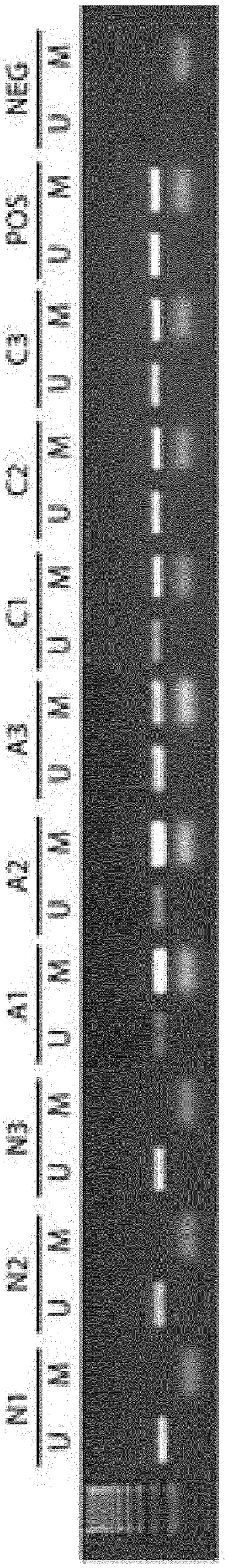


Fig. 1

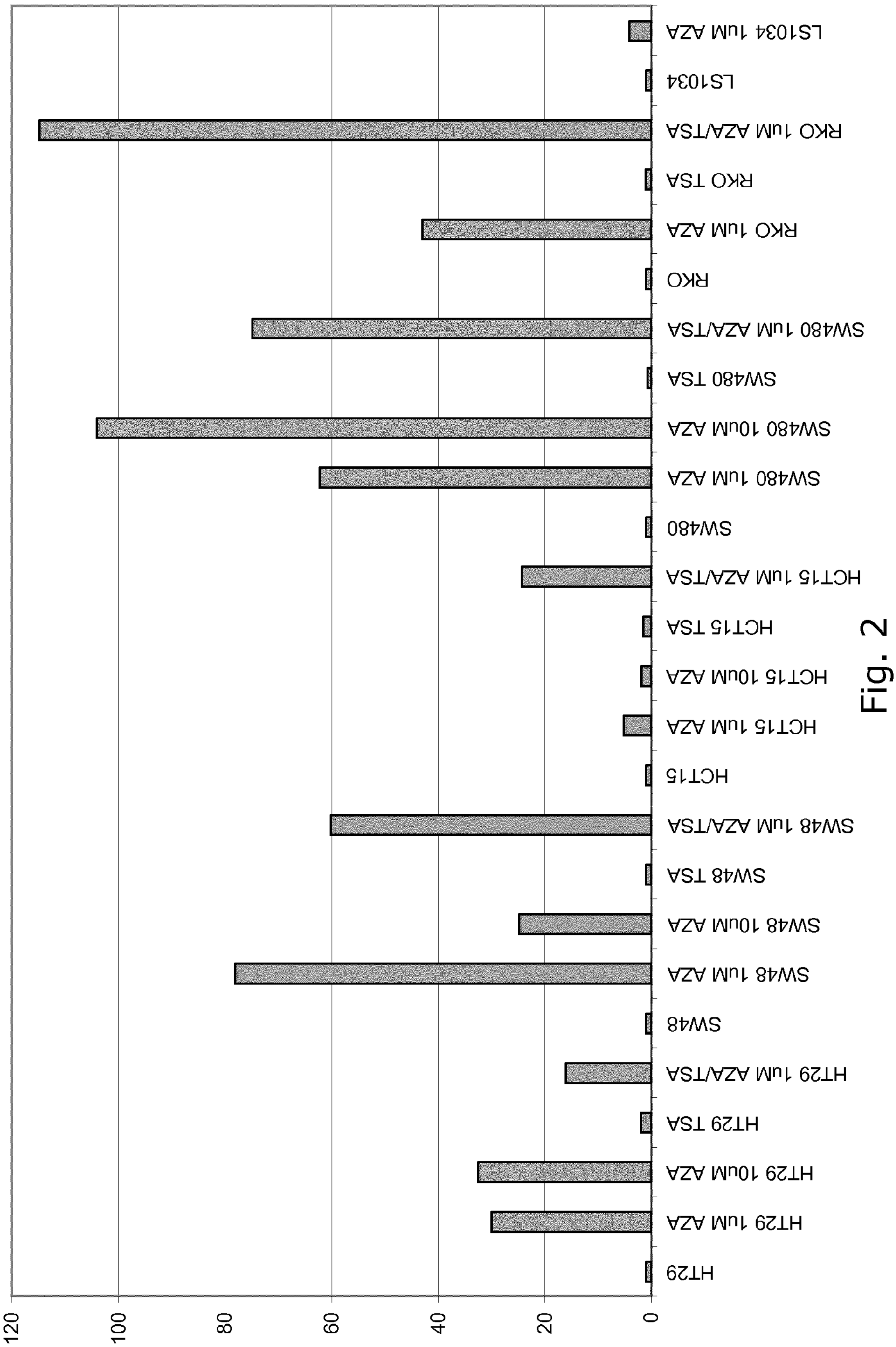
HT29
HT29 1uM AZA
HT29 10uM AZA
HT29 TSA
HT29 1uM AZA/TSA
SW48
SW48 1uM AZA
SW48 10uM AZA
SW48 TSA
SW48 1uM AZA/TSA
HCT15
HCT15 1uM AZA
HCT15 10uM AZA
HCT15 TSA
HCT15 1uM AZA/TSA
SW480
SW480 1uM AZA
SW480 10uM AZA
SW480 TSA
SW480 1uM AZA/TSA
RKO
RKO 1uM AZA
RKO TSA
RKO 1uM AZA/TSA
LS1034
LS1034 1uM AZA
0
20
40
60
80
100
120

Fig. 2

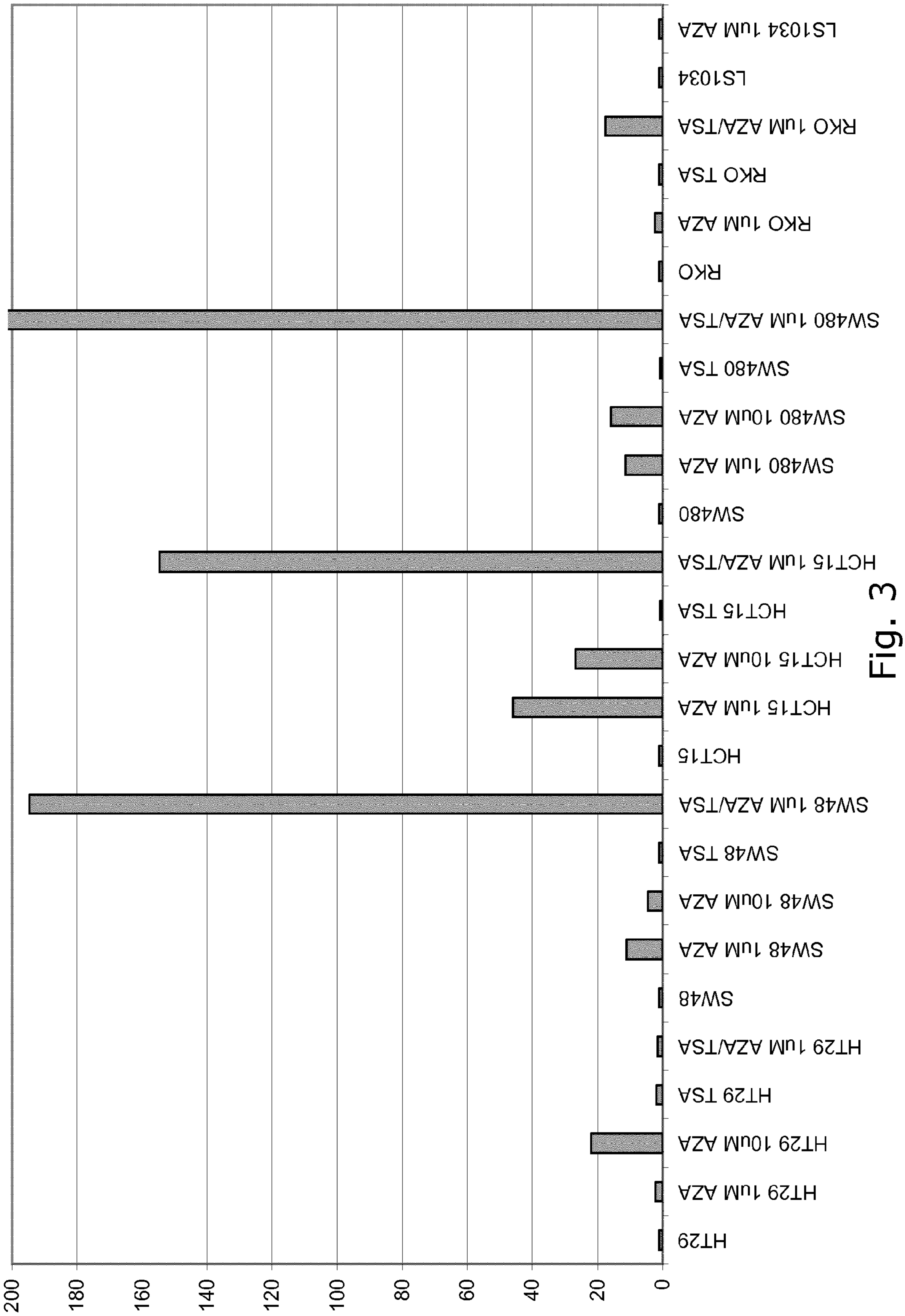
HT29
HT29 1uM AZA
HT29 10uM AZA
HT29 TSA
HT29 1uM AZA/TSA
SW48
SW48 1uM AZA
SW48 10uM AZA
SW48 TSA
SW48 1uM AZA/TSA
HCT15
HCT15 1uM AZA
HCT15 10uM AZA
HCT15 TSA
HCT15 1uM AZA/TSA
SW480
SW480 1uM AZA
SW480 10uM AZA
SW480 TSA
SW480 1uM AZA/TSA
RKO
RKO 1uM AZA
RKO TSA
RKO 1uM AZA/TSA
LS1034
LS1034 1uM AZA
0
20
40
60
80
100
120
140
160
180
200

Fig. 3

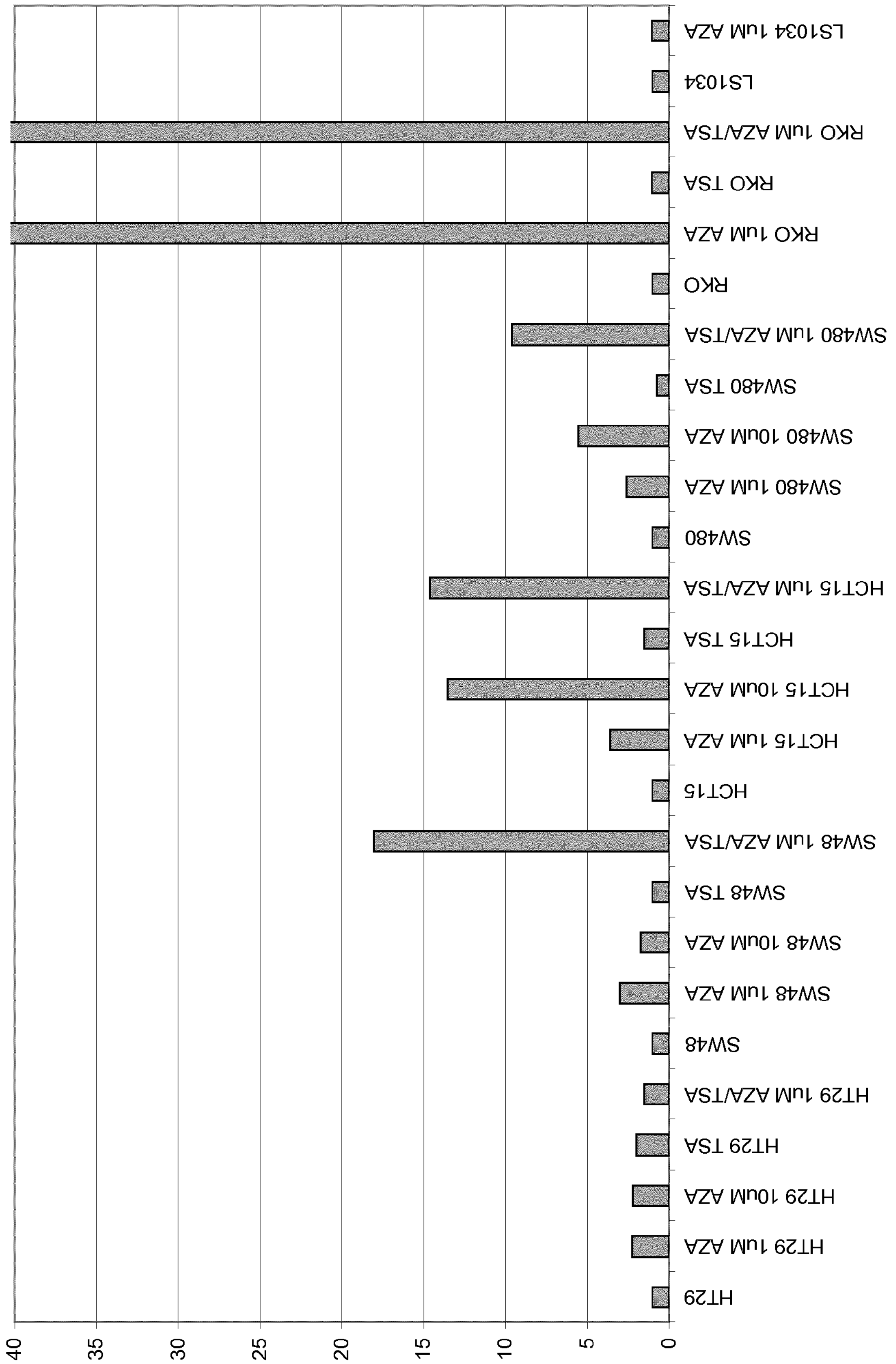

LS1034 1uM AZA
LS1034
RKO 1uM AZA/TSA
RKO TSA
RKO 1uM AZA
RKO
SW480 1uM AZA/TSA
SW480 TSA
SW480 10uM AZA
SW480 1uM AZA
SW480
HCT15 1uM AZA/TSA
HCT15 TSA
HCT15 10uM AZA
HCT15 1uM AZA
HCT15
SW48 1uM AZA/TSA
SW48 TSA
SW48 10uM AZA
SW48 1uM AZA
SW48
HT29 1uM AZA/TSA
HT29 TSA
HT29 10uM AZA
HT29 1uM AZA
HT29
40
35
30
25
20
15
10
5
0


Fig. 4

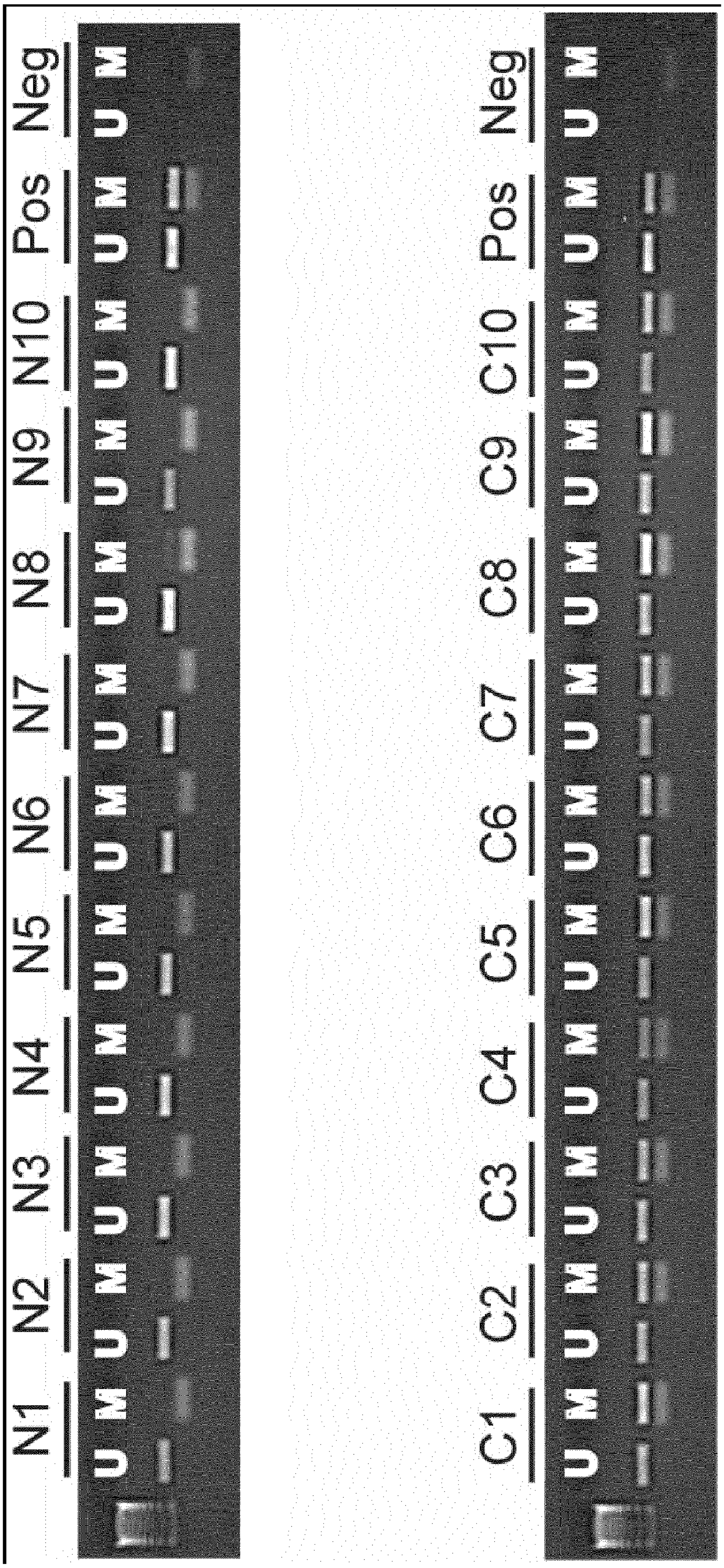
N1 N2 N3 N4 N5 N6 N7 N8 N9 N10 Pos Neg
U M U M U M U M U M U M U M U M U M U M U M U M
C1 C2 C3 C4 C5 C6 C7 C8 C9 C10 Pos Neg
U M U M U M U M U M U M U M U M U M U M U M U M

Fig. 5

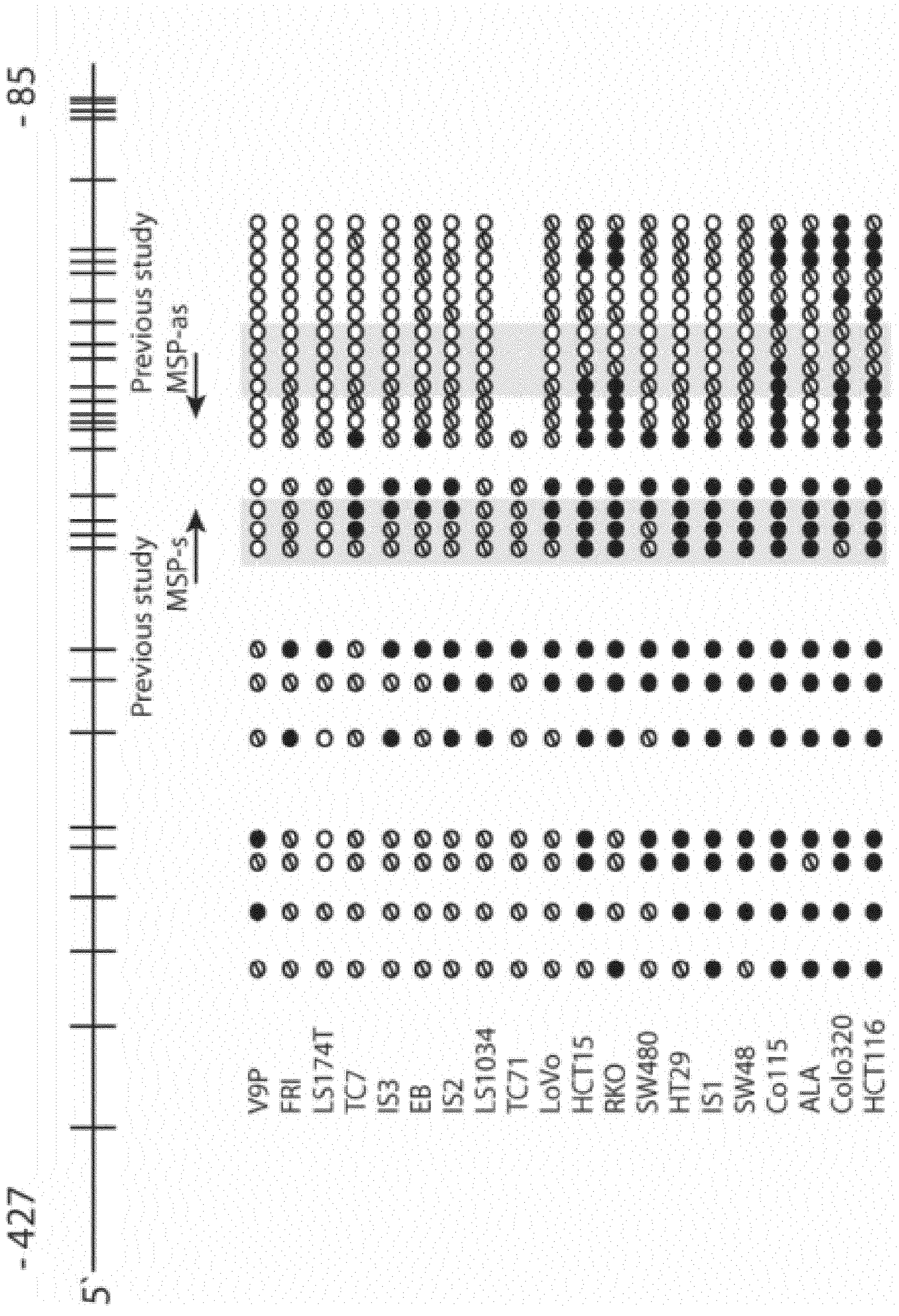


Fig. 6 (cont. next page)

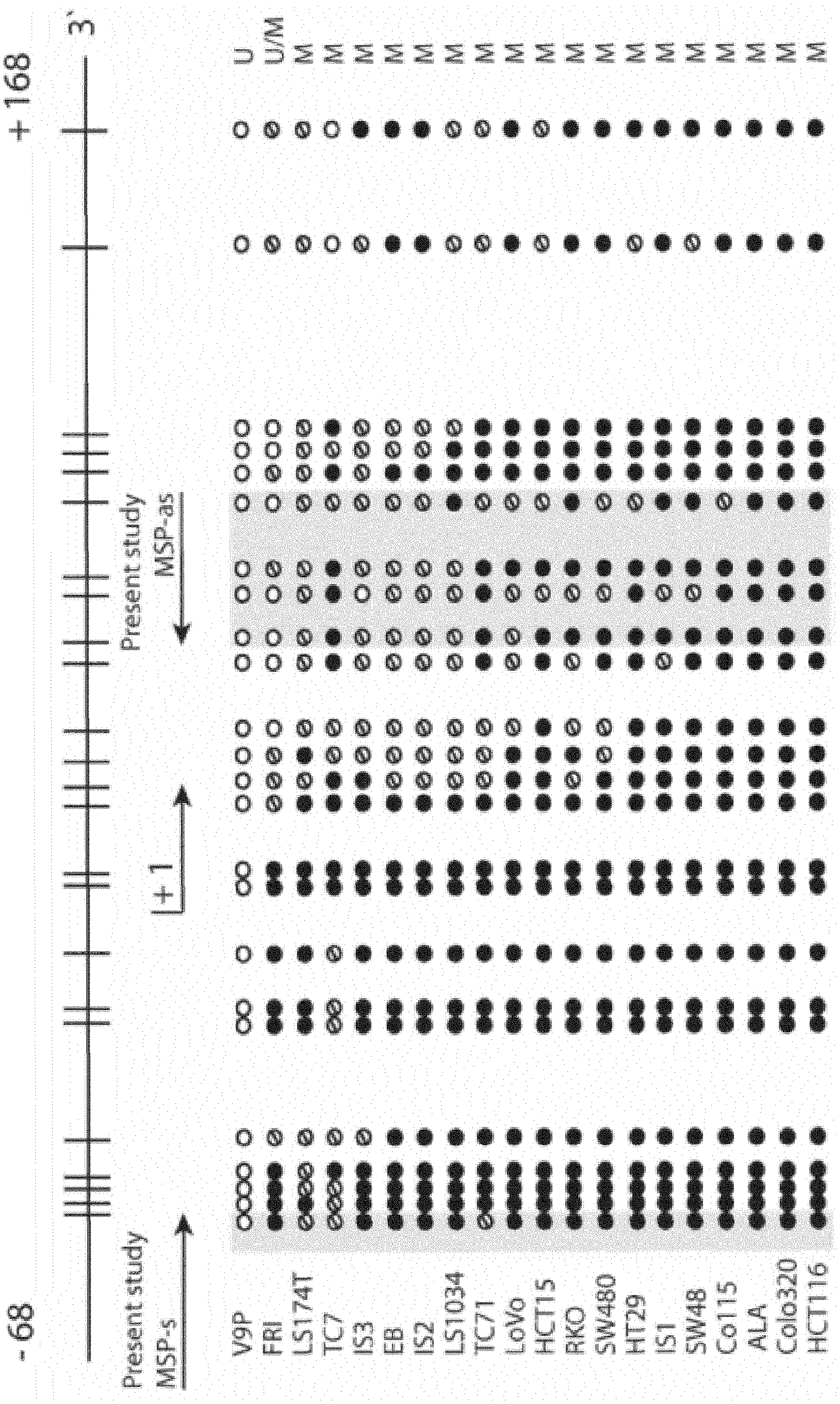


Fig. 6 (cont. from previous page)

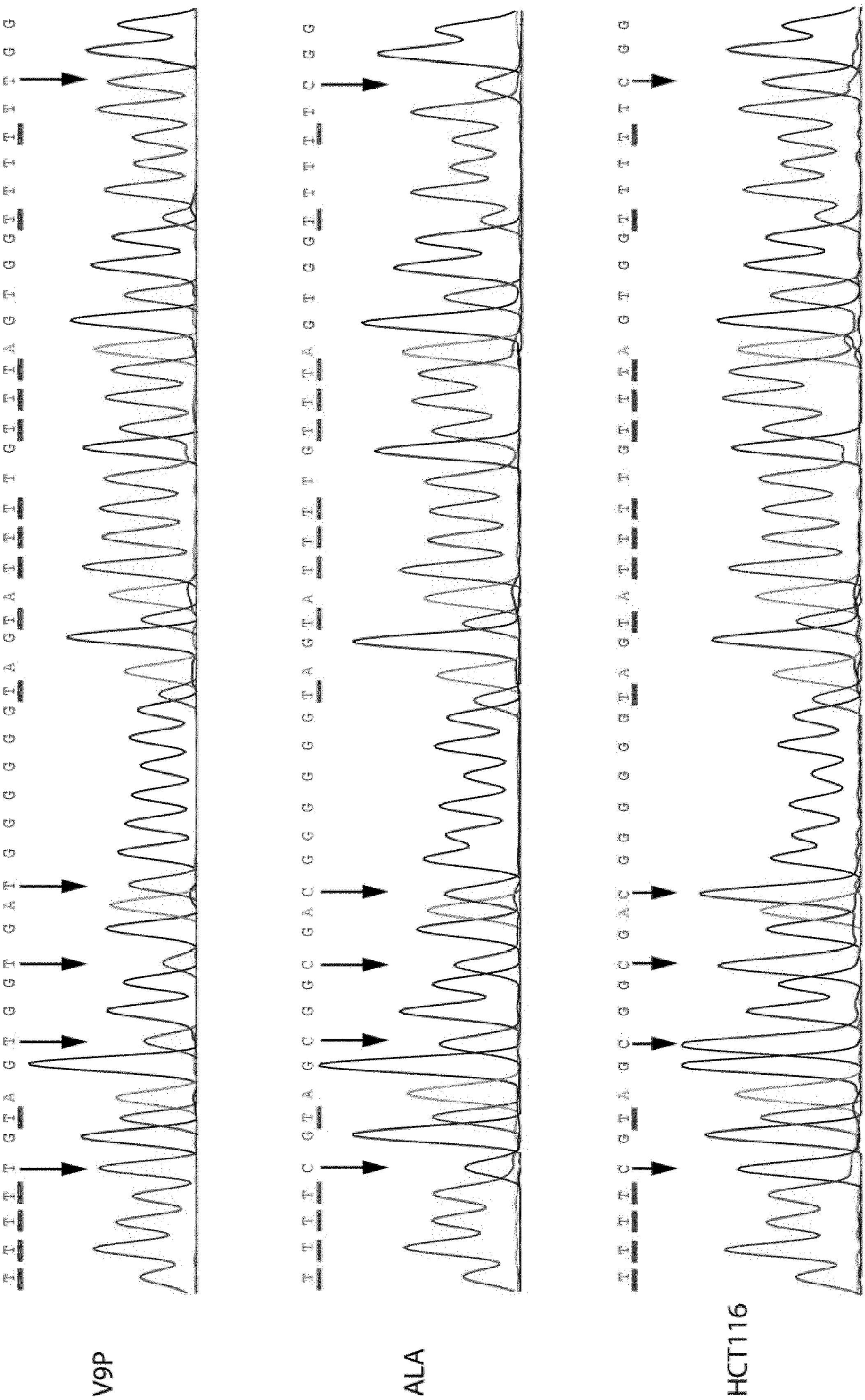


Fig. 7

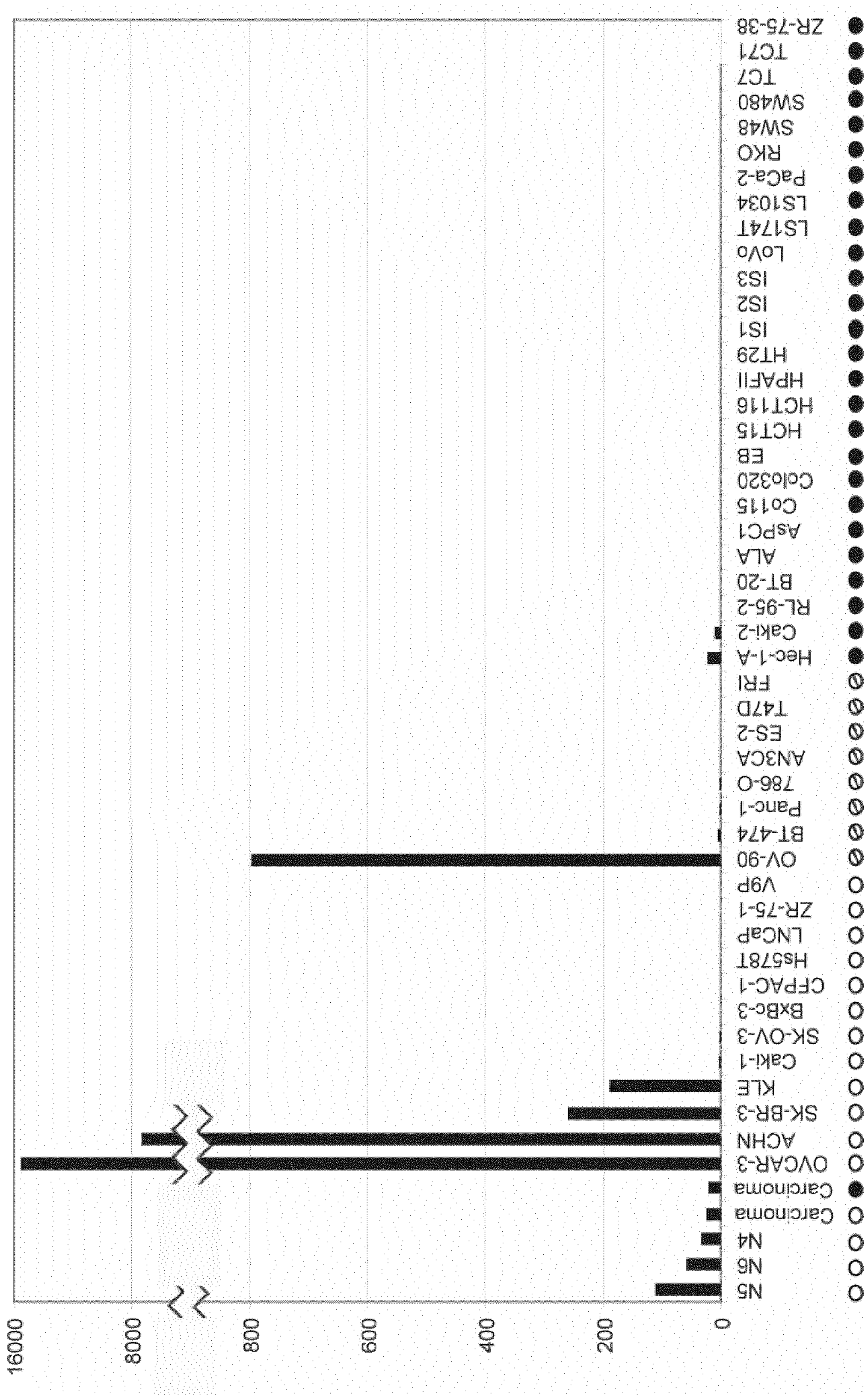
16000
8000
800
600
400
200
0
N5
N6
N4
Carcinoma
Carcinoma
OVCAR-3
ACHN
SK-BR-3
KLE
Caki-1
SK-OV-3
BxBc-3
CFPAC-1
Hs578T
LNCaP
ZR-75-1
V9P
OV-90
BT-474
Panc-1
786-O
AN3CA
ES-2
T47D
FRI
Hec-1-A
Caki-2
RL-95-2
BT-20
ALA
AsPC1
Co115
Colo320
EB
HCT15
HCT116
HPAFII
HT29
IS1
IS2
IS3
LoVo
LS174T
LS1034
PaCa-2
RKO
SW48
SW480
TC7
TC71
ZR-75-38

Fig. 8

Fig. 9

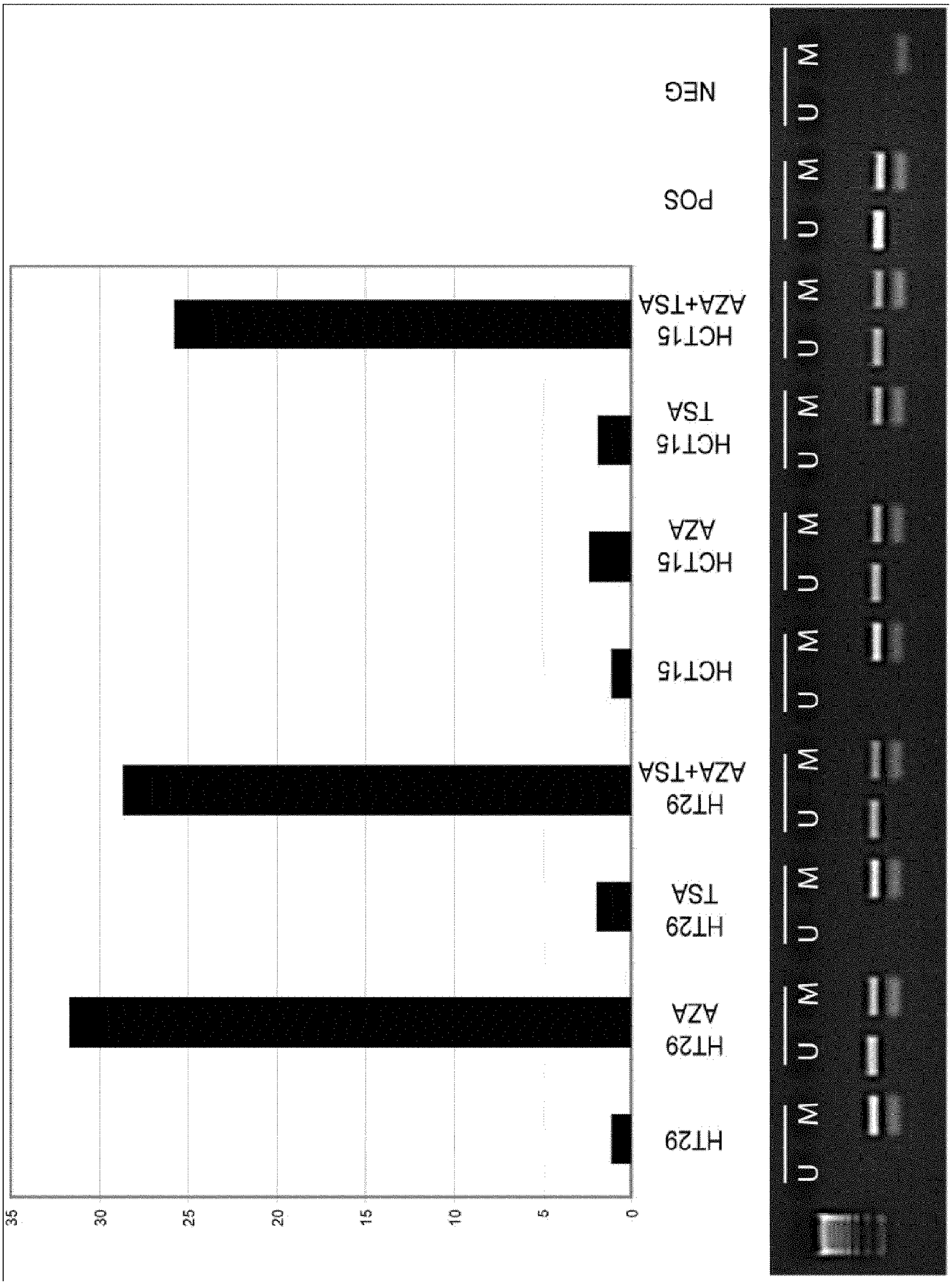
HT29
HT29 AZA
HT29 TSA
HT29 AZA+TSA
HCT15
HCT15 AZA
HCT15 TSA
HCT15 AZA+TSA
POS
NEG
U
M
0
5
10
15
20
25
30
35

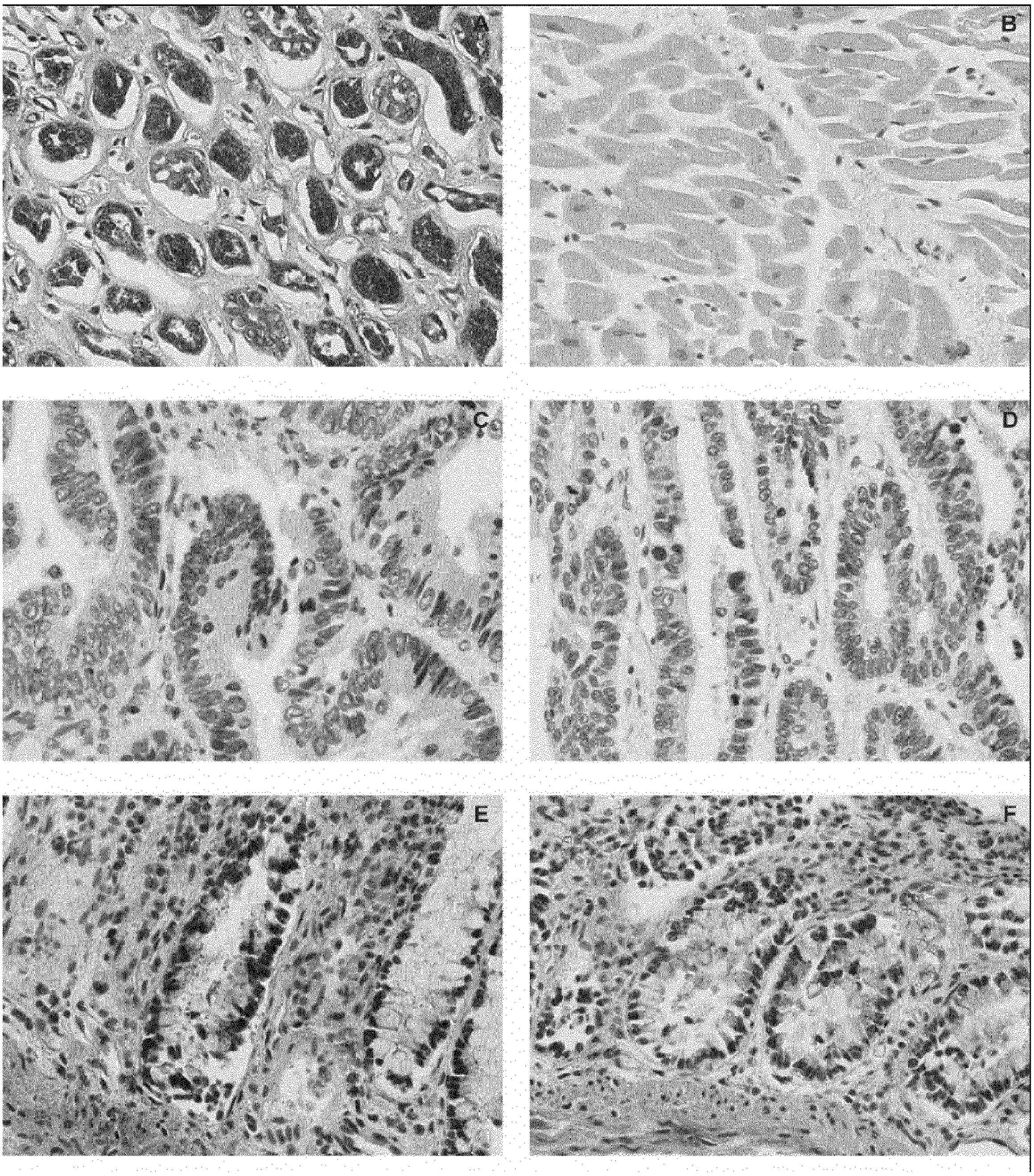


Fig. 10

Europäisches Patentamt
European Patent Office
Office européen des brevets

# EUROPEAN SEARCH REPORT

Application Number
EP 14 17 6354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/19845 A (UNIV JOHNS HOPKINS MED [US]) 22 March 2001 (2001-03-22)<br>* FBN1;<br>page 31, lines 7-11; claims 10-12 *<br>----- | 1-13 | INV.<br>C12Q1/68 |
| X | WO 02/070742 A (EPIGENOMICS AG [DE]) 12 September 2002 (2002-09-12)<br>* page 47; example 1 *<br>----- | 1-13 | |
| X | WANG YIPENG ET AL: ""Promoter array" studies identify cohorts of genes directly regulated by methylation, copy number change, or transcription factor binding in human cancer cells"<br>In:<br>2005, ANNALS OF THE NEW YORK ACADEMY OF SCIENCES NEW YORK ACAD SCIENCES, 2 EAST 63RD ST, NEW YORK, NY 10021 USA SERIES : ANNALS OF THE NEW YORK ACADEMY OF SCIENCES (ISSN 0077-8923(PRINT)), PAGE(S) 162-185, 7TH NIH SYMPOSIUM ON THERAPEUTIC OLIGONUCLEOTIDES; BETHESDA, MD, USA; DECEMBER 13 -14, 2004, XP008152217,<br>ISSN: null<br>pages 162-185,<br>* the whole document *<br>----- | 1-13 | TECHNICAL FIELDS SEARCHED (IPC)<br>C12Q |
| A | WO 2005/005601 A (UNIV MICHIGAN [US]; CLARKE MICHAEL F [US]; LIU RUI [US] CLARKE MICHAEL) 20 January 2005 (2005-01-20)<br>* discloses CRIP1 (= CNRIP1), TAHCCP1 (= SPG20), FBN1, SNCA, and INA;<br>page 32, line 16 *<br>* page 49, line 27 *<br>* page 30, line 2 *<br>* page 40, line 66 *<br>* page 39, line 74 *<br>-----<br>-/-- | 1 | |

1 The present search report has been drawn up for all claims

| Place of search | Date of completion of the search | Examiner |
|---|---|---|
| The Hague | 28 October 2014 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 6354

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/014781 A (TRUSTEES OF THE UNIVERSITY OF [US] S THE BOARD OF TRUSTEES OF THE [US]) 17 February 2005 (2005-02-17)<br>* SPG20 and FBN1 involved in ovarian serous papillary carcinoma;<br>claim 8 *<br>----- | 1 | |
| | | | TECHNICAL FIELDS SEARCHED (IPC) |

The present search report has been drawn up for all claims

1

| Place of search | Date of completion of the search | Examiner |
|---|---|---|
| The Hague | 28 October 2014 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 17 6354

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2014

| Patent document cited in search report | Publication date | Patent family member(s) | Publication date |
|---|---|---|---|
| WO 0119845 A | 22-03-2001 | AU 783645 B2 | 17-11-2005 |
| | | AU 7586900 A | 17-04-2001 |
| | | AU 2006200264 A1 | 23-02-2006 |
| | | CA 2388419 A1 | 22-03-2001 |
| | | EP 1218399 A1 | 03-07-2002 |
| | | EP 2290105 A1 | 02-03-2011 |
| | | JP 2003509039 A | 11-03-2003 |
| | | US 6783933 B1 | 31-08-2004 |
| | | US 2005026207 A1 | 03-02-2005 |
| | | US 2009148848 A1 | 11-06-2009 |
| | | WO 0119845 A1 | 22-03-2001 |
| WO 02070742 A | 12-09-2002 | AT 429511 T | 15-05-2009 |
| | | CA 2439854 A1 | 12-09-2002 |
| | | EP 1364069 A1 | 26-11-2003 |
| | | JP 2004529630 A | 30-09-2004 |
| | | US 2002137086 A1 | 26-09-2002 |
| | | WO 02070742 A1 | 12-09-2002 |
| WO 2005005601 A | 20-01-2005 | AU 2004256425 A1 | 20-01-2005 |
| | | AU 2008202471 A1 | 26-06-2008 |
| | | CA 2528669 A1 | 20-01-2005 |
| | | EP 1639090 A2 | 29-03-2006 |
| | | EP 2003196 A2 | 17-12-2008 |
| | | EP 2481814 A2 | 01-08-2012 |
| | | JP 2007516693 A | 28-06-2007 |
| | | KR 20060031809 A | 13-04-2006 |
| | | US 2006019256 A1 | 26-01-2006 |
| | | US 2008292546 A1 | 27-11-2008 |
| | | US 2012315216 A1 | 13-12-2012 |
| | | US 2013244256 A1 | 19-09-2013 |
| | | WO 2005005601 A2 | 20-01-2005 |
| WO 2005014781 A | 17-02-2005 | AT 426680 T | 15-04-2009 |
| | | CA 2528606 A1 | 17-02-2005 |
| | | EP 1633854 A2 | 15-03-2006 |
| | | US 2005048535 A1 | 03-03-2005 |
| | | US 2011223181 A1 | 15-09-2011 |
| | | US 2013244900 A1 | 19-09-2013 |
| | | WO 2005014781 A2 | 17-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MARAZUELA M et al.** *J Histochem Cytochem,* 2003, vol. 51, 665-674
- **SAMBROOK et al.** Molecular Cloning: A Laboratory Manual. Cold Spring Harbor, 1989
- **ZWEIG, M. H. ; CAMPBELL, G.** *Clin. Chem.,* 1993, vol. 39, 561-577
- **MORI Y ; CAI K ; CHENG Y ; WANG S ; PAUN B ; HAMILTON JP ; JIN Z ; SATO F ; BERKI AT ; KAN T.** A genome-wide search identifies epigenetic silencing of somatostatin, tachykinin-1, and 5 other genes in colon cancer. *Gastroenterology,* 2006, vol. 131, 797-808
- **LIND GE ; KLEIVI K ; MELING GI ; TEIXEIRA MR ; THIIS-EVENSEN E ; ROGNUM TO ; LOTHE RA.** ADAMTS1, CRABP1, and NR3C1 Identified as Epigenetically Deregulated genes in Colorectal Tumourigenesis. *Cell Oncol,* 2006, vol. 28, 259-272
- **LAIRD PW.** The power and the promise of DNA methylation markers. *Nat Rev Cancer,* 2003, vol. 3, 253-266
- **MULLER HM ; OBERWALDER M ; FIEGL H ; MORANDELL M ; GOEBEL G ; ZITT M ; MUHLTHALER M ; OFNER D ; MARGREITER R ; WIDSCHWENDTER M.** Methylation changes in faecal DNA: a marker for colorectal cancer screening?. *Lancet,* 2004, vol. 363, 1283-1285
- **CHEN WD ; HAN ZJ ; SKOLETSKY J ; OLSON J ; SAH J ; MYEROFF L ; PLATZER P ; LU S ; DAWSON D ; WILLIS J.** Detection in fecal DNA of colon cancer-specific methylation of the nonexpressed vimentin gene. *J Natl Cancer Inst,* 2005, vol. 97, 1124-1132
- **C.GRUNAU ; S.J.CLARK ; A.ROSENTHAL.** Bisulfite genomic sequencing: systematic investigation of critical experimental parameters. *Nucleic Acids Res.,* 2001, vol. 29, E65
- **M.F.FRAGA ; M.ESTELLER.** DNA methylation: a profile of methods and applications. *Biotechniques,* 2002, vol. 33, 632-649
- **B.SMITH-SØRENSEN ; G.E.LIND ; R.I.SKOTHEIM ; S.D.FOSSÅ ; Ø.FODSTAD ; A.E.STENWIG ; K.S.JAKOBSEN ; R.A.LOTHE.** Frequent promoter hypermethylation of the 06-Methylguanine-DNA Methyltransferase (MGMT) gene in testicular cancer. *Oncogene,* 2002, vol. 21, 8878-8884
- **J.G.HERMAN ; J.R.GRAFF ; S.MYÖHÄNEN ; B.D.NELKIN ; S.B.BAYLIN.** Methylation-specific PCR: a novel PCR assay for methylation status of CpG islands. *Proc. Natl. Acad. Sci. U. S. A.,* 1996, vol. 93, 9821-9826
- **S.DERKS ; M.H.LENTJES ; D.M.HELLEBREKERS ; A.P.DE BRUINE ; J.G.HERMAN ; E.M.VAN.** Methylation-specific PCR unraveled. *Cell Oncol,* 2004, vol. 26, 291-299
- **L.C.LI ; R.DAHIYA.** MethPrimer: designing primers for methylation PCRs. *Bioinformatics,* 2002, vol. 18, 1427-1431
- **J.R.MELKI ; P.C.VINCENT ; S.J.CLARK.** Concurrent DNA hypermethylation of multiple genes in acute myeloid leukemia. *Cancer Res.,* 1999, vol. 59, 3730-3740